(19) Europäisches Patentamt
European Patent Office
Office européen des brevets

(11) **EP 4 123 021 A1**

(12) **EUROPEAN PATENT APPLICATION**
published in accordance with Art. 153(4) EPC

(43) Date of publication:
**25.01.2023 Bulletin 2023/04**

(21) Application number: **21776088.3**

(22) Date of filing: **22.03.2021**

(51) International Patent Classification (IPC):
***C12N 7/00*** *(2006.01)* ***C12N 15/113*** *(2010.01)*
***A61P 35/00*** *(2006.01)* ***A61K 35/761*** *(2015.01)*

(52) Cooperative Patent Classification (CPC):
**A61K 35/761; A61P 35/00; C12N 7/00; C12N 15/113**

(86) International application number:
**PCT/KR2021/003488**

(87) International publication number:
**WO 2021/194180 (30.09.2021 Gazette 2021/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.03.2020 KR 20200035174**

(71) Applicant: **Curigin Co.,Ltd.**
**Seoul 04778 (KR)**

(72) Inventors:
- **CHOI, Jin-Woo**
  **Seongnam-si Gyeonggi-do 13559 (KR)**
- **PARK, Seong-Hoon**
  **Wanju-gun Jeollabuk-do 55363 (KR)**
- **PETER CHARLES, Goughnour**
  **Suwon-si Gyeonggi-do 16692 (KR)**
- **YOO, Jung-Ki**
  **Yangju-si Gyeonggi-do 11456 (KR)**
- **CHOI, Chung-Gab**
  **Incheon 21596 (KR)**
- **UM, Ki-Hwan**
  **Ansan-si Gyeonggi-do 15338 (KR)**
- **LEE, Eui-Jin**
  **Gunpo-si Gyeonggi-do 15815 (KR)**
- **LEE, Hyung-Been**
  **Seoul 04779 (KR)**

(74) Representative: **BCKIP Part mbB**
**Siegfriedstraße 8**
**80803 München (DE)**

Remarks:
The complete document including Reference Table(s) and the Sequence Listing(s) can be downloaded from the EPO website

(54) **STRUCTURE OF ONCOLYTIC VIRUS COMPRISING BISPECIFIC NUCLEIC ACID MOLECULE**

(57) The present invention relates to an anti-tumor adenovirus and an anticancer composition comprising same. Double-stranded siRNA of the present invention simultaneously inhibits the expression of a first nucleic acid and a second nucleic acid, thus promoting the death of cancer cells, exhibits more remarkable anticancer activity as compared to co-treatment of respective siRNAs, has a synergistic effect of improving cancer cell death in combined treatment with an anticancer agent. The adenovirus comprising a shRNA-encoding expression cassette expressing the double-stranded siRNA, and a hTERT promoter evades immune responses in the body and is specifically delivered to cancer cells, thus having a systemic therapeutic effect, can be locally delivered, has excellent selectivity, and exhibits a significant anticancer effect even in minimally invasive treatment, and thus, the adenovirus can be effectively used as an anticancer composition or an anticancer adjuvant in various carcinomas.

Fig.19

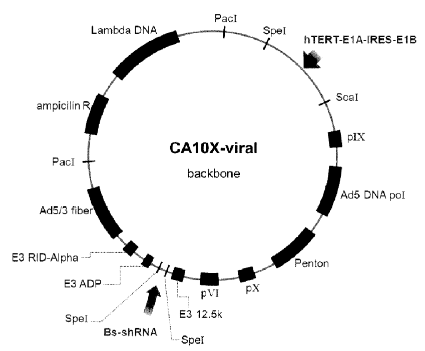


EP 4 123 021 A1

## Description

[Technical Field]

The present invention relates to an anti-tumor adenovirus and an anticancer composition including the same.

[Background Art]

Cancer is one of diseases that causes the most significant number of deaths all around the world, the development of innovative cancer medicine helps patients to save medical expenses incurred during treatment and allows the medical community to create higher value-added medicines. According to the statistics in 2008, the market size of molecular targeted therapy to overcome drug-resistant problems in existing anticancer drugs was $17.5 billion in seven major countries (US, Japan, France, Germany, Italy, Spain, and UK). It was expected that the size would be about $45 billion, and its growth rate would be 9.5% in 2018 as compared to 2008. Cancer therapy is divided into surgery, radiation therapy, chemotherapy, and biological therapy. For the chemotherapy among them, chemotherapy drugs inhibit the growth of tumor cells or kill them, which has toxicity and harmful effects even on normal cells. Though the anticancer agent causes an immediate reaction, it gradually loses effectiveness after a certain period of time, which is called drug resistance. Thus, it is urgent to develop anticancer drugs that react selectively on tumor cells and has no effect from drug resistance (the present address of combating cancer Biowave 2004. 6 (19)). The new anticancer agents have recently developed, which uses the molecular genetic information and targets molecular properties of cancer. There have been reports that the anticancer drugs for a specific molecular target showed only by cancer cells have no drug resistance.

The technology to inhibit gene expression is an important tool to develop medicines for curing diseases and verify the targets. As the role of RNA interference (hereinafter referred to as RNAi) was revealed, it was found that RNAi reacts with sequence-specific mRNA of diverse kinds of mammalian cells (Silence of the transcripts: RNA interference in medicine. J Mol Med (2005) 83: 764773). RNAi is a phenomenon that inhibits a specific protein expression by specifically combining the small interfering ribonucleic acid (small interfering RNA, hereinafter referred to as siRNA) including a double helical structure having a size of 21 to 25 nucleotides to the mRNA transcript with a complementary sequence and decomposing the corresponding transcripts. In a cell, double-stranded RNA (dsRNA) is processed by an endonuclease being called Dicer to be translated into siRNA with 21 to 23 base pairs (bps). The siRNA combines with RISC (RNA-induced silencing complex), and a guide strand (antisense) recognizes and decomposes targeted mRNA. Thus, these processes sequence-specifically interfere the targeted gene expression (NUCLEIC-ACID THERAPEUTICS: BASIC PRINCIPLES AND RECENT APPLICATIONS. Nature Reviews Drug Discovery. 2002. 1, 503-514). According to Bertrand *et al.,* it was found that the siRNA has a more excellent effect of inhibiting expression of the mRNA in vivo and in vitro as compared to the antisense oligonucleotides (ASO) on the same target gene, and the effect is long-lasting (Comparison of Antisense Oligonucleotides and siRNAs in Cell Culture and in Vivo, Biochem. Biophys. Res. Commun., 296: 1000-1004, 2002). In the global market size, RNAi technology-based therapeutics markets, including siRNA, was analyzed to create 12 trillion won or more by 2020. As the target to apply the technology would be dramatically expanded, it would be evaluated as a future gene therapy technology to treat challenging diseases that are hard to cure with existing antibody- and compound-based medicines. Moreover, as the siRNA mechanism sequence-specifically controls the targeted gene expression by complementary combining with a targeted mRNA, it has a great advantage to develop a lead compound that is optimized to all the targeted protein, including targeted materials which is impossible to make a medicine. While the existing antibody-based medicines or small molecule drugs require longer periods and higher cost to develop and optimize a specifically targeted protein, the siRNA mechanism may be applied to a wider range of targets and reduce the time to develop medicines (Progress Towards in Vivo Use of siRNAs. MOLECULAR THERAPY. 2006 13(4):664-670). Accordingly, there have been recent studies on selectively inhibiting a specific gene expression in the transcription level and developing medicines to cure diverse kinds of disease, specifically the tumor, while RNAi phenomenon provided a possible solution to the problems arising from the existing chemically synthesized medicine development. Furthermore, siRNA-based medicine has another advantage to predict side effect because it has a specific target compared to existing ones. However, in case of tumor caused by various gene problems, the target specificity is a primary cause of impeding the effect of a therapy.

[Disclosure]

[Technical Problem]

An aspect of the present invention is directed to providing an anti-tumor adenovirus.
In addition, an aspect of the present invention is directed to providing a composition for treating cancer.

[Technical Solution]

An embodiment of the present invention provides an anti-tumor adenovirus including a nucleotide sequence having a first nucleic acid as a target sequence and a nucleotide sequence having a second nucleic acid as a target sequence.

In addition, an embodiment of the present invention provides a composition for treating cancer including the anti-tumor adenovirus.

[Advantageous Effects]

According to an embodiment of the present invention, double-stranded siRNA of an embodiment of the present invention simultaneously inhibits the expression of a first nucleic acid and a second nucleic acid, thus promoting the death of cancer cells, exhibits more remarkable anticancer activity as compared to co-treatment of respective siRNAs, has a synergistic effect of improving cancer cell death in combined treatment with an anticancer agent. The adenovirus including a shRNA-encoding expression cassette expressing the double-stranded siRNA, and a hTERT promoter evades immune responses in the body and is specifically delivered to cancer cells, thus having a systemic therapeutic effect, can be locally delivered, has excellent selectivity, and exhibits a significant anticancer effect even in minimally invasive treatment, and thus, the adenovirus can be effectively used as an anticancer composition or an anticancer adjuvant in various carcinomas.

[Description of Drawings]

FIG. 1 is a view showing a map of a vector for intracellular expression of shRNA including a double target siRNA set of the present invention.

FIG. 2 is a view identifying the effect of inhibiting mTOR or STAT3 gene expression by a double target double-stranded siRNA of sets 1 to 9 of the present invention.

FIG. 3 is a view identifying the effect of inhibiting the expression of BCL2 gene (left) and BI-1 gene (right) by the double target siRNA set 10 (si-BB1) of the present invention.

FIG. 4 is a view identifying the effect of inhibiting the expression of the BCL2 gene and the BI-1 gene by the double target siRNA sets 11 to 15 of the present invention:

NC: control siRNA; and
si-BB2 to si-BB6: siRNA sets 11 to 15 of the present invention.

FIG. 5 is a view identifying the effect of inhibiting the expression of AR gene and mTOR gene by the double target siRNA set 16 of the present invention in a cancer cell line:

A: h460 cell line;
B: pc3 cell line;
NC: control siRNA;
siAR: siRNA for AR;
simTOR: siRNA for mTOR; and
si-AT1: AR and mTOR double target siRNA set 16 of the present invention.

FIG. 6 is a view identifying the effect of inhibiting the expression of AR gene and mTOR gene in an A549 cell line by the double target siRNA sets 17 to 28 of the present invention:

NC: control siRNA; and
si-AT2 to si-AT13: siRNA sets 17 to 28 of the present invention.

FIG. 7 is a view identifying the effect of inhibiting the expression of c-MET and PD-L1 genes by a double target siRNA (double strand) set capable of simultaneously inhibiting c-MET and PD-L1 of the present invention in various cancer cell lines.

FIG. 8 is a view identifying the expression levels of mTOR and STAT3 by a vector including a sequence encoding the TTGGATCCAA loop shRNA sequence represented by SEQ ID NO: 66 or the TTCAAGAGAG loop shRNA sequence represented by SEQ ID NO: 67 according to the amount of DNA in the shRNA expression cassette.

FIG. 9 is a view comparing the gene expression inhibition effect of two single target siRNAs connected in series with the double target shRNA of the present invention.
FIG. 10 is a view identifying the cell survival rate of human lung cancer cell line A549 cells when mTOR and STAT3 are simultaneously inhibited with the double target siRNA of the present invention (double target siRNA of sets 1 to 9).
FIG. 11 is a view identifying the cell survival rate of human lung cancer cell line A549 cells when mTOR and STAT3 are simultaneously inhibited with the double target siRNA of the present invention after cisplatin treatment.
FIG. 12 is a view identifying the cell survival rate of human lung cancer cell line A549 cells when mTOR and STAT3 are simultaneously inhibited with the double target siRNA of the present invention after paclitaxel treatment.
FIG. 13 is a view identifying the cell survival rate of human lung cancer cell line A549 cells when mTOR and STAT3 are simultaneously inhibited with the double target siRNA of the present invention after 5-FU (5-fluorouracil) treatment.
FIG. 14 is a view identifying the death of cancer cells by the co-treatment with the double target siRNA set of the present invention and an anticancer agent:

A: co-treatment with anticancer agent + Bcl2 siRNA + BI-1 siRNA; and
B: co-treatment with the double target siRNA set 10 (si-BB 1) of the present invention + an anticancer agent.

FIG. 15 is a view identifying the cancer cell killing effect of ABT-737, which is a Bcl2 inhibitor used as an anticancer agent, and the double target siRNA set 10 (si-BB 1) of the present invention, and the synergistic effect by the co-treatment therewith.
FIG. 16 is a view comparing the cancer cell killing effect by the co-treatment with double target siRNA set 10 (si-BB 1) and an anticancer agent with a group treated with siRNA for BCL2 gene and siRNA for BI-1 gene.
FIG. 17 is a view identifying the cancer cell killing effect by the co-treatment with an anticancer agent of double target siRNA set 1 in cancer cell lines:

NC: control siRNA;
no treat: a control group not treated with an anticancer agent;
si-AT1: AR and mTOR double target siRNA set 16 of the present invention;
A: a DU145 cell line; and
B: an H460 cell line.

FIG. 18 is a view schematically illustrating the structure of the adenovirus of the present invention.
FIG. 19 is a view showing a vector map of an adenoviral vector of the present invention:
Bs-shRNA: a sequence insertion site encoding the double target shRNA of the present invention.
FIG. 20 is a view identifying the effect of inhibiting the expression of mTOR and STAT3 genes by the recombinant adenovirus CA102 of the present invention, which comprise hTERT promoter and expresses double target shRNA, in bladder cancer cell lines T24 and 253JBV.
FIG. 21 is a view identifying the effect of inhibiting the expression of mTOR and STAT3 genes by the recombinant adenovirus CA102 of the present invention, which comprise hTERT promoter and expresses double target shRNA, in head and neck cancer cell lines FaDu and HSC-2.
FIG. 22 is a view identifying the effect of inhibiting the expression of mTOR and STAT3 genes by the recombinant adenovirus CA102 of the present invention, which comprise hTERT promoter and expresses double target shRNA, in skin squamous carcinoma cell lines A431 and HSC-5.
FIG. 23 is a view identifying the effect of inhibiting the expression of mTOR and STAT3 genes at the protein level by the recombinant adenovirus CA102 of the present invention in bladder cancer cell lines T24 and 253J-BV.
FIG. 24 is a view identifying the effect of inhibiting the expression of BCL2 and BI-1 genes by the recombinant adenovirus CA101 of the present invention including the hTERT promoter and the double target shRNA expression cassette.
FIG. 25 is a view identifying the effect of inhibiting the expression of AR and mTOR genes by the recombinant adenovirus CA103 of the present invention including the hTERT promoter and the double target shRNA expression cassette in the prostate cancer cell line LNcap.
FIG. 26 is a view identifying *in vitro* the effect of inhibiting the expression of AR and mTOR genes by the recombinant adenovirus CA103 of the present invention including the hTERT promoter and the double target shRNA expression cassette in prostate cancer cell lines C42B and 22Rv1.
FIG. 27 is a view identifying *in vivo* the effect of inhibiting the expression of AR and mTOR genes by the recombinant adenovirus CA103 of the present invention including the hTERT promoter and the double target shRNA expression cassette.
FIG. 28 is a view identifying the effect of inhibiting the expression of c-MET and PD-L1 genes by the recombinant adenovirus CA104 of the present invention including the hTERT promoter and the double target shRNA expression cassette.

FIG. 29 is a view identifying the killing effect of a cancer cell line by the recombinant adenovirus CA101 of the present invention including the hTERT promoter and the double target shRNA expression cassette.

FIG. 30 is a view identifying the killing effect of bladder cancer cell lines RT4, T24 and 253J-BV by the recombinant adenovirus CA102 of the present invention.

FIG. 31 is a view identifying the killing effect of the head and neck cancer cell lines FaDu and HSC-2 by the recombinant adenovirus CA102 of the present invention.

FIG. 32 is a view identifying the killing effect of the skin squamous carcinoma cell lines A431 and HSC-5 by the recombinant adenovirus CA102 of the present invention.

FIG. 33 is a view identifying the killing effect of the cancer cell line LNcap by the recombinant adenovirus CA103 of the present invention including the hTERT promoter and the double target shRNA expression cassette.

FIG. 34 is a view identifying the killing effect of the cancer cell lines C42B and 22Rv1 cell lines by the recombinant adenovirus CA103 of the present invention including the hTERT promoter and the double target shRNA expression cassette.

FIG. 35 is a view identifying the anticancer effect of the recombinant adenovirus CA102 of the present invention on bladder cancer cells (253J-BV) *in vivo.*

FIG. 36 is a view identifying the anticancer effect of the recombinant adenovirus CA102 of the present invention on head and neck cancer cells (FaDu) *in vivo.*

FIG. 37 is a view identifying the anticancer effect of the recombinant adenovirus CA102 of the present invention on tumors (bladder cancer) formed *in vivo.*

FIG. 38 is a view identifying the anticancer effect of CA102 according to the number of administrations on tumors (bladder cancer) formed *in vivo.*

FIG. 39 is a view identifying *in vivo* the glioblastoma treatment effect according to the dose of recombinant adenovirus CA102 of the present invention.

FIG. 40 is a view identifying *in vivo* the prostate cancer treatment effect of the recombinant adenovirus CA103 of the present invention including the hTERT promoter and the double target shRNA expression cassette.

FIG. 41 is a view identifying *in vivo* the bladder cancer treatment effect by the co-treatment with the recombinant adenovirus CA102 of the present invention and the cisplatin.

[Modes of the Invention]

Hereinafter, the present invention will be described in detail by way of embodiments of the present invention. However, the following embodiments are presented as examples of the present invention, and the present invention is not limited thereto. The present invention allows various modifications and applications within the description of the claims to be described later and the scope of equivalents interpreted therefrom.

Unless otherwise indicated, nucleic acids are written left to right in a 5' to 3' orientation. Numeric ranges recited within the specification are inclusive of the numbers defining the range and include each integer or any non-integer fraction within the defined range.

Unless otherwise defined, all technical and scientific terms used herein have the same meaning as commonly understood by those skilled in the art to which the present invention pertains. Although any methods and materials similar or equivalent to those described herein may be used in the practice for testing of the present invention, preferred methods and materials are now described.

In an aspect, an embodiment of the present invention relates to an anti-tumor adenovirus including: a human telomere promoter (hTERT); and an expression cassette including a nucleotide sequence having a first nucleic acid as a target sequence and a nucleotide sequence having a second nucleic acid as a target sequence.

In an embodiment, the nucleotide sequence having the first nucleic acid as the target sequence and the nucleotide sequence targeting the second nucleic acid may be partially or 100% reverse complementary sequences. When expressed in *vivo,* the sequence targeting the first nucleic acid and the sequence targeting the second nucleic acid may form a double strand, preferably shRNA.

In an embodiment, the nucleotide sequence having the first nucleic acid as the target sequence and the nucleotide sequence targeting the second nucleic acid may partially or 100% complementarily bind to form a double strand during gene expression.

In an embodiment, the human telomere promoter may be operably linked with an endogenous gene of an adenovirus.

As used herein, the term "operably linked" refers to a functional linkage between a gene expression regulatory sequence (for example, an array of binding site of promoter, signal sequence, or transcription factor) and different gene sequences, and accordingly, the regulatory sequence regulates the transcription and/or translation of the different gene sequences.

In an embodiment, the hTERT promoter may include the nucleotide sequence represented by SEQ ID NO: 74. In addition, the hTERT promoter sequence may include various known modified sequences.

In an embodiment, the endogenous gene of the adenovirus has a structure of 5'ITR-C1-C2-C3-C4-C5 3'ITR, in which the C1 may include E1A (SEQ ID NO: 75), E1B (SEQ ID NO: 77), or E1A-E1B; in which the C2 may include E2B-L1-L2-L3-E2A-L4; in which the C3 may not include E3 or may include E3; in which the C4 may include L5; and in which the C5 may not include E4 or may include E4, and may include the nucleotide sequence represented by SEQ ID NO: 78.

In an embodiment, the adenovirus may have a partial deletion of an E3 region, and the deleted nucleotide sequence may include the nucleotide sequence represented by SEQ ID NO: 82.

In an embodiment, the expression cassette may be located at a C3 region of the endogenous gene of the adenovirus.

In an embodiment, the hTERT promoter may be operably linked with E1A and E1B of the endogenous gene of the adenovirus.

In an embodiment, an IRES sequence (SEQ ID NO: 76) may be further included between E1A and E1B of the endogenous gene of adenovirus.

In an embodiment, the expression cassette is capable of encoding and expressing shRNA.

In an embodiment, the shRNA may simultaneously inhibit the expression of the first nucleic acid and the second nucleic acid.

In an embodiment, the anti-tumor adenovirus of an embodiment of the present invention may inhibit expression by degrading mRNA of a nucleic acid or inhibiting translation by RNA interference.

In an embodiment, the expression cassette of an embodiment of the present invention is capable of simultaneously inhibiting the first nucleic acid and the second nucleic acid by expressing a double-stranded siRNA in which a sense strand specific for the first nucleic acid or the second nucleic acid and an anti-sense strand specific for the second nucleic acid or the first nucleic acid form partially complementary binding.

As used herein, the term "inhibition of expression" means to lead decline in the expression or translation of a target gene, and preferably means that accordingly the expression of the target gene becomes undetectable or resultantly exists at the meaningless level.

As used herein, the term, "small interfering RNA (siRNA)" means short double-stranded RNA capable of inducing RNA interference (RNAi) phenomenon by cleavage of a specific mRNA. Generally, the siRNA consists of a sense RNA strand having a sequence homologous to the mRNA of the target gene and an antisense RNA strand having a complementary sequence thereof. However, in the double-stranded siRNA of the present invention, the sense RNA strand is siRNA specific for a first nucleic acid or a second nucleic acid (antisense strand to the first nucleic acid or the second nucleic acid), and the antisense RNA strand is siRNA specific for a second nucleic acid or a first nucleic acid (antisense strand to the second nucleic acid or the first nucleic acid), so that the double-stranded siRNA may simultaneously inhibit the expression of the first nucleic acid or the second nucleic acid, respectively.

As used herein, the term "short hairpin RNA (shRNA)" means RNA in which single-stranded RNA may partially contain nucleotide sequences having palindrome to form a double-stranded structure in the 3'-region, thereby having a hairpin-like structure, and after expression in cells, it may be cleaved by dicer, which is one type of RNase present in cells to be converted into siRNA. The length of the double-stranded structure is not particularly limited, but is preferably 10 nucleotides or more, and more preferably 20 nucleotides or more. In the present invention, the shRNA may be included in an expression cassette. The shRNA may be produced by converting U to T into a set sequence consisting of the siRNA antisense strand and the sense strand for each gene, and then connecting TTGGATCCAA (TTGGATCCAA loop) or TTCAAGAGAG (TTCAAGAGAG loop), antisense strand and TT to 3' of the sense strand to prepare an expression cassette encoding shRNA and express the same in cells.

In one embodiment, the first nucleic acid may include a nucleotide sequence having at least 60% complementarity with a reverse complementary sequence of the second nucleic acid, and the second nucleic acid may include a nucleotide sequence having at least 60% complementarity with a reverse complementary sequence of the first nucleic acid.

In an embodiment, the first nucleic acid may include a nucleotide sequence having at least 70%, 80%, 85%, 90%, 95%, 96%, 97%, 98% or 99% complementarity with the reverse complementary sequence of the second nucleic acid. The second nucleic acid may include a nucleotide sequence having at least 70%, 80%, 85%, 90%, 95%, 96%, 97%, 98% or 99% complementarity with the reverse complementary sequence of the first nucleic acid.

Variants of the nucleotide sequence having the first nucleic acid as the target sequence or the nucleotide sequence having the second nucleic acid as the target sequence included in the expression cassette are included within the scope of the present invention. The expression cassette of an embodiment of the present invention is a concept including a functional equivalent of a nucleic acid molecule constituting the same, for example, some nucleotide sequences of a nucleic acid molecule have been modified by deletion, substitution, or insertion, but variants have the same function as the nucleotide sequence molecule. The "% sequence homology" for a nucleic acid molecule is identified by comparing comparative regions between two optimally aligned sequences, and part of the nucleic acid molecule sequence within the comparative regions may include an addition or a deletion (i.e., a gap) compared to the reference sequence (without any addition or deletion) for the optimum arrangement of the two sequences.

In an embodiment, the nucleic acid may be a cancer-related gene.

In an embodiment, the cancer-related gene may be an oncogene whose expression is increased in cancer, and the oncogene may be an apoptosis-related gene, a transcription factor gene, a metastasis-related gene, an angiogenesis-related gene, a cancer cell-specific gene, or a tyrosine-kinase gene.

In an embodiment, the apoptosis-related gene may be ABL1, AKT1, AKT2, BARD1, BAX, BCL11B, BCL2, BCL2A1, BCL2L1, BCL2L12, BCL3, BCL6, BIRC2, BIRC3, BIRC5, BRAF, CARD11, CAV1, CBL, CDC25A, CDKN1A, CFLAR, CNR2, CTNNB1, CUL4A, DAXX, DDIT3, E2F1, E2F3, E2F5, ESPL1, FOXO1, HDAC1, HSPA5, IGF1R, IGF2, JUN, JUNB, JUND, MALT1, MAP3K7, MCL1, MDM2, MDM4, MYB, MYC, NFKB2, NPM1, NTRK1, PAK1, PAX3, PML, PRKCA, PRKCE, PTK2B, RAF1, RHOA, TGFB1, TNFRSF1B, TP73, TRAF6, YWHAG, YWHAQ, or YWHAZ; the transcription factor gene may be AR, ARID3A, ASCL1, ATF1, ATF3, BCL11A, BCL11B, BCL3, BCL6, CDC5L, CDX2, CREB1, CUX1, DDIT3, DLX5, E2F1, E2F3, E2F5, ELF4, ELK1, ELK3, EN2, ERG, ETS1, ETS2, ETV1, ETV3, ETV4, ETV6, FEV, FEZF1, FLI1, FOS, FOSL1, FOXA1, FOXG1, FOXM1, FOXO1, FOXP1, FOXQ1, GATA1, GATA6, GFI1, GFI1B, GLI1, GLI2, GLI3, HES6, HHEX, HLF, HMGA1, HMGA2, HOXA1, HOXA9, HOXD13, HOXD9, ID1, ID2, IKZF1, IRF2, IRF4, JUN, JUNB, JUND, KAT6A, KDM2A, KDM5B, KLF2, KLF4, KLF5, KLF6, KLF8, KMT2A, LEF1, LHX1, LMX1B, MAF, MAFA, MAFB, MBD1, MECOM, MEF2C, MEIS1, MITF, MYB, MYC, MYCL, MYCN, NANOG, NCOA3, NFIB, NFKB2, NKX2-1, OTX2, PATZ1, PAX2, PAX3, PAX4, PAX8, PBX1, PBX2, PITX2, PLAG1, PLAGL2, PPARG, PPP1R13L, PRDM10, PRDM13, PRDM14, PRDM15, PRDM16, PRDM6, PRDM8, PRDM9, RARA, REL, RERE, RUNX1, RUNX3, SALL4, SATB1, SFPQ, SIX1, SNAI1, SOX2, SOX4, SPI1, SREBF1, STAT3, TAF1, TAL1, TAL2, TBX2, TBX3, TCF3, TFCP2, TFE3, THRA, TLX1, TP63, TP73, TWIST1, WT1, YBX1, YY1, ZBTB16, ZBTB7A, ZIC2, ZNF217, or ZNF268; the metastasis-related gene may be AKT1, AKT2, AR, CBL, CDH1, CRK, CSF1, CTNNB1, CTTN, CXCR4, EGFR, FGFR1, FLT3, FYN, GLI1, ILK, ITGA3, JAK2, MET, PDGFRB, PLXNB1, PRKCI, PTCH1, PTPN11, RAC1, RHOA, RHOC, ROCK1, SMO, SNAI1, SRC, TCF3, or WT1; the angiogenesis-related gene may be BRAF, CAV1, CTGF, EGFR, ERBB2, ETS1, FGF4, FGF6, FGFR1, FGFR3, FGFR4, ID1, NRAS, PDGFB, PDGFRA, PDGFRB, or SPARC; and the tyrosine-kinase gene may be ABL1, ABL2, ALK, AXL, BLK, EGFR, EPHA2, ERBB2, ERBB3, ERBB4, FES, FGFR1, FGFR2, FGFR3, FGFR4, FGR, FLT3, FYN, ITK, JAK1, JAK2, KIT, LCK, MERTK, MET, MST1R, NTRK1, NTRK3, PDGFRA, PDGFRB, PTK2B, PTK7, RET, ROS1, SRC, SYK, TEC, or YES1.

In an embodiment, the oncogene may be SEPTIN9, ACOD1, ACTN4, ADAM28, ADAM9, ADGRF1, ADRBK2, AFF1, AFF3, AGAP2, AGFG1, AGRN, AHCYL1, AHI1, AIMP2, AKAP13, AKAP9, AKIRIN2, AKTIP, ALDH1A1, ALL1, ANIB1, ANP32C, ANP32D, AQP1, ARAF, ARHGEF1, ARHGEF2, ARHGEF5, ASPSCR1, AURKA, BAALC, BAIAP2L1, BANP, BCAR4, BCKDHB, BCL9, BCL9L, BCR, BMI1, BMP7, BOC, BRD4, BRF2, CABIN1, CAMK1D, CAPG, CBFB, CBLB, CBLL1, CBX7, CBX8, CCDC28A, CCDC6, CCNB1, CCNB2, CCND1, CCNE1, CCNL1, CD24, CDC25C, CDC6, CDH17, CDK1, CDK14, CDK4, CDK5R2, CDK6, CDK8, CDKN1B, CDKN3, CDON, CEACAM6, CENPW, CHD1L, CHIC1, CHL1, CKS1B, CMC4, CNTN2, COPS3, COPS5, CRKL, CRLF2, CROT, CRTC1, CRYAB, CSF1R, CSF3, CSF3R, CSNK2A1, CSNK2A2, CT45A1, CTBP2, CTNND2, CTSZ, CUL7, CXCL1, CXCL2, CXCL3, CYGB, CYP24A1, DCD, DCUN1D1DDB2, DDHD2, DDX6, DEK, DIS3, DNPH1, DPPA2, DPPA4, DSG3, DUSP12, DUSP26, ECHS1, ECT2, EEF1A1, EEF1A2, EEF1D, EIF3E, EIF3I, EIF4E, EIF5A2, ELAVL1, ELL, EML4, EMSY, ENTPD5, EPCAM, EPS8, ERAS, ERGIC1, ERVW-1, EVI2A, EVI5, EWSR1, EZH2, FAM189B, FAM72A, FAM83D, FASN, FDPS, FGF10, FGF3, FGF5, FGF8, FR1OP, FHL2, FIP1L1, FNDC3B, FRAT1, FUBP1, FUS, FZD2, GAB2, GAEC1, GALNT10, GALR2, GLO1, GMNN, GNA12, GNA13, GNAI2, GNAQ, GNAS, GOLPH3, GOPC, GPAT4, GPM6A, GPM6B, GPR132, GREM1, GRM1, GSK3A, GSM1, H19, HAS1, HAX1, HDGFRP2, HMGN5, HNRNPA1, HOTAIR, HOTTIP, HOXA-AS2, HRAS, HSPA1A, HSPA4, HSPB1, HULC, IDH1, IFNG, IGF2BP1, IKBKE, IL7R, INPPL1, INTS1, INTS2, INTS3, INTS4, INTS5, INTS7, INTS8, IRS2, IST1, JUP, KDM4C, KIAA0101, KIAA1524, KIF14, KRAS, KSR2, LAMTOR5, LAPTM4B, LCN2, LDHB, LETMD1, LIN28A, LIN28B, LMO1, LMO2, LMO3, LMO4, LSM1, LUADT1, MACC1, MACROD1, MAGEA11, MALAT1, MAML2, MAP3K8, MAPRE1, MAS1, MCC, MCF2, MCF2L, MCTS1, MEFV, MFHAS1, MFNG, MIEN1, MINA, MKL2, MLANA, MLLT1, MLLT11, MLLT3, MLLT4, MMP12, MMS22L, MN1, MNAT1, MOS, MPL, MPST, MRAS, MRE11A, MSI1, MTCP1, MTDH, MTOR, MUC1, MUC4, MUM1, MYD88, NAAA, NANOGP8, NBPF12, NCOA4, NEAT1, NECTIN4, NEDD4, NEDD9, NET1, NINL, NME1, NOTCH1, NOTCH4, NOV, NSD1, NUAK2, NUP214, NUP98, NUTM1, OLR1, PA2G4, PADI2, PAK7, PARK7, PARM1, PBK, PCAT1, PCAT5, PDGFA, PDZK1IP1, PELP1, PFN1P3, PIGU, PIK3CA, PIK3R1, PIM1, PIM2, PIM3, PIR, PIWIL1, PLAC8, PLK1, PPM1D, PPP1R10, PPP1R14A, PPP2R1A, PRAME, PRDM12, PRMT5, PSIP1, PSMD10, PTCH2, PTMA, PTP4A1, PTP4A2, PTP4A3, PTTG1, PTTG1IP, PTTG2, PVT1, RAB11A, RAB18, RAB22A, RAB23, RAB8A, RALGDS, RAP1A, RASSF1, RBM14, RBM15, RBM3, RBMY1A1, RFC3, RGL4, RGR, RHO, RING1, RINT1, RIT1, RNF43, RPL23, RRAS, RRAS2, RSF1, RUNX1T1, S100A4, S100A7, S100A8, SAG, SART3, SBSN, SEA, SEC62, SERTAD1, SERTAD2, SERTAD3, SET, SETBP1, SETDB1, SGK1, SIRT1, SIRT6, SKI, SKIL, SKP2, SLC12A5, SLC3A2, SMR3B, SMURF1, SNCG, SNORA59A, SNORA80E, SPAG9, SPATA4, SPRY2, SQSTM1, SRSF1, SRSF2, SRSF3, SRSF6, SS18, SSX1, SSX2, SSX2B, STIL, STMN1, STRA6, STYK1, SUZ12, SWAP70, SYT1, TAC1, TACSTD2, TAF15, TALDO1, TAZ, TBC1D1, TBC1D15, TBC1D3, TBC1D3C, TBC1D7, TCL1A, TCL1B, TCL6, TCP1, TFG, TGM3, TINCR, TKTL1, TLE1, TMEM140, TMPOP2, TMPRSS2, TNS4, TPD52, TPR, TRE17, TREH, TRIB1, TRIB2, TRIM28, TRIM32, TRIM8, TRIO, TRIP6, TSPAN1, TSPY1, TXN, TYMS, TYRP1, UBE2C, UBE3C, UCA1, UCHL1, UHRF1, URI1, USP22, USP4, USP6, VAV1, VAV2, VAV3, VIM, WAPL, WHSC1, WHSC1L1, WISP1, WNT1, WNT10A, WNT10B, WNT2, WNT3, WNT5A, WWTR1, XCL1, XIAP, YAP1, YEATS4, YY1AP1, ZEB1-AS1, ZFAND4, ZFAS1, ZMYM2, ZNF703, or ZNHIT6.

In an embodiment, the cancer cell specific gene may be programmed death-ligand 1 (PD-L1) expressed on the surface of tumor cells.

In an embodiment, the first nucleic acid and the second nucleic acid targeted by the transcripts of the expression cassette of an embodiment of the present invention may be each different nucleic acid selected respectively from the group consisting of ABL1, AKT1, AKT2, BARD1, BAX, BCL11B, BCL2, BCL2A1, BCL2L1, BCL2L12, BCL3, BCL6, BIRC2, BIRC3, BIRC5, BRAF, CARD11, CAV1, CBL, CDC25A, CDKN1A, CFLAR, c-MET, CNR2, CTNNB1, CUL4A, DAXX, DDIT3, E2F1, E2F3, E2F5, ESPL1, FOXO1, HDAC1, HSPA5, IGF1R, IGF2, JUN, JUNB, JUND, MALT1, MAP3K7, MCL1, MDM2, MDM4, MYB, MYC, NFKB2, NPM1, NTRK1, PAK1, PAX3, PML, PRKCA, PRKCE, PTK2B, RAF1, RHOA, TGFB1, TNFRSF1B, TP73, TRAF6, YWHAG, YWHAQ, YWHAZ, AR, ARID3A, ASCL1, ATF1, ATF3, BCL11A, BCL11B, BCL3, BCL6, CDC5L, CDX2, CREB1, CUX1, DDIT3, DLX5, E2F1, E2F3, E2F5, ELF4, ELK1, ELK3, EN2, ERG, ETS1, ETS2, ETV1, ETV3, ETV4, ETV6, FEV, FEZF1, FLI1, FOS, FOSL1, FOXA1, FOXG1, FOXM1, FOXO1, FOXP1, FOXQ1, GATA1, GATA6, GFI1, GFI1B, GLI1, GLI2, GLI3, HES6, HHEX, HLF, HMGA1, HMGA2, HOXA1, HOXA9, HOXD13, HOXD9, ID1, ID2, IKZF1, IRF2, IRF4, JUN, JUNB, JUND, KAT6A, KDM2A, KDM5B, KLF2, KLF4, KLF5, KLF6, KLF8, KMT2A, LEF1, LHX1, LMX1B, MAF, MAFA, MAFB, MBD1, MECOM, MEF2C, MEIS1, MITF, MYB, MYC, MYCL, MYCN, NANOG, NCOA3, NFIB, NFKB2, NKX2-1, OTX2, PATZ1, PAX2, PAX3, PAX4, PAX8, PBX1, PBX2, PD-L1, PITX2, PLAG1, PLAGL2, PPARG, PPP1R13L, PRDM10, PRDM13, PRDM14, PRDM15, PRDM16, PRDM6, PRDM8, PRDM9, RARA, REL, RERE, RUNX1, RUNX3, SALL4, SATB1, SFPQ, SIX1, SNAI1, SOX2, SOX4, SPI1, SREBF1, STAT3, TAF1, TAL1, TAL2, TBX2, TBX3, TCF3, TFCP2, TFE3, THRA, TLX1, TP63, TP73, TWIST1, WT1, YBX1, YY1, ZBTB16, ZBTB7A, ZIC2, ZNF217, ZNF268, AKT1, AKT2, AR, CBL, CDH1, CRK, CSF1, CTNNB1, CTTN, CXCR4, EGFR, FGFR1, FLT3, FYN, GLI1, ILK, ITGA3, JAK2, MET, PDGFRB, PLXNB1, PRKCI, PTCH1, PTPN11, RAC1, RHOA, RHOC, ROCK1, SMO, SNAI1, SRC, TCF3, WT1, BRAF, CAV1, CTGF, EGFR, ERBB2, ETS1, FGF4, FGF6, FGFR1, FGFR3, FGFR4, ID1, NRAS, PDGFB, PDGFRA, PDGFRB, SPARC, ABL1, ABL2, ALK, AXL, BLK, EGFR, EPHA2, ERBB2, ERBB3, ERBB4, FES, FGFR1, FGFR2, FGFR3, FGFR4, FGR, FLT3, FYN, ITK, JAK1, JAK2, KIT, LCK, MERTK, MET, MST1R, NTRK1, NTRK3, PDGFRA, PDGFRB, PTK2B, PTK7, RET, ROS1, SRC, SYK, TEC, YES1, SEPTIN9, ACOD1, ACTN4, ADAM28, ADAM9, ADGRF1, ADRBK2, AFF1, AFF3, AGAP2, AGFG1, AGRN, AHCYL1, AHI1, AIMP2, AKAP13, AKAP9, AKIRIN2, AKTIP, ALDH1A1, ALL1, ANIB1, ANP32C, ANP32D, AQP1, ARAF, ARHGEF1, ARHGEF2, ARHGEF5, ASPSCR1, AURKA, BAALC, BAIAP2L1, BANP, BCAR4, BCKDHB, BCL9, BCL9L, BCR, BMI1, BMP7, BOC, BRD4, BRF2, CABIN1, CAMK1D, CAPG, CBFB, CBLB, CBLL1, CBX7, CBX8, CCDC28A, CCDC6, CCNB1, CCNB2, CCND1, CCNE1, CCNL1, CD24, CDC25C, CDC6, CDH17, CDK1, CDK14, CDK4, CDK5R2, CDK6, CDK8, CDKN1B, CDKN3, CDON, CEACAM6, CENPW, CHD1L, CHIC1, CHL1, CKS1B, CMC4, CNTN2, COPS3, COPS5, CRKL, CRLF2, CROT, CRTC1, CRYAB, CSF1R, CSF3, CSF3R, CSNK2A1, CSNK2A2, CT45A1, CTBP2, CTNND2, CTSZ, CUL7, CXCL1, CXCL2, CXCL3, CYGB, CYP24A1, DCD, DCUN1D1DDB2, DDHD2, DDX6, DEK, DIS3, DNPH1, DPPA2, DPPA4, DSG3, DUSP12, DUSP26, ECHS1, ECT2, EEF1A1, EEF1A2, EEF1D, EIF3E, EIF3I, EIF4E, EIF5A2, ELAVL1, ELL, EML4, EMSY, ENTPD5, EPCAM, EPS8, ERAS, ERGIC1, ERVW-1, EVI2A, EVI5, EWSR1, EZH2, FAM189B, FAM72A, FAM83D, FASN, FDPS, FGF10, FGF3, FGF5, FGF8, FR1OP, FHL2, FIP1L1, FNDC3B, FRAT1, FUBP1, FUS, FZD2, GAB2, GAEC1, GALNT10, GALR2, GLO1, GMNN, GNA12, GNA13, GNAI2, GNAQ, GNAS, GOLPH3, GOPC, GPAT4, GPM6A, GPM6B, GPR132, GREM1, GRM1, GSK3A, GSM1, H19, HAS1, HAX1, HDGFRP2, HMGN5, HNRNPA1, HOTAIR, HOTTIP, HOXA-AS2, HRAS, HSPA1A, HSPA4, HSPB1, HULC, IDH1, IFNG, IGF2BP1, IKBKE, IL7R, INPPL1, INTS1, INTS2, INTS3, INTS4, INTS5, INTS7, INTS8, IRS2, IST1, JUP, KDM4C, KIAA0101, KIAA1524, KIF14, KRAS, KSR2, LAMTOR5, LAPTM4B, LCN2, LDHB, LETMD1, LIN28A, LIN28B, LMO1, LMO2, LMO3, LMO4, LSM1, LUADT1, MACC1, MACROD1, MAGEA11, MALAT1, MAML2, MAP3K8, MAPRE1, MAS1, MCC, MCF2, MCF2L, MCTS1, MEFV, MFHAS1, MFNG, MIEN1, MINA, MKL2, MLANA, MLLT1, MLLT11, MLLT3, MLLT4, MMP12, MMS22L, MN1, MNAT1, MOS, MPL, MPST, MRAS, MRE11A, MSI1, MTCP1, MTDH, MTOR, MUC1, MUC4, MUM1, MYD88, NAAA, NANOGP8, NBPF12, NCOA4, NEAT1, NECTIN4, NEDD4, NEDD9, NET1, NINL, NME1, NOTCH1, NOTCH4, NOV, NSD1, NUAK2, NUP214, NUP98, NUTM1, OLR1, PA2G4, PADI2, PAK7, PARK7, PARM1, PBK, PCAT1, PCAT5, PD-L1, PDGFA, PDZK1IP1, PELP1, PFN1P3, PIGU, PIK3CA, PIK3R1, PIM1, PIM2, PIM3, PIR, PIWIL1, PLAC8, PLK1, PPM1D, PPP1R10, PPP1R14A, PPP2R1A, PRAME, PRDM12, PRMT5, PSIP1, PSMD10, PTCH2, PTMA, PTP4A1, PTP4A2, PTP4A3, PTTG1, PTTG1IP, PTTG2, PVT1, RAB11A, RAB18, RAB22A, RAB23, RAB8A, RALGDS, RAP1A, RASSF1, RBM14, RBM15, RBM3, RBMY1A1, RFC3, RGL4, RGR, RHO, RING1, RINT1, RIT1, RNF43, RPL23, RRAS, RRAS2, RSF1, RUNX1T1, S100A4, S100A7, S100A8, SAG, SART3, SBSN, SEA, SEC62, SERTAD1, SERTAD2, SERTAD3, SET, SETBP1, SETDB1, SGK1, SIRT1, SIRT6, SKI, SKIL, SKP2, SLC12A5, SLC3A2, SMR3B, SMURF1, SNCG, SNORA59A, SNORA80E, SPAG9, SPATA4, SPRY2, SQSTM1, SRSF1, SRSF2, SRSF3, SRSF6, SS18, SSX1, SSX2, SSX2B, STIL, STMN1, STRA6, STYK1, SUZ12, SWAP70, SYT1, TAC1, TACSTD2, TAF15, TALDO1, TAZ, TBC1D1, TBC1D15, TBC1D3, TBC1D3C, TBC1D7, TCL1A, TCL1B, TCL6, TCP1, TFG, TGM3, TINCR, TKTL1, TLE1, TMEM140, TMPOP2, TMPRSS2, TNS4, TPD52, TPR, TRE17, TREH, TRIB1, TRIB2, TRIM28, TRIM32, TRIM8, TRIO, TRIP6, TSPAN1, TSPY1, TXN, TYMS, TYRP1, UBE2C, UBE3C, UCA1, UCHL1, UHRF1, URI1, USP22, USP4, USP6, VAV1, VAV2, VAV3, VIM, WAPL, WHSC1, WHSC1L1, WISP1, WNT1, WNT10A, WNT10B, WNT2, WNT3, WNT5A, WWTR1, XCL1, XIAP, YAP1, YEATS4, YY1AP1, ZEB1-AS1, ZFAND4, ZFAS1, ZMYM2, ZNF703, and ZNHIT6.

In an embodiment, the first nucleic acid may be a signal transducer and activator of transcription 3 (STAT3), and the second nucleic acid may be a mammalian target of rapamycin (mTOR), in which case the expression cassette may include a nucleic acid in which U is converted into T in the nucleotide sequence represented by SEQ ID NOS: 1 and 2, SEQ ID NOS: 3 and 4, SEQ ID NOS: 5 and 6, SEQ ID NOS: 7 and 8, SEQ ID NOS: 9 and 10, SEQ ID NOS: 11 and 12, SEQ ID NOS: 13 and 14, SEQ ID NOS: 15 and 16, or SEQ ID NOS: 17 and 18. In the above, 17mer of 21mer in siRNA represented by SEQ ID NOS: 1 and 2, 16mer of 20mer in siRNA represented by SEQ ID NOS: 3 and 4, 15mer of 19mer in siRNA represented by SEQ ID NOS: 5 and 6, 14mer of 18mer in siRNA represented by SEQ ID NOS: 7 and 8, 16mer of 17mer in siRNA represented by SEQ ID NOS: 9 and 10, 17mer of 20mer in siRNA represented by SEQ ID NOS: 11 and 12, 16mer of 19mer in siRNA represented by SEQ ID NOS: 13 and 14, 15mer of 18mer in siRNA represented by SEQ ID NOS: 15 and 16, and 14mer of 17mer in siRNA represented by SEQ ID NOS: 17 and 18 may be complementarily linked. In addition, when the first nucleic acid is STAT3 and the second nucleic acid is mTOR, the shRNA expression DNA (DNA sequence encoding STAT3 and mTOR double target shRNA) included in the expression cassette may include the nucleotide sequence represented by SEQ ID NO: 66 or 67.

In an embodiment, the first nucleic acid may be B-cell lymphoma 2 (BCL2), and the second nucleic acid may be BAX inhibitor 1 (BI-1), in which case the expression cassette may include the nucleotide sequences represented by SEQ ID NOS: 19 and 20, SEQ ID NOS: 21 and 22, SEQ ID NOS: 23 and 24, SEQ ID NOS: 25 and 26, SEQ ID NOS: 27 and 28, and SEQ ID NOS: 29 and 30. In the above, siRNA set 10 of 21mer composed of the SEQ ID NOS: 19 and 20 has a complementary binding length of 15mer therebetween. siRNA set 11 of 20mer composed of the SEQ ID NOS: 21 and 22 has a complementary binding length of 14mer therebetween. siRNA set 12 of 20mer composed of the SEQ ID NOS: 23 and 24 has a complementary binding length of 14mer therebetween. siRNA set 13 of 19mer composed of the SEQ ID NOS: 25 and 26 has a complementary binding length of 13mer therebetween. siRNA set 14 of 19mer composed of the SEQ ID NOS: 27 and 28 has a complementary binding length of 13mer therebetween. siRNA set 15 of 18mer composed of the SEQ ID NOS: 29 and 30 has a complementary binding length of 12mer therebetween. siRNA (Antisense Bcl-2) represented by SEQ ID NO: 19, 21, 23, 25, 27 or 29 of Table 2 below may be complementarily linked with the mRNA of Bcl-2, and siRNA (Antisense BI-1) represented by SEQ ID NO: 20, 22, 24, 26, 28, or 30 may be complementarily linked with the mRNA of BI-1. In addition, when the first nucleic acid is BCL2 and the second nucleic acid is BI-1, the shRNA expression DNA included in the expression cassette may include the nucleotide sequence represented by SEQ ID NO: 68 or 69.

In an embodiment, the first nucleic acid may be an androgen receptor (AR), and the second nucleic acid may be a mammalian target of rapamycin (mTOR), in which case the expression cassette may include the nucleotide sequences represented by SEQ ID NOS: 31 and 32, SEQ ID NOS: 33 and 34, SEQ ID NOS: 35 and 36, SEQ ID NOS: 37 and 38, SEQ ID NOS: 39 and 40, SEQ ID NOS: 41 and 42, SEQ ID NOS: 43 and 44, SEQ ID NOS: 45 and 46, SEQ ID NOS: 47 and 48, SEQ ID NOS: 49 and 50, SEQ ID NOS: 51 and 52, SEQ ID NOS: 53 and 54, or SEQ ID NOS: 55 and 56. In the above, siRNA set 16 of 20mer composed of the SEQ ID NOS: 31 and 32 has a complementary binding length of 18mer therebetween. siRNA set 17 of 19mer composed of the SEQ ID NOS: 33 and 34 has a complementary binding length of 17mer therebetween. siRNA set 18 of 18mer composed of the SEQ ID NOS: 35 and 36 has a complementary binding length of 16mer therebetween. siRNA set 19 of 17mer composed of the SEQ ID NOS: 37 and 38 has a complementary binding length of 15mer therebetween. siRNA set 20 of 19mer composed of the SEQ ID NOs: 39 and 40 has a complementary binding length of 15mer therebetween. siRNA set 21 of 18mer composed of the SEQ ID NOS: 41 and 42 has a complementary binding length of 14mer therebetween. siRNA set 22 of 17mer composed of the SEQ ID NOS: 43 and 44 has a complementary binding length of 13mer therebetween. siRNA set 23 of 23mer composed of the SEQ ID NOS: 45 and 46 has a complementary binding length of 19mer therebetween. siRNA set 24 of 22mer composed of the SEQ ID NOS: 47 and 48 has a complementary binding length of 18mer therebetween. siRNA set 25 of 22mer composed of the SEQ ID NOS: 49 and 50 has a complementary binding length of 18mer therebetween. siRNA set 26 of 21mer composed of the SEQ ID NOS: 51 and 52 has a complementary binding length of 17mer therebetween. siRNA set 27 of 20mer composed of the SEQ ID NOS: 53 and 54 has a complementary binding length of 16mer therebetween. And siRNA set 28 of 21mer composed of the SEQ ID NOS: 55 and 56 has a complementary binding length of 17mer therebetween. siRNA (Antisense AR) represented by SEQ ID NO: 31, 33, 35, 37, 39, 41, 43, 45, 47, 49, 51, 53, or 55 may be complementarily linked with the mRNA of AR, and siRNA (Antisense mTOR) represented by SEQ ID NO: 32, 34, 36, 38, 40, 42, 44, 46, 48, 50, 52, 54, or 56 may be complementarily linked with the mRNA of mTOR. In addition, when the first nucleic acid is AR and the second nucleic acid is mTOR, the shRNA expression DNA included in the expression cassette may include the nucleotide sequence represented by SEQ ID NO: 70 or 71.

In an embodiment, the first nucleic acid may be MGMT (O-6-methylguanine-DNA methyltransferase), and the second nucleic acid may be mTOR, in which case the expression cassette may include the nucleotide sequences represented by SEQ ID NOS: 57 and 58.

In an embodiment, the first nucleic acid may be BCL2, and the second nucleic acid may be MCL1 (MCL1 apoptosis regulator), in which case the expression cassette may include the nucleotide sequences represented by SEQ ID NOS: 59 and 60.

In an embodiment, the first nucleic acid may be STAT3, and the second nucleic acid may be TFEB (transcription factor EB), in which case the expression cassette may include the nucleotide sequences represented by SEQ ID NOS: 61 and 62.

In an embodiment, the first nucleic acid may be c-MET (Homo sapiens MET proto-oncogene), and the second nucleic acid may be PD-L1 (Programmed death-ligand 1), in which case the expression cassette may include a nucleic acid in which U is converted into T in the nucleotide sequences represented by SEQ ID NOS: 63 and 64. In the above, 15mer of 19mer in siRNA represented by SEQ ID NOS: 63 and 64 may be complementarily linked. In addition, when the first nucleic acid is c-MET and the second nucleic acid is PD-L1, the shRNA expression DNA included in the expression cassette may include the nucleotide sequence represented by SEQ ID NO: 72 or 73.

In an embodiment, the expression cassette may include a nucleotide sequence sequentially encoding a nucleotide sequence having a first nucleic acid as a target sequence, a loop sequence capable of forming a hairpin structure, and a nucleotide sequence having a second nucleic acid as a target sequence.

In an embodiment, an expression of the expression cassette may be regulated by a U6 promoter.

In an embodiment, the adenovirus may be an adenovirus with a serotype 5 of group C.

In an embodiment, the anti-tumor virus of an embodiment of the present invention may have a high oncolytic ability compared to a wild-type adenovirus, and may have a high oncolytic ability compared to an adenovirus in which the hTERT promoter is introduced into the wild-type adenovirus.

In an aspect, an embodiment of the present invention relates to a composition for treating cancer including the anti-tumor virus of an embodiment of the present invention.

In an embodiment, the composition of an embodiment of the present invention may further include an anticancer agent, for example, acivicin, aclarubicin, acodazole, achromycin, adozelesin, alanosine, aldesleukin, allopurinol sodium, altretamine, aminoglutethimide, amonafide, ampligen, amsacrine, androgens, anguidine, aphidicolin glycinate, asaley, asparaginase, 5-azacitidine, azathioprine, Bacillus Calmette-Guerin (BCG), Baker's antifol, β-2-deoxythioguanosine, bisantrene HCl, bleomycin sulfate, busulfan, buthionine sulfoximine, BWA 773U82, BW 502U83.HCl, BW 7U85 mesylate, ceracemide, carbetimer, carboplatin, carmustine, chlorambucil, chloroquinoxaline-sulfonamide, chlorozotocin, chromomycin A3, cisplatin, cladribine, corticosteroids, Corynebacterium parvum, CPT-11, crisnatol, cyclocytidine, cyclophosphamide, cytarabine, cytembena, dabis maleate, dacarbazine, dactinomycin, daunorubicin HCl, deazauridine, dexrazoxane, dianhydrogalactitol, diaziquone, dibromodulcitol, didemnin B, diethyldithiocarbamate, diglycoaldehyde, dihydro-5-azacytidine, doxorubicin, echinomycin, dedatrexate, edelfosine, eflornithine, Elliott's solution, elsamitrucin, epirubicin, esorubicin, estramustine phosphate, estrogens, etanidazole, ethiofos, etoposide, fadrazole, fazarabine, fenretinide, filgrastim, finasteride, flavone acetic acid, floxuridine, fludarabine phosphate, 5'-fluorouracil, Fluosol™, flutamide, gallium nitrate, gemcitabine, goserelin acetate, hepsulfam, hexamethylene bisacetamide, homoharringtonine, hydrazine sulfate, 4-hydroxyandrostenedione, hydrozyurea, idarubicin HCl, ifosfamide, 4-ipomeanol, iproplatin, isotretinoin, leucovorin calcium, leuprolide acetate, levamisole, liposome daunorubicin, liposome-encapsulated doxorubicin, lomustine, lonidamine, maytansine, mechlorethamine hydrochloride, melphalan, menogaril, merbarone, 6-mercaptopurine, mesna, methanol extraction of Bacillus Calmette-Guerin, methotrexate, N-methylformamide, mifepristone, mitoguazone, mitomycin-C, mitotane, mitoxantrone hydrochloride, monocyte/macrophage colony-stimulating factor, nabilone, nafoxidine, neocarzinostatin, octreotide acetate, ormaplatin, oxaliplatin, paclitaxel, pala, pentostatin, piperazinedione, pipobroman, pirarubicin, piritrexim, piroxantrone hydrochloride, PIXY-321, plicamycin, porfimer sodium, prednimustine, procarbazine, progestins, pyrazofurin, razoxane, sargramostim, semustine, spirogermanium, spiromustine, streptonigrin, streptozocin, sulofenur, suramin sodium, tamoxifen, taxotere, tegafur, teniposide, terephthalamidine, teroxirone, thioguanine, thiotepa, thymidine injection, tiazofurin, topotecan, toremifene, tretinoin, trifluoperazine hydrochloride, trifluridine, trimetrexate, tumor necrosis factor (TNF), uracil mustard, vinblastine sulfate, vincristine sulfate, vindesine, vinorelbine, vinzolidine, Yoshi 864, zorubicin, cytosine arabinoside, etoposide, melphalan, taxol and mixtures thereof. It includes preferably cisplatin, paclitaxel, 5-fluorouracil (5-FU), methotrexate, doxorubicin, daunorubicin, cytosine arabinoside, etoposide, melphalan, chlorambucil, cyclophosphamide, vindesine, mitomycin, bleomycin, tamoxifen, and taxol, and more preferably cisplatin, paclitaxel, 5-fluorouracil (5-FU), but is not limited thereto in order to achieve the object of showing a synergistic effect on the anticancer effect by co-treating with the composition of an embodiment of the present invention.

The cancer may be any one selected from the group consisting of colon cancer, breast cancer, uterine cancer, cervical cancer, ovarian cancer, prostate cancer, brain tumor, head and neck carcinoma, melanoma, myeloma, leukemia, lymphoma, gastric cancer, lung cancer, pancreatic cancer, non-small cell lung cancer, liver cancer, esophageal cancer, small intestine cancer, anal cancer, fallopian tube cancer, endometrial cancer, vaginal cancer, vulva cancer, Hodgkin lymphoma, bladder cancer, kidney cancer, ureter cancer, kidney cell carcinoma, kidney pelvic carcinoma, bone cancer, skin cancer, head cancer, cervical cancer, skin melanoma, choroidal melanoma, endocrine gland cancer, thyroid carcinoma, parathyroid gland cancer, adrenal cancer, soft tissue sarcoma, urethral cancer, penile cancer, central nervous system (CNS) tumor, primary CNS lymphoma, spinal cord tumor, polymorphic glioblastoma and pituitary adenoma.

As used herein, the term "promoter" refers to an untranslated nucleic acid sequence usually located upstream of the coding region, which contains the binding site for RNA polymerase and initiates transcription of the gene downstream of the promoter into mRNA. In the expression cassette of the present invention, any promoter may be used, as long as it is able to initiate shRNA expression. Specifically, the promoter of the present invention may be a constitutive promoter which constitutively induces the expression of a target gene, or an inducible promoter which induces the expression of a target gene at a specific site and a specific time, and examples thereof include a U6 promoter, an H1 promoter, a CMV (cytomegalovirus) promoter, a SV40 promoter, a CAG promoter (Hitoshi Niwa et al., Gene, 108:193-199, 1991), a CaMV 35S promoter (Odell et al., Nature 313:810-812, 1985), a Rsyn7 promoter (U.S. patent application Ser. No. 08/991,601), a rice actin promoter (McElroy et al., Plant Cell 2:163-171, 1990), ubiquitin promoter (Christensen et al., Plant Mol. Biol. 12:619-632, 1989), an ALS promoter (U.S. Patent Application Ser. No. 08/409,297), and the like. Additionally, any known promoter apparent to those skilled in the art, such as promoters disclosed in U.S. Patent Nos. 5,608,149, 5,608,144, 5,604,121, 5,569,597, 5,466,785, 5,399,680, 5,268,463 and 5,608,142, may be used without limitation. Preferably, the promoter of the present invention may be a U6 promoter, an H1 promoter, or a CMV promoter. According to one preferred embodiment of the present invention, a U6 promoter may be used.

The composition of an embodiment of the present invention may further include an adjuvant. The adjuvant may be used without limitation as long as it is known in the pertinent technical field. However, it may further include, for example, Freund's complete or incomplete adjuvants to enhance its effectiveness.

The composition according to the present invention may be produced in the form of incorporation of an active ingredient into a pharmaceutically acceptable carrier. In this regard, the pharmaceutically acceptable carrier includes a carrier, excipient and diluent commonly used in the pharmaceutical field. Pharmaceutically acceptable carriers for use in the compositions of the present invention include, but are not limited to, lactose, dextrose, sucrose, sorbitol, mannitol, xylitol, erythritol, maltitol, starch, acacia rubber, alginate, gelatin, calcium phosphate, calcium silicate, cellulose, methylcellulose, polyvinylpyrrolidone, water, methyl hydroxybenzoate, propyl hydroxybenzoate, talc, magnesium stearate and mineral oil.

The composition of an embodiment of the present invention may be formulated in the form of oral preparations such as powder, granule, tablet, capsule, suspension, emulsion, syrup or aerosol, external preparation, suppositories or sterilized injection solutions according to each conventional method.

Formulations may be prepared by using generally used excipients or diluents such as fillers, extenders, binders, wetting agents, disintegrating agents and surfactant. Solid formulations for oral administration include tablets, pills, powders, granules and capsules. These solid formulations are prepared by mixing one or more excipients such as starch, calcium carbonate, sucrose, lactose and gelatin to active ingredients. Except for the simple excipients, lubricants, for example magnesium stearate, and talc may be used. Liquid formulations for oral administrations include suspensions, solutions, emulsions and syrups, and the above-mentioned formulations may include various excipients such as wetting agents, sweeteners, aromatics and preservatives in addition to generally used simple diluents such as water and liquid paraffin. Formulations for parenteral administration include sterilized aqueous solutions, water-insoluble excipients, suspensions, emulsions, lyophilized preparations and suppositories. Water-insoluble excipients and suspensions may include propylene glycol, polyethylene glycol, vegetable oil like olive oil, injectable ester like ethylolate, etc. Suppositories may include witepsol, tween 61, cacao butter, laurin butter, glycerogelatin, etc.

The composition according to an embodiment of the present invention may be administered to a subject by various routes. All modes of administration may be expected, for example, by oral, intravenous, intramuscular, subcutaneous, intraperitoneal injection.

The administration amount of the pharmaceutical composition according to an embodiment of the present invention is selected in consideration of the age, weight, sex, physical condition, etc. of the subject. It is apparent that the concentration of the single domain antibody included in the pharmaceutical composition may be variously selected depending on the subject. It is preferably included in the pharmaceutical composition at a concentration of 0.01 μg/ml to 5,000 μg/ml. When the concentration is less than 0.01 μg/ml, the pharmaceutical activity may not be exhibited. When the concentration is more than 5,000 μg/ml, it may be toxic to the human body.

The composition of an embodiment of the present invention may be used for preventing or treating cancer and complications thereof and may also be used as an anticancer adjuvant.

Further, the present invention provides a method of preventing and treating cancer, in which the method includes administering to a subject the composition of an embodiment of the present invention in a pharmaceutically effective amount.

The composition of an embodiment of the present invention is administered in therapeutically or pharmaceutically effective amounts. The term "pharmaceutically effective amount" means an amount sufficient to treat a disease at a reasonable benefit/risk ratio applicable to medical treatment. The effective dose level may be determined by factors such as the subject's species, severity, age, gender, drug activity, drug sensitivity, the time of administration, the route of administration, the rate of excretion, the duration of the treatment and co-administered drugs, and other factors well known in the medical fields.

In an aspect, an embodiment of the present invention relates to a use of the anti-tumor adenovirus of an embodiment of the present invention for preventing or treating a tumor.

In an aspect, an embodiment of the present invention relates to a method of treating a tumor using the anti-tumor adenovirus of an embodiment of the present invention.

[Mode for Carrying Out Invention]

The present invention is described in more detail with reference to the following Examples. However, the following Examples are only for the purpose of illustrating the present invention, and therefore, the present invention is not limited thereto.

## Example 1. Preparation of Double Target siRNA

### 1-1. mTOR and STAT3 Double Target siRNAs

The double target siRNA (double strand) which is capable of simultaneously inhibiting signal transducer and activator of transcription 3 (STAT3) and mammalian target of rapamycin (mTOR) was prepared by sequences as shown in Table 1 below (Bioneer, Daejeon, Korea). Specifically, 17mer of 21mer in siRNA represented by SEQ ID NOS: 1 and 2 of Set 1, 16mer of 20mer in siRNA represented by SEQ ID NOS: 3 and 4 of Set 2, 15mer of 19mer in siRNA represented by SEQ ID NOS: 5 and 6 of Set 3, 14mer of 18mer in siRNA represented by SEQ ID NOS: 7 and 8 of Set 4, and 16mer of 17mer in siRNA represented by SEQ ID NOS: 9 and 10 of Set 5 are complementarily linked. In addition, 17mer of 20mer in siRNA represented by SEQ ID NOS: 11 and 12 of Set 6, 16mer of 19mer in siRNA represented by SEQ ID NOS: 13 and 14 of Set 7, 15mer of 18mer in siRNA represented by SEQ ID NOS: 15 and 16 of Set 8, and 14mer of 17mer in siRNA represented by SEQ ID NOS: 17 and 18 of Set 9 are complementarily linked. After two sequences of each set of Table 1 below are introduced into cells in the form of a double strand, the siRNA of antisense_mTOR of each set is complementarily linked to the target site of mTOR mRNA (gi|206725550|ref|NM_004958.3| Homo sapiens mechanistic target of rapamycin (serine/threonine kinase) (MTOR), mRNA). Further, siRNA of antisense_STAT3 of each set is complementarily linked to the target site of STAT3 mRNA (gi|47080104|ref|NM_139276.2| Homo sapiens signal transducer and activator of transcription 3 (acute-phase response factor) (STAT3), transcript variant 1, mRNA), thereby reducing mTOR and STAT3 gene expression.

[Table 1]

| set | siRNA | Sequence (sense), 5'-3' | Seq. Nos. | Sequence (antisense), 5'-3' | Seq. Nos. | Length | Complementary bond length |
|---|---|---|---|---|---|---|---|
| 1 | si-MS1 | gacuguggcauccaccugcau | 1 | augcagguaggcgccucaguc | 2 | 21 | 17 |
| 2 | si-MS2 | gacuguggcauccaccugca | 3 | ugcagguaggcgccucaguc | 4 | 20 | 16 |
| 3 | si-MS3 | gacuguggcauccaccugc | 5 | gcagguaggcgccucaguc | 6 | 19 | 15 |
| 4 | si-MS4 | gacuguggcauccaccug | 7 | cagguaggcgccucaguc | 8 | 18 | 14 |
| 5 | si-MS5 | ucagccacagcagcuug | 9 | caagcugcuguagcuga | 10 | 17 | 16 |
| 6 | si-MS6 | gcagcgcaugcggcccagca | 11 | ugcugggccgcaguggcugc | 12 | 20 | 17 |
| 7 | si-MS7 | gcagcgcaugcggcccagc | 13 | gcugggccgcaguggcugc | 14 | 19 | 16 |
| 8 | si-MS8 | gcagcgcaugcggcccag | 15 | cugggccgcaguggcugc | 16 | 18 | 15 |
| 9 | si-MS9 | gcagcgcaugcggccca | 17 | ugggccgcaguggcugc | 18 | 17 | 14 |

1-2. BCL2 and BI-1 Double Target siRNAs

The double target siRNA (double strand) of 21mer which is capable of simultaneously inhibiting BCL2 (B-cell lymphoma 2) and BI-1 (BAX inhibitor 1) was prepared by sequences as shown in Table 2 below (Bioneer, Daejeon, Korea). Specifically, siRNA set 10 of 21mer composed of the SEQ ID NOS: 19 and 20 of Table 2 below has a complementary binding length of 15mer therebetween. siRNA set 11 of 20mer composed of the SEQ ID NOS: 21 and 22 has a complementary binding length of 14mer therebetween. siRNA set 12 of 20mer composed of the SEQ ID NOS: 23 and 24 has a complementary binding length of 14mer therebetween. siRNA set 13 of 19mer composed of the SEQ ID NOS: 25 and 26 has a complementary binding length of 13mer therebetween. siRNA set 14 of 19mer composed of the SEQ ID NOS: 27 and 28 has a complementary binding length of 13mer therebetween. siRNA set 15 of 18mer composed of the SEQ ID NOS: 29 and 30 has a complementary binding length of 12mer therebetween. siRNA (Antisense Bcl-2) represented by SEQ ID NO: 19, 21, 23, 25, 27 or 29 of Table 2 below is complementarily linked with the mRNA of Bcl-2, and siRNA (Antisense BI-1) represented by SEQ ID NO: 20, 22, 24, 26, 28, or 30 is complementarily linked with the mRNA of BI-1. Accordingly, siRNA sets 10 to 15 of an embodiment of the present invention simultaneously reduce the expression of Bcl-2 and BI-1 genes.

[Table 2]

| set | siRNA | Sequence (sense), 5'-3' | Seq. Nos. | Sequence (antisense), 5'-3' | Seq. Nos. | Length (mer) | Complementary bond length (mer) |
|---|---|---|---|---|---|---|---|
| 10 | si-BB1 | AAGAAGAGGAGAAAAAAAUGA | 19 | UCAUUUCUUCUCUUUCUUCUU | 20 | 21 | 15 |
| 11 | si-BB2 | AAGAAGAGGAGAAAAAAAUG | 21 | CAUUUCUUCUCUUUCUUCUU | 22 | 20 | 14 |
| 12 | si-BB3 | AGAAGAGGAGAAAAAAAUGA | 23 | CAUUUCUUCUCUUUCUUCUU | 24 | 20 | 14 |
| 13 | si-BB4 | GAAGAGGAGAAAAAAAUGA | 25 | UCAUUUCUUCUCUUUCUUC | 26 | 19 | 13 |
| 14 | si-BB5 | AGAAGAGGAGAAAAAAAUG | 27 | CAUUUCUUCUCUUUCUUCU | 28 | 19 | 13 |
| 15 | si-BB6 | GAAGAGGAGAAAAAAAUG | 29 | CAUUUCUUCUCUUUCUUC | 30 | 18 | 12 |

1-3. AR and mTOR Double Target siRNA

The double target siRNA (double strand) set which is capable of simultaneously inhibiting AR (androgen receptor) and mTOR (mammalian target of rapamycin) was prepared by sequences as shown in Table 3 below (Bioneer, Daejeon, Korea). Specifically, siRNA set 16 of 20mer composed of the SEQ ID NOS: 31 and 32 has a complementary binding length of 18mer therebetween. siRNA set 17 of 19mer composed of the SEQ ID NOS: 33 and 34 has a complementary binding length of 17mer therebetween. siRNA set 18 of 18mer composed of the SEQ ID NOS: 35 and 36 has a complementary binding length of 16mer therebetween. siRNA set 19 of 17mer composed of the SEQ ID NOS: 37 and 38 has a complementary binding length of 15mer therebetween. siRNA set 20 of 19mer composed of the SEQ ID NOs: 39 and 40 has a complementary binding length of 15mer therebetween. siRNA set 21 of 18mer composed of the SEQ ID NOS: 41 and 42 has a complementary binding length of 14mer therebetween. siRNA set 22 of 17mer composed of the SEQ ID NOS: 43 and 44 has a complementary binding length of 13mer therebetween. siRNA set 23 of 23mer composed of the SEQ ID NOS: 45 and 46 has a complementary binding length of 19mer therebetween. siRNA set 24 of 22mer composed of the SEQ ID NOS: 47 and 48 has a complementary binding length of 18mer therebetween. siRNA set 25 of 22mer composed of the SEQ ID NOS: 49 and 50 has a complementary binding length of 18mer therebetween. siRNA set 26 of 21mer composed of the SEQ ID NOS: 51 and 52 has a complementary binding length of 17mer therebetween. siRNA set 27 of 20mer composed of the SEQ ID NOS: 53 and 54 has a complementary binding length of 16mer therebetween. siRNA set 28 of 21mer composed of the SEQ ID NOS: 55 and 56 has a complementary binding length of 17mer therebetween. siRNA (Antisense AR) represented by SEQ ID NO: 31, 33, 35, 37, 39, 41, 43, 45, 47, 49, 51, 53, or 55 of Table 3 below is complementarily linked with the mRNA of AR, and siRNA (Antisense mTOR) represented by SEQ ID NO: 32, 34, 36, 38, 40, 42, 44, 46, 48, 50, 52, 54, or 56 is complementarily linked with the mRNA of mTOR. Accordingly, siRNA sets 16 to 28 of an embodiment of the present invention simultaneously reduce the expression of AR and mTOR genes.

[Table 3]

| set | siRNA | Sequence (sense), 5'-3' | Seq. Nos. | Sequence (antisense), 5'-3' | Seq. Nos. | Length (mer) | Complementary bond length (mer) |
|---|---|---|---|---|---|---|---|
| 16 | si-AT1 | GCUGCUGCUGCUGCCUGGGG | 31 | CCCCAUGCAGCUGCAGCAGC | 32 | 20 | 18 |
| 17 | si-AT2 | CUGCUGCUGCUGCCUGGGG | 33 | CCCCAUGCAGCUGCAGCAG | 34 | 19 | 17 |
| 18 | si-AT3 | UGCUGCUGCUGCCUGGGG | 35 | CCCCAUGCAGCUGCAGCA | 36 | 18 | 16 |
| 19 | si-AT4 | GCUGCUGCUGCCUGGGG | 37 | CCCCAUGCAGCUGCAGC | 38 | 17 | 15 |
| 20 | si-AT5 | CCACCCCCACCACCACCAC | 39 | GUGGUGGCAGCGGUGGUGG | 40 | 19 | 15 |
| 21 | si-AT6 | CCACCCCCACCACCACCA | 41 | UGGUGGCAGCGGUGGUGG | 42 | 18 | 14 |
| 22 | si-AT7 | CCACCCCCACCACCACC | 43 | GGUGGCAGCGGUGGUGG | 44 | 17 | 13 |
| 23 | si-AT8 | UGGAGGCAGAGAGUGAGAGAGAA | 45 | UUCUCUCAGACGCUCUCCCUCCA | 46 | 23 | 19 |
| 24 | si-AT9 | GGAGGCAGAGAGUGAGAGAGAA | 47 | UUCUCUCAGACGCUCUCCCUCC | 48 | 22 | 18 |

(continued)

| set | siRNA | Sequence (sense), 5'-3' | Seq. Nos. | Sequence (antisense), 5'-3' | Seq. Nos. | Length (mer) | Complementary bond length (mer) |
|---|---|---|---|---|---|---|---|
| 25 | si-AT10 | UGGAGGCAGAGAG UGAGAGAGA | 49 | UCUCUCAGACGCUCUCC CUCCA | 50 | 22 | 18 |
| 26 | si-AT11 | UGGAGGCAGAGAG UGAGAGAG | 51 | CUCUCAGACGCUCUCCC UCCA | 52 | 21 | 17 |
| 27 | si-AT12 | GGAGGCAGAGAGU GAGAGAG | 53 | CUCUCAGACGCUCUCCC UCC | 54 | 20 | 16 |
| 28 | si-AT13 | GGAGGCAGAGAGU GAGAGAGA | 55 | UCUCUCAGACGCUCUCC CUCC | 56 | 21 | 17 |

1-4. MGMT and mTOR Double Target siRNA

The double target siRNA (double strand) set which is capable of simultaneously reducing (inhibiting) MGMT (O-6-methylguanine-DNA methyltransferase, NM_002412.5) and mTOR (NM_004958.3) gene expression was prepared by sequences as shown in Table 4 below (Bioneer, Daejeon, Korea).

[Table 4]

| set | siRNA | Sequence (sense), 5'-3' | Seq. No. | Sequence (antisense), 5'-3' | Seq. No. |
|---|---|---|---|---|---|
| 29 | si-MM | GCAGCUUGGAGGCAGAGCG | 57 | CGCUCUCCCUCCAUGCUGC | 58 |

1-5. BCL2 and MCL1 Double Target siRNA

The double target siRNA (double strand) set which is capable of simultaneously reducing (inhibiting) BCL2 (NM_000633.2) and MCL1 (MCL1 apoptosis regulator, NM_021960.5) gene expression was prepared by sequences as shown in Table 5 below (Bioneer, Daejeon, Korea).

[Table 5]

| set | siRNA | Sequence (sense), 5'-3' | Seq. No. | Sequence (antisense), 5'-3' | Seq. No. |
|---|---|---|---|---|---|
| 30 | si-BM | GCCAAGGCCACACAGCCAA | 59 | UUGGCUUUGUGUCCUUGGC | 60 |

1-6. STAT3 and TFEB Double Target siRNAs

The double target siRNA (double strand) set which is capable of simultaneously reducing (inhibiting) STAT3 (NM_139276.2) and TFEB (transcription factor EB, NM_007162.2) gene expression was prepared by sequences as shown in Table 6 below (Bioneer, Daejeon, Korea).

[Table 6]

| set | siRNA | Sequence (sense), 5'-3' | Seq. No. | Sequence (antisense), 5'-3' | Seq. No. |
|---|---|---|---|---|---|
| 31 | si-ST | CCAGCCAGACCCAGAAGGA | 61 | UCCUUCUUGGACAGGCUGG | 62 |

1-7. c-MET and PD-L1 Double Target siRNAs

The double target siRNA (double strand) set which is capable of simultaneously inhibiting c-MET (Homo sapiens MET proto-oncogene) and PD-L1 (Programmed death-ligand 1) was prepared by sequences as shown in Table 7 below (Bioneer, Daejeon, Korea). Specifically, siRNA set 32 of 19mer composed of the SEQ ID NOS: 63 and 64 has a complementary binding length of 15mer therebetween. siRNA (Antisense c-MET) represented by SEQ ID NO: 63 of Table 7 below is complementarily linked with the mRNA of c-MET, and siRNA (Antisense PD-L1) represented by SEQ ID NO: 64 is complementarily linked with the mRNA of PD-L1. Accordingly, the siRNA set of an embodiment of the present invention simultaneously reduces the expression of c-MET and PD-L1 genes.

[Table 7]

| set | siRNA | Sequence (sense) 5'-3' | Seq. No. | siRNA | Sequence (antisense) 5'-3' | Seq. No. | length (mer) | Complementary bond length (mer) |
|---|---|---|---|---|---|---|---|---|
| 32 | c-MET | ACCACACAUCUGAC UUGGU | 63 | PD-L1 | ACCAAUUCAGCUG UAUGGU | 64 | 19 | 15 |

**Example 2. Preparation of Double Target shRNA**

2-1. mTOR and STAT3 Target shRNA

In order to enable the expression of the siRNA prepared in the above Example in cells, an expression cassette expressing shRNA was prepared. Specifically, shRNAs (TTGGATCCAA loop shRNA and TTCAAGAGAG loop shRNA) including a double-target siRNA (SEQ ID NOS: 1 and 2) siRNA double-stranded sequence and a loop sequence of set 1 among siRNAs were prepared as a representative (Table 8). Each of the prepared shRNA expression cassettes was placed following the U6 promoter (SEQ ID NO: 65) at the cleavage sites of the restriction enzymes PstI and EcoRV of each pE3.1 vector (FIG. 1), thereby preparing recombinant expression vectors which are capable of expressing two kinds of shRNAs including double target siRNA targeting mTOR and STAT3 in the cells.

[Table 8]

| mTOR and STAT3 Double Target shRNA | Sequences (5'→3') | Seq. Nos. |
|---|---|---|
| TTGGATCCAA loop shRNA | gactgtggcatccacctgcatTTGGATCCAAatgcaggtaggcgcctcagtcTT | 66 |
| TTCAAGAGAG loop shRNA | gactgtggcatccacctgcatTTCAAGAGAGatgcaggtaggcgcctcagtcTT | 67 |

2-2. BCL2 and BI-1 Target shRNAs

In order to enable the expression of the siRNA prepared in the above Example in cells, an expression cassette expressing shRNA was prepared. Specifically, shRNAs (TTGGATCCAA loop shRNA and TTCAAGAGAG loop shRNA) including a double-target siRNA (SEQ ID NOS: 19 and 20) siRNA double-stranded sequence and a loop sequence of set 10 among siRNAs were prepared as a representative (Table 9). Each of the prepared shRNA expression cassettes was placed following the U6 promoter (SEQ ID NO: 65) at the cleavage sites of the restriction enzymes PstI and EcoRV of each pE3.1 vector (FIG. 1), thereby preparing recombinant expression vectors which are capable of expressing two kinds of shRNAs including double target siRNA targeting BCL2 and BI-1 in the cells.

[Table 9]

| **BCL2 and BI-1 Double Target shRNA** | **Sequences (5'→3')** | **Seq. Nos.** |
|---|---|---|
| TTGGATCCAA loop shRNA | aagaagaggagaaaaaaatgaTTGGATCCAAtcatttcttctctttcttcttTT | 68 |
| TTCAAGAGAG loop shRNA | aagaagaggagaaaaaaatgaTTCAAGAGAGtcatttcttctctttcttcttTT | 69 |

2-3. AR and mTOR Target shRNA

In order to enable the expression of the siRNA prepared in the above Example in cells, an expression cassette expressing shRNA was prepared. Specifically, shRNAs (TTGGATCCAA loop shRNA and TTCAAGAGAG loop shRNA) including a double-target siRNA (SEQ ID NOS: 31 and 32) siRNA double-stranded sequence and a loop sequence of set 16 among siRNAs were prepared as a representative (Table 10). Each of the prepared shRNA expression cassettes was placed following the U6 promoter (SEQ ID NO: 65) at the cleavage sites of the restriction enzymes PstI and EcoRV of each pE3.1 vector (FIG. 1), thereby preparing recombinant expression vectors which are capable of expressing two kinds of shRNAs including double target siRNA targeting AR and mTOR in the cells.

[Table 10]

| **AR and mTOR Double Target shRNA** | **Sequences (5'→3')** | **Seq. Nos.** |
|---|---|---|
| TTGGATCCAA loop shRNA | GCTGCTGCTGCTGCCTGGGGTTGGATCCAACCCCATGCAGCTG CAGCAGCTT | 70 |

(continued)

| AR and mTOR Double Target shRNA | Sequences (5'→3') | Seq. Nos. |
|---|---|---|
| TTCAAGAGAG loop shRNA | GCTGCTGCTGCTGCCTGGGGTTCAAGAGAGCCCCATGCAGCTG CAGCAGCTT | 71 |

2-4. c-MET and PD-L1 Target shRNA

In order to enable the expression of the siRNA prepared in the above Example in cells, an shRNA expression cassette (TTGGATCCAA loop shRNA and TTCAAGAGAG loop shRNA) including a double target siRNA double-stranded sequence and a loop sequence was prepared. Specifically, TTGGATCCAA (TTGGATCCAA loop) or TTCAAGAGAG (TTCAAGAGAG loop), antisense strand and TT are linked to 3' of a sense strand of the siRNA set (SEQ ID NOS: 63 and 64) of Table 7 in a 5' to 3' direction so that a DNA sequence encoding the siRNA was prepared and shown in Table 11 (siRNAs are denoted in uppercase letters and additional sequences ae denoted in lowercase letters). Each of the prepared shRNA expression cassettes was placed following the U6 promoter (SEQ ID NO: 65) at the cleavage sites of the restriction enzymes PstI and EcoRV of each pE3.1 vector (FIG. 1), thereby preparing recombinant expression vectors which are capable of expressing two shRNAs including double target siRNA targeting c-MET and PD-L1 in the cells.

[Table 11]

| c-MET and PD-L1 Double Target shRNA | Sequences (5'→3') | Seq. Nos. |
|---|---|---|
| TTGGATCCAA loop shRNA | ACCACACAUCUGACUUGGUttggatccaaACCAAUUCAGCUGUAU GGUtt | 72 |
| TTCAAGAGAG loop shRNA | ACCACACAUCUGACUUGGUttcaagagagACCAAUUCAGCUGUAU GGUtt | 73 |

**Example 3. Identification of Gene Expression Inhibitory Effect by Double Target siRNA**

3-1. mTOR and STAT3 Expression Inhibition

Hela cells were seeded on a 12-well plate. Then, until the cell density reached 50%, the cells were cultured in RPMI medium (Hyclone) supplemented with 10% FBS (Hyclone) at 37°C and 5% $CO_2$. Then, the cells were transfected with the double target siRNA of sets 1 to 9 prepared in Example 1 using lipofectamine 3000 (Invitrogen, Carlsbad, CA, USA) to perform the knock-down of mTOR and STAT3, simultaneously. After 48 hours of the transfection, the cells were disrupted, and total RNAs were extracted with GeneJET RNA Purification Kit (Invitrogen). The reverse transcription reaction was performed with RevoScriptTM RT PreMix (iNtRON BIOTECHNOLOGY) using the extracted total RNA as a template. 20 μl of a sample containing 25 to 200 ng of the reverse transcribed cDNA, AmpONE taq DNA polymerase (GeneAll) and TaqMan Gene Expression assays (Applied Biosystems) were used. They were reacted with mTOR (Hs00234522_m1), STAT3 (Hs01047580_m1) and GAPDH (Hs02758991_g1) using ABI PRISM 7700 Sequence Detection System and QS3 Real-time PCR (Biosystems). The real-time PCR reaction conditions were [2 minutes at 50°C, 10 minutes at 95°C, and two cycles of 15 seconds at 95°C and 60 seconds at 60°C], and the reaction was repeated in total 40 cycles. All reactions were repeated three times, and the mean value of these was obtained. The results were normalized to the mRNA values of the housekeeping gene GAPDH.

As a result, it was identified that mTOR and STAT3 had about 20% to 40% residual expression compared to the control by double target siRNAs of Sets 1 to 9, and it was found that the double target siRNA simultaneously inhibited expression of both genes (FIG. 2).

3-2. BCL2 and BI-1 Expression Inhibition

Hela cells were each seeded on a 12-well plate. Then, until the cell density reached 50%, the cells were cultured in RPMI medium (Hyclone) supplemented with 10% FBS (Hyclone) at 37°C and 5% $CO_2$. Thereafter, 3 μl of lipofectamine 3000 (Invitrogen, Carlsbad, CA, USA) was used to transfect the double target siRNA set 10 (si-BB 1) and the double target siRNA sets 11 to 15 prepared in the Example 1 (Table 2) to the wells in which the Hela cells were cultured at 80 pmole per well to perform the knock-down of BCL2 and BI-1, simultaneously. After 48 hours of the transfection, the cells were disrupted, and total RNAs were extracted with GeneJET RNA Purification Kit (Invitrogen). While using the extracted total RNAs as a template, the same was subjected to reverse transcription into cDNA via RT-PCR reaction. Then, mRNA expression levels of BCL2 and BI-1 by the double target siRNA was identified via q-PCR reaction. For reference, the probes used were Bcl2 (Thermo, Hs00608023_m1), BI-1 (Thermo, Dm01835892_g1), and GAPDH (Thermo, Hs02786624_g1). The PCR was performed using QS3 equipment. All reactions were repeated three times, and the mean value of these was obtained. The results were normalized to the mRNA values of GAPDH as a housekeeping gene.

As a result, the expression of both the BCL2 and BI-1 was reduced by the double target siRNA set, and thus, it was found that the double target siRNA of an embodiment of the present invention simultaneously inhibited expression of both genes (FIG. 3 and FIG. 4).

Accordingly, the double target siRNA of an embodiment of the present invention simultaneously inhibits the expression of both genes, and thus, it was identified that remarkable anticancer activity is shown by promoting the death of cancer cells, thus suggesting that the double target siRNA can be usefully used as an anticancer composition or an anticancer adjuvant for various carcinomas.

### 3-3. AR and mTOR Expression Inhibition

PC3 cell line, and h460 and A549 cell lines were each seeded on a 12-well plate. Then, until the cell density reached 50%, the cells were cultured in RPMI medium (Hyclone) supplemented with 10% FBS (Hyclone) at 37°C and 5% $CO_2$. Thereafter, 3 μl of lipofectamine 3000 (Invitrogen, Carlsbad, CA, USA) was used to transfect the double target siRNA sets 16 to 28 prepared in the Example 1 (Table 3) to the wells in which the cells were cultured at 80 pmole per well to perform the knock-down of AR and mTOR, simultaneously. Herein, set 16 was knocked down by transfection into h460 cells and set 17 was knocked down by transfection into PC3 cells. As positive controls, siRNA for AR and siRNA for mTOR described in Table 12 below were transfected, respectively. After 48 hours of the transfection, the cells were disrupted, and total RNAs were extracted with GeneJET RNA Purification Kit (Invitrogen). While using the extracted total RNAs as a template, the same was subjected to reverse transcription into cDNA via RT-PCR reaction. Then, mRNA expression levels of each of siRNA and AR and mTOR by the double target siRNA sets 16 to 28 (si-AT1 to siAT13) of an embodiment of the present invention was identified via q-PCR reaction. In order to identify the mRNA expression levels, a primer set and reaction mixture for AR or mTOR [10X reaction Buffer 2 μl, HQ Buffer 2 μl, dNTP 1.6 μl, Primer (F, R, 10 pmole/μl) 1 μl each, Template (500 ng) 2 μl, Taq 0.2 μl, DW 10.2 μl, Total vol. 20 μl] were used. mRNA of AR and mTOR in cell lysates knocked down by PCR conditions [2 minutes at 95°C, 30 cycles at 95°C for 20 seconds, 10 seconds at 60°C and 30 to 60 seconds at 72°C, and 5 minutes at 72°C] was converted into cDNA. In addition, the reverse transcribed cDNA was used as a template, the reaction mixture [Template (RT-PCR product) 6 μl, Taqman probe 3 μl, 10X reaction Buffer 6 μl, HQ Buffer 6 μl, dNTP 4.8 μl, Taq 0.6 μl, DW 10.2 μl, Total vol. 60 μl] was prepared, and qPCR was performed [10 minutes at 95°C, 15 seconds at 95°C and 40 cycles per minute at 60°C]. For reference, the probes used were AR (Thermo, Hs00171172_m1), mTOR (Thermo, Hs00234508_m1), and GAPDH (Thermo, Hs02786624_g1). The PCR was performed using QS3 equipment. All reactions were repeated three times, and the mean value of these was obtained. The results were normalized to the mRNA values of GAPDH as a housekeeping gene.

[Table 12]

| | | **Sequences (5'→3')** |
|---|---|---|
| AR siRNA | sense | CACAAGUCCCGGAUGUACA(dTdT) |
| | anti-sense | UGUACAUCCGGGACUUGUG(dTdT) |
| mTOR siRNA | sense | GUGGAAACAGGACCCAUGA(dTdT) |
| | anti-sense | UCAUGGGUCCUGUUUCCAC(dTdT) |

As a result, the expression of both AR and mTOR was reduced by the double target siRNA sets 16 and 17 of an embodiment of the present invention in both PC3 cells and h460 cell lines (FIG. 5), and the degree of reduction was shown to be similar to or superior to the effect of each siRNA. In addition, the expression of both AR and mTOR was reduced by the double target siRNA sets 17 and 28 of an embodiment of the present invention (FIG. 6). Thus, it was found that the double target siRNA of an embodiment of the present invention could effectively inhibit the expression of both genes simultaneously.

3-4. c-MET and PD-L1 Expression Inhibition

Glioblastoma cell line U-87, prostate cancer cell line CWR22Rv-1 (22Rv-1), melanoma cell line A431, and non-small cell lung cancer cell line HCC827 were each seeded on a 12-well plate. Then, until the cell density reached 50%, the cells were cultured in RPMI medium (Hyclone) supplemented with 10% FBS (Hyclone) at 37°C and 5% $CO_2$. Thereafter, 3 μl of lipofectamine 3000 (Invitrogen, Carlsbad, CA, USA) was used to transfect the double target siRNA set prepared in the Example (Table 7) to the wells in which the cells were cultured at 80 pmole per well to perform the knock-down of c-MET and PD-L1, simultaneously. After 48 hours of the transfection, the cells each were disrupted, and total RNAs were extracted with GeneJET RNA Purification Kit (Invitrogen). While using the extracted total RNAs as a template, the same was subj ected to reverse transcription into cDNA via RT-PCR reaction. Then, mRNA expression levels of each of siRNA and c-MET and PD-L1 by the double target siRNA set of an embodiment of the present invention was identified via q-PCR reaction. In order to identify the mRNA expression levels, a primer set and reaction mixture for PD-L1 or c-MET [10X reaction Buffer 2 μl, HQ Buffer 2 μl, dNTP 1.6 μl, Primer (F, R, 10 pmole/μl) 1 μl each, Template (500 ng) 2 μl, Taq 0.2 μl, DW 10.2 μl, Total vol. 20 μl] were used. mRNA of c-MET and PD-L1 in cell lysates knocked down by PCR conditions [2 minutes at 95°C, 30 cycles at 95°C for 20 seconds, 10 seconds at 60°C and 30 to 60 seconds at 72°C, and 5 minutes at 72°C] was converted into cDNA. In addition, the reverse transcribed cDNA was used as a template, the reaction mixture [Template (RT-PCR product) 6 μl, Taqman probe 3 μl, 10X reaction Buffer 6 μl, HQ Buffer 6 μl, dNTP 4.8 μl, Taq 0.6 μl, DW 10.2 μl, Total vol. 60 μl] was prepared, and qPCR was performed using QS3 equipment [10 minutes at 95μC, 15 seconds at 95μC and 40 cycles per minute at 60μC]. All reactions were repeated three times, and the mean value of these was obtained. The results were normalized to the mRNA values of GAPDH as a housekeeping gene.

As a result, the expression of both c-MET and PD-L1 was reduced by the double target siRNA set of an embodiment of the present invention in all of the U-87 cell line, 22Rv-1 cell line, A431 cell line and HCC827 cell line (FIGS. 7A to 7D). Thus, it was found that the double target siRNA of an embodiment of the present invention could effectively inhibit the expression of both genes simultaneously.

**Example 4. Identification of Gene Expression Inhibitory Effect by Double Target siRNA and shRNA**

4-1. Gene Expression Inhibitory Effect by Double Target shRNA

The vector including the TTGGATCCAA loop shRNA sequence represented by SEQ ID NO: 66 or the TTCAAGAGAG loop shRNA sequence represented by SEQ ID NO: 67 (Table 8) encoding the mTOR and STAT3 target shRNAs prepared in Example 2 above was transfected with A549 cells and glioblastoma cells U-87 (U87MG) at 0, 1, and 2 μg, respectively, using lipofectamine 3000. After 48 hours of the transfection, the degree of reduction in gene expression of mTOR and STAT3 was identified using the Real-time PCR analysis method described in the above Examples.

As a result, the expression of mTOR and STAT3 was reduced in both types of shRNA including the double target siRNA of an embodiment of the present invention, and showed a tendency to reduce by about 20% in proportion to the amount of DNA of the shRNA (FIG. 8).

4-2. Comparison of Gene Expression Inhibitory Effect by Single Target siRNA and Double Target shRNA

The gene expression inhibitory effect of two single target siRNAs linked in series using two promoters and the double target shRNA of an embodiment of the present invention was compared. Specifically, the siRNA for mTOR and siRNA for STAT3 were serially linked in the sequence of mTOR-STAT3 or STAT3-mTOR, and then the gene expression inhibitory effect on mTOR and STAT3 was compared with the mTOR/STAT3 double target shRNA of an embodiment of the present invention.

As a result, in the 293T cell line, compared to when two siRNAs for each gene were linked in series using a promoter (direct shmTOR-STAT3 or direct shSTAT3-mTOR), it was found that the gene expression inhibitory effect was significantly high when the double target shRNA of an embodiment of the present invention was used (FIG. 9).

**Example 5. Identification of Cancer Cell Killing Effect by Double Target siRNA**

In order to identify effects on cancer cell death by double target siRNA of sets 1 to 9 of an embodiment of the present invention, human lung cancer cell line A549 cells were seeded to $5 \times 10^3$ cells/well in a 96-well plate, and then the cells were transfected with the double target siRNA of sets 1 to 9 using lipofectamine 3000. After 48 hours of the transfection and additional 24 hours, the cells were treated with 5 mg/mL MTT (Promega, Ltd.) and incubated for 4 hours. Thereafter, the medium was removed, and the cells were treated with 150 μl of solubilization solution and stop solution and incubated at 37°C for 4 hours. The absorbance of the reaction solution was measured at 570 nm, and the cell viability was calculated using the following equation.

[Equation 1]

$$\text{Cell viability} = \text{absorbance of experimental group (570 nm)/absorbance of control group (570 nm)} \times 100(\%)$$

As a result, it was identified that when mTOR and STAT3 were simultaneously inhibited by the double target siRNA of sets 1 to 9 of an embodiment of the present invention, the cell viability was significantly reduced as compared to the control group. Accordingly, it was identified that the double target siRNA of sets 1 to 9 of an embodiment of the present invention effectively led to the cancer cell death (FIG. 10).

**Example 6. Identification of Cancer Cell Killing Effect by Co-Treatment with Double Target siRNA and Anticancer Agent**

6-1. Co-Treatment of mTOR and STAT3 Double Target siRNA with Anticancer Agent

6-1-1. Co-Treatment with Cisplatin

Human lung cancer cell line A549 cells were seeded at $5 \times 10^3$ cells/well in 96-well plates. Then, the cells were transfected with each of the double target siRNAs (mTOR and STAT3 co-knock down) of sets 1 to 9 of an embodiment of the present invention using lipofectamine 3000. After 48 hours of the transfection, the cells were treated with 5 μM of cisplatin and incubated for 10 hours. Thereafter, the MTT reaction was performed as in the Examples above, and the absorbance thereof was measured at 570 nm to calculate the cell viability.

As a result, it was identified that when mTOR and STAT3 were simultaneously inhibited by the double target siRNA of sets 1 to 9 of an embodiment of the present invention in combination with cisplatin, the cell viability was reduced to about 50% to 70%, and there was a significant difference compared to the control group. Accordingly, it was identified that when the two genes were simultaneously inhibited even in combination with the anticancer agent, the cell killing effect was significantly improved (FIG. 11).

6-1-2. Co-Treatment with Paclitaxel

Human lung cancer cell line A549 cells were seeded at $5 \times 10^3$ cells/well in 96-well plates. Then, the cells were transfected with each of the double target siRNAs (mTOR and STAT3 co-knock down) of sets 1 to 9 of an embodiment of the present invention using lipofectamine 3000. After 48 hours of the transfection, the cells were treated with 5 μM of paclitaxel and incubated for 10 hours. Thereafter, the MTT reaction was performed as in Example 4 above, and the absorbance thereof was measured at 570 nm to calculate the cell viability.

As a result, it was identified that when mTOR and STAT3 were simultaneously inhibited by the double target siRNA of sets 1 to 9 of an embodiment of the present invention in combination with paclitaxel, the cell viability was reduced to about 30% to 50%, and there was a significant difference compared to the control group. Accordingly, it was identified that when the two genes were simultaneously inhibited even in combination with the anticancer agent, the cell killing effect was significantly improved (FIG. 12).

6-1-3. Co-Treatment with 5-Fluorouracil (5-FU)

Human lung cancer cell line A549 cells were seeded at $5 \times 10^3$ cells/well in 96-well plates. Then, the cells were transfected with each of the double target siRNAs (mTOR and STAT3 co-knock down) of sets 1 to 9 of an embodiment of the present invention using lipofectamine 3000. After 48 hours of the transfection, the cells were treated with 5 μM of paclitaxel and incubated for 10 hours. Thereafter, the MTT reaction was performed as in Example 4 above, and the absorbance thereof was measured at 570 nm to calculate the cell viability.

As a result, it was identified that when mTOR and STAT3 were simultaneously inhibited by the double target siRNA of sets 1 to 9 of an embodiment of the present invention in combination with 5-fluorouracil, the cell viability was reduced to about 30%, and there was a significant difference compared to the control group. Accordingly, it was identified that when the two genes were simultaneously inhibited even in combination with the anticancer agent, the cell killing effect was significantly improved (FIG. 13).

6-2. Co-Treatment of BCL2 and BI-1 Double Target siRNA with Anticancer Agent

6-2-1. Synergistic Anticancer Effect of BCL2 and BI-1 Double Target siRNA

Hela cells, a human cervical cancer cell line, were transfected with the double target siRNA set 1 (si-BB1) of an embodiment of the present invention or individual BCL2 siRNA and BI-1 siRNA as a control group, respectively, followed by treatment with each type of anticancer agent for 6 hours. The degree of death of the cancer cell lines was identified by MTT analysis. Specifically, 200 pmole of siRNA per well was transfected with lipofectamine 7.5 μl into Hela cells seeded in a 6-well plate, and then incubated for 48 hours. The cell line was seeded in a 96-well plate again and cultured to 50% cell density ($2.5 \times 10^4$). In the case of a control group, the concentration of the anticancer drug was treated with Taxol 0.5 μM, Cisplatin 20 μM, and Etoposide 10 μM, respectively. The cells treated with the double target siRNA set of an embodiment the present invention were treated with each of the half concentrations of taxol 0.25 μM, cisplatin 10 μM, and etoposide 5 μM. After 6 hours, MTT analysis was performed as in the Examples above to identify the degree of death of cancer cells.

As a result, in the case of the control group transfected with the siRNA for each of BCL2 or BI-1, the death of cancer cells hardly occurred, and only the co-treatment with the anticancer agent caused a part of the cancer cell killing effect by the anticancer agent (FIG. 14A). In the case of the double target siRNA set 1 (si-BB1) of an embodiment of the present invention, it was found that the death of cancer cells occurred significantly, and the cancer cell killing effect synergistically increased even with the co-treatment with an anticancer agent at a significantly lower concentration than the control group treated with BCL2 siRNA and BI-1 siRNA (FIG. 14B). Thus, it could be inferred that the double target siRNA of an embodiment of the present invention itself exhibited anticancer activity, and that the synergistic effect by the co-treatment with the anticancer agent was also specific to the double target siRNA.

6-2-2. Comparison of Effects of BCL2 and BI-1 Dual Target siRNAs with Bcl2 Inhibitors

The inhibitory effect of the cancer cell death of the double target siRNA (set 1 si-BB 1) for BCL2 and BI-1 of an embodiment of the present invention was compared with an ABT-737 drug, which is used as a cancer cell therapeutic agent through the inhibition of BCL2. Specifically, after seeding the LnCap cell line, which is a prostate cancer cell, as in the Examples above, siRNA set 1 (si-BB1) for BCL2 and BI-1 of an embodiment of the present invention was transfected, and 3 μM of ABT-737 was treated and incubated for 12 hours, and then the degree of death of cancer cell lines was identified by MTT analysis.

As a result, the death of LnCap cell lines was increased by treatment with ABT-737 or siRNA set 1 (si-BB1) for BCL2 and BI-1 of an embodiment of the present invention. In particular, when ABT-737 and double target siRNA of an embodiment of the present invention were co-treated, it was found that the death of cancer cells was significantly increased synergistically (FIG. 15).

6-2-3. Comparison of Anticancer Effects of BCL2 and BI-1 Double Target siRNA and each siRNA

After culturing the PC3 cell line, a human prostate cancer cell line, in a 6-well plate, respectively, the double target siRNA set 10 (si-BB 1) of an embodiment of the present invention and the siRNA for each of BCL2 or BI-1 in Table 13 below as a control group were transfected, respectively. After 48 hours, cisplatin was treated with 10 to 20 μM. After 12 hours of cisplatin treatment, 5 mg/mL MTT (Promega, Ltd.) was treated with the cells and incubated for 4 hours. Thereafter, the medium was removed, and the cells were treated with 150 μl of solubilization solution and stop solution and incubated at 37°C for 4 hours. The absorbance of the reaction solution was measured at 570 nm, and the cell viability was calculated using Equation 1 above.

[Table 13]

| | | **Sequences (5'→3')** |
|---|---|---|
| BCL2 siRNA | sense | CAGAAGUCUGGGAAUCGAU(dTdT) |
| | anti-sense | AUCGAUUCCCAGACUUCUG(dTdT) |
| BI-1 siRNA | sense | GGAUCGCAAUUAAGGAGCA(dTdT) |
| | anti-sense | UGCUCCUUAAUUGCGAUCC(dTdT) |

As a result, it was found that the cancer cell death was significantly increased in the group treated with the double target siRNA set 10 of an embodiment of the present invention in combination with cisplatin, compared to the control group treated with the control siRNA without cisplatin, and that the degree thereof was significantly increased, compared to the group treated with siRNA for each of BCL2 and BI-1 (FIG. 16).

6-3. Co-Treatment of AR and mTOR Double Target siRNA with Anticancer Agent

After culturing the DU145 cell line and the H460 cell line in a 6-well plate, respectively, and after transfection with the double target siRNA set 16 (si-AT1) of an embodiment of the present invention, 50 μM of cisplatin, 20 μM of etoposide, or 1 μM of taxol was treated 48 hours later, and incubated for 16 hours. Thereafter, 5 mg/mL MTT (Promega, Ltd.) was treated with the cells and incubated for 4 hours. Thereafter, the medium was removed, and the cells were treated with 150 μl of solubilization solution and stop solution and incubated at 37°C for 4 hours. The absorbance of the reaction solution was measured at 570 nm, and the cell viability was calculated.

As a result, it was found that the double target siRNA of an embodiment of the present invention alone induced apoptosis with respect to the DU145 cell line, which is a prostate cancer cell line (a group not treated with an anticancer agent (no treat)), and that the double target siRNA of an embodiment of the present invention induced apoptosis even in the cisplatin-treated group, which did not show a cell killing effect. In addition, it was identified that DU145 apoptosis was remarkably improved by co-treatment with the double target siRNA set 16 (si-AT1) of an embodiment of the present invention for etoposide and taxol, which exhibited some anticancer activity (FIG. 17A). In addition, in the lung cancer cell line H460, similar to the DU145 cell line, the double target siRNA of an embodiment of the present invention exhibited an apoptotic effect, and it was found that the co-treatment with etoposide and taxol remarkably exhibited anticancer activity (FIG. 17B).

**Example 7. Preparation of Double Target shRNA Coding Adenovirus**

After inserting the hTERT promoter (SEQ ID NO: 74)-E1A (SEQ ID NO: 75)-IRES (SEQ ID NO: 76)-E1B sequence (SEQ ID NO: 77) (total sequence: SEQ ID NO: 78) between SpeI and ScaI of an adenoviral vector, the U6 promoter and BCL2 and BI-1 double target shRNAs (U6 promoter + BCL2 and BI-1 double target shRNA coding sequence: SEQ ID NO: 79), mTOR and STAT3 double target shRNAs (U6 promoter + mTOR and STAT3 double target shRNA coding sequence: SEQ ID NO: 80) and AR and mTOR double target shRNA sequence (U6 promoter + AR and mTOR double target shRNA coding sequence: SEQ ID NO: 81) prepared in the Examples above were inserted between SpeI in the E3 region, respectively, so that the hTERT promoter and the double target shRNA were encoded and expressed, and an infectious recombinant adenovirus was prepared (BCL2 and BI-1 double target shRNA coding and expression adenovirus: CA101; mTOR and STAT3 double target shRNA coding and expression adenovirus: CA102; AR and mTOR double target shRNA coding and expression adenovirus: CA103; and c-MET and PD-L1 double target shRNA coding (expression) adenovirus: CA104) (*see* FIG. 19). In addition, a recombinant adenovirus into which only hTERT promoter was inserted as a control group was also prepared (CA10G). Thereafter, the sequence of the prepared adenoviral vector was analyzed, and when there was no abnormality, the virus genome was linearized using the PacI restriction enzyme, and each virus was produced by transducing 293A cells using a $CaCl_2$ method.

**Example 8. Identification of Gene Expression Inhibition of Double Target shRNA Coding Adenovirus**

8-1. Identification of mTOR and STAT3 Expression Inhibition of CA102

8-1-1. Identification of mRNA Level

8-1-1-1. Bladder Cancer

The expression inhibitory effect of the recombinant adenovirus CA102 prepared in Example 7 on the target genes mTOR and STAT3 was identified in the bladder cancer cell line. Specifically, human bladder cancer cell lines, T24 cells ($0.5 \times 10^5$/well) and 253JBV cells ($1 \times 10^5$/well), were each seeded on a 12-well plate. Then, one hour later, CA10G and CA102 were treated at an MOI of 10 in each well, and 72 hours later, RNA prep was performed using an RNA prep kit (Takara, 9767A). Thereafter, RNA was quantified using Nanodirp, 400 ng/20 μl per tube was added using RT premix (intron, 25081), mixed well with the contents of the premix, and then cDNA was synthesized by reacting at 45°C for 1 hr and at 95°C for 5 minutes using a PCR device. The synthesized cDNA 2 μl was used as a template, and a PCR mixture (total volume, 20 μl) corresponding to an experimental group was made (template 2 μl, forward primer 0.5 μl (10 pmole/μl), reverse primer 0.5 μl (10 pmole/μl), 10 μl 2X master mix (Bioline, BIO-94005) and 7 μl DW). The prepared PCR mixture was vortexed, mixed well, and centrifuged, followed by reaction for 40 cycles of 5 minutes at 95°C, 10 seconds at 95°C, and 30 seconds at 60°C in a qPCR device (Applied Biosystems, QS3). The results were analyzed using the program in the qPCR device.

As a result, in both T24 cells and 253JBV cells, the recombinant adenovirus CA102 of an embodiment of the present invention, which encodes and expresses the hTERT promoter and mTOR and STAT3 double target shRNA, was shown to significantly inhibit the expression of mTOR and STAT3 genes compared to recombinant adenovirus CA10G including only the hTERT promoter (FIG. 20).

8-1-1-2. Head and Neck Cancer

As a result of identifying the expression inhibitory effect of the recombinant adenovirus CA102 prepared in Example 7 on the target genes mTOR and STAT3 in the head and neck cancer cell lines HSC-2 and Fadu by the method of the Examples above, in both cell lines, the recombinant adenovirus CA102 of an embodiment of the present invention, which encodes and expresses the hTERT promoter and mTOR and STAT3 double target shRNA, was shown to significantly inhibit the expression of mTOR and STAT3 genes compared to recombinant adenovirus CA10G including only the hTERT promoter (FIG. 21).

8-1-1-3. Skin Squamous Carcinoma

As a result of identifying the expression inhibitory effect of the recombinant adenovirus CA102 prepared in Example 7 on the target genes mTOR and STAT3 in the skin squamous carcinoma cell lines A431 and HSC-5 by the method of the Examples above, in both cell lines, the recombinant adenovirus CA102 of an embodiment of the present invention, which encodes and expresses the hTERT promoter and mTOR and STAT3 double target shRNA, was shown to significantly inhibit the expression of mTOR and STAT3 genes compared to recombinant adenovirus CA10G including only the hTERT promoter (FIG. 22).

8-1-2. Identification of Protein Expression Inhibition

After the recombinant adenovirus CA102 prepared in Example 7 was treated in bladder cancer cell lines T24 cells and 253JBV cells, the expression inhibitory effect on target genes mTOR and STAT3 was identified at the protein level by Western blot analysis. As a result, in both cell lines, it was identified that the recombinant adenovirus CA102 of an embodiment of the present invention inhibited the protein expression of mTOR and STAT3 (FIG. 23).

8-2. Identification of BCL2 and BI-1 Expression Inhibition of CA101

The expression inhibitory effect of the recombinant adenovirus CA101 prepared in Example 7 on the target genes BCL2 and BI-1 was identified. Specifically, U-87 cells (1 × $10^5$/well) were seeded on a 12-well plate. Then, one hour later, CA10G and CA101 each were treated at an MOI of 10 in each well, and 72 hours later, RNA prep was performed using an RNA prep kit (Takara, 9767A). Thereafter, RNA was quantified using Nanodirp, 400 ng/20 μl per tube was added using RT premix (intron, 25081), mixed well with the contents of the premix, and then cDNA was synthesized by reacting at 45°C for 1 hr and at 95°C for 5 minutes using a PCR device. The synthesized cDNA 2 μl was used as a template, and a PCR mixture (total volume, 20 μl) corresponding to an experimental group was made (template 2 μl, forward primer 0.5 μl (10 pmole/μl), reverse primer 0.5 μl (10 pmole/μl), 10 μl 2X master mix (Bioline, BIO-94005) and 7 μl DW). The prepared PCR mixture was vortexed, mixed well, and centrifuged, followed by reaction for 40 cycles of 5 minutes at 95°C, 10 seconds at 95°C, and 30 seconds at 60°C in a qPCR device (Applied Biosystems, QS3). The results were analyzed using the program in the qPCR device.

As a result, in U-87 cells, the recombinant adenovirus CA101 of an embodiment of the present invention, which encodes and expresses the hTERT promoter and BCL2 and BI-1 double target shRNA, was shown to significantly inhibit the expression of BCL2 and BI-1 genes compared to recombinant adenovirus CA10G including only the hTERT promoter (FIG. 24).

8-3. Identification of AR and mTOR Expression Inhibition of CA103

8-3-1. *in vitro*

The expression inhibitory effect of the recombinant adenovirus CA103 prepared in Example 7 on the target genes AR and mTOR was identified. Specifically, human prostate cancer cell lines LNcap, C42B and 22Rv1 were each seeded on a 12-well plate at 1 × $10^5$/well. Then, one hour later, CA10G and CA103 were treated at an MOI of 2 or 5 in each well, and 72 hours later, RNA prep was performed using an RNA prep kit (Takara, 9767A). Thereafter, RNA was quantified using Nanodirp, 400 ng/20 μl per tube was added using RT premix (intron, 25081), mixed well with the contents of the premix, and then cDNA was synthesized by reacting at 45°C for 1 hr and at 95°C for 5 minutes using a PCR device. The synthesized cDNA 2 μl was used as a template, and a PCR mixture (total volume, 20 μl) corresponding to an experimental group was made (template 2 μl, forward primer 0.5 μl (10 pmole/μl), reverse primer 0.5 μl (10 pmole/μl), 10 μl 2X master mix (Bioline, BIO-94005) and 7 μl DW). The prepared PCR mixture was vortexed, mixed well, and centrifuged, followed by reaction for 40 cycles of 5 minutes at 95°C, 10 seconds at 95°C, and 30 seconds at 60°C in a qPCR device (Applied Biosystems, QS3). The results were analyzed using the program in the qPCR device.

As a result, in LNcap cell lines, the recombinant adenovirus CA103 of an embodiment of the present invention, which encodes and expresses the hTERT promoter and AR and mTOR double target shRNA, was shown to significantly inhibit the expression of AR and mTOR genes compared to recombinant adenovirus CA10G including only the hTERT promoter (FIG. 25). In addition, also in C42B and 22Rv1 cell lines, it was shown that the recombinant adenovirus CA103 of an embodiment of the present invention significantly inhibited the expression of AR and mTOR genes compared to CA10G (FIG. 26).

8-3-2. *in vivo*

After subcutaneous transplantation of a prostate cancer cell line (22Rv-1) into balb c nu/nu mice to prepare a prostate cancer mouse model, the recombinant adenoviruses CA10G and CA103 prepared in Example 7 were administered directly intratumorally once ($2 \times 10^8$ pfu/spot, 3 times), the tumor was excised 21 days later, and the expression level of AR and mTOR genes in the tumor was identified by Western blot analysis and IHC analysis. As a result of Western blot analysis, the expression of mTOR and AR was reduced in the CA103-administered group compared to the control group and the CA10G-administered group. Also in the IHC analysis result, in the CA103-administered group, the fluorescence expression of mTOR and AR was reduced by 70% to 90% or more compared to the control group and the CA10G-administered group, identifying that CA103 effectively inhibited the expression of mTOR and AR, the target genes in the tumor (FIG. 27).

8-4. Identification of c-MET and PD-L1 Expression Inhibition of CA104

The expression inhibitory effect of the recombinant adenovirus CA104 prepared in Example 7 on the target genes c-MET and PD-L1 was identified. Specifically, A431 cell lines ($1 \times 10^5$/well) were seeded on a 12-well plate. Then, one hour later, CA10G and CA104 were treated at an MOI of 2 or 5 in each well, and 72 hours later, RNA prep was performed using an RNA prep kit (Takara, 9767A). Thereafter, RNA was quantified using Nanodirp, 400 ng/20 μl per tube was added using RT premix (intron, 25081), mixed well with the contents of the premix, and then cDNA was synthesized by reacting at 45°C for 1 hr and at 95°C for 5 minutes using a PCR device. The synthesized cDNA 2 μl was used as a template, and a PCR mixture (total volume, 20 μl) corresponding to an experimental group was made (template 2 μl, forward primer 0.5 μl (10 pmole/μl), reverse primer 0.5 μl (10 pmole/μl), 10 μl 2X master mix (Bioline, BIO-94005) and 7 μl DW). The prepared PCR mixture was vortexed, mixed well, and centrifuged, followed by reaction for 40 cycles of 5 minutes at 95°C, 10 seconds at 95°C, and 30 seconds at 60°C in a qPCR device (Applied Biosystems, QS3). The results were analyzed using the program in the qPCR device.

As a result, in A431 cells, the recombinant adenovirus CA104 of an embodiment of the present invention, which encodes and expresses the hTERT promoter and c-MET and PD-L1 double target shRNA, was shown to significantly inhibit the expression of c-MET and PD-L1 genes compared to recombinant adenovirus CA10G including only the hTERT promoter (FIG. 28).

**Example 9. Identification of Anticancer Effect of Double Target shRNA Coding Adenovirus**

9-1. Identification of Anticancer Effect of CA101

The cancer cell killing effects of the recombinant adenoviruses CA10G and CA101 prepared in Example 7 were compared. Specifically, after 1 hour after spreading U87MG cells ($5 \times 10^3$/well) on each of 96-well plates, CA10G and CA101 were treated at an MOI of 1, 2, 5, 10, 30, or 50 in each well, and then 72 hours later, an MTT reagent was added and incubated at 37°C for 3 hours. After 3 hours, the medium was removed from each well, and 100 μl of DMSO was added. Immediately thereafter, the absorbance was measured at a wavelength of 540 nm using a microplate reader, and MTT analysis was performed.

As a result, it was identified that CA101 of an embodiment of the present invention significantly killed cancer cells compared to CA10G (FIG. 29).

9-2. Identification of Anticancer Effect of CA102

9-2-1. Bladder Cancer

The cancer cell killing effects of the recombinant adenoviruses CA10G and CA102 prepared in Example 7 were compared. Specifically, after 1 hour after spreading T24 cells ($2.5 \times 10^3$/well), 253J-BV cells ($5 \times 10^3$/well) and human bladder epithelial cell line RT4 cells ($5 \times 10^3$/well) on each of 96-well plates, CA10G and CA102 were treated at an MOI of 1, 2, 5, 10, 20, or 50 in each well, and then 72 hours later, an MTT reagent was added and incubated at 37°C for 3 hours. After 3 hours, the medium was removed from each well, and 100 $\mu$l of DMSO was added. Immediately thereafter, the absorbance was measured at a wavelength of 540 nm using a microplate reader, and MTT analysis was performed.

As a result, it was identified that CA102 of an embodiment of the present invention significantly killed cancer cells compared to CA10G (FIG. 30).

9-2-2. Head and Neck Cancer

In order to identify the head and neck cancer cell killing effect of the recombinant adenovirus CA102 of an embodiment of the present invention, the recombinant adenoviruses CA10G and CA102 prepared in Example 4 were treated with the head and neck cancer cell lines HSC-2 and Fadu, and the cell killing effect was compared by MTT analysis. As a result, on the basis of 10 MOI treatment, it was found that CA10G treatment resulted in about 40% of cell death, whereas CA102 encoding and expressing mTOR and STAT3 double target shRNA induced at least 70% of cell death (FIG. 31).

9-2-3. Skin Squamous Carcinoma

In order to identify the skin squamous carcinoma cell killing effect of the recombinant adenovirus CA102 of an embodiment of the present invention, the recombinant adenoviruses CA10G and CA102 prepared in Example 4 were treated with the skin squamous carcinoma cell lines A431 and HSC-5, and the cell killing effect was compared by MTT analysis. As a result, on the basis of 10 MOI treatment, it was found that the cell killing effect of CA102 was significantly higher than that of CA10G (FIG. 32).

9-3. Identification of Anticancer Effect of CA103

The cancer cell killing effects of the recombinant adenoviruses CA10G and CA103 prepared in Example 7 were compared. Specifically, after 1 hour after seeding LNcap, C42B, and 22Rv1 cell lines on each of 96-well plates at $5 \times 10^3$/well, CA10G and CA103 were treated at an MOI of 1, 2, 5, 10, 20, 40, or 50 in each well, and then 72 hours later, an MTT reagent was added and incubated at 37°C for 3 hours. After 3 hours, the medium was removed from each well, and 100 $\mu$l of DMSO was added. Immediately thereafter, the absorbance was measured at a wavelength of 540 nm using a microplate reader, and MTT analysis was performed.

As a result, in the case of the LNcap cell line, it was identified that CA103 of an embodiment of the present invention significantly killed cancer cells compared to CA10G (FIG. 33). Also, in the case of the C42B and 22Rv1 cell lines, it was identified that CA103 of an embodiment of the present invention significantly killed cancer cells (FIG. 34).

**Example 10. Identification of *in vivo* Anticancer Effect of Double Target shRNA Coding Adenovirus**

10-1. Identification of Anticancer Effect of CA102

10-1-1. Anticancer Effect on Cancer Cells *in vivo*

In order to identify the anticancer effect of the recombinant adenovirus CA102 of an embodiment of the present invention on cancer cells in the body, $1.0 \times 10^7$ bladder cancer cell line 253J-BV and head and neck cancer cell line FaDu each were cultured on a 100 $mm^3$ plate, and then, the recombinant adenoviruses CA10G and CA102 of an embodiment of the present invention were treated for 1 hour at MOI of 2 and MOI of 5, respectively (a control group was treated with PBS), replaced with fresh medium, and cultured for 2 hours. Thereafter, cells were harvested, mixed with Matrigel at a ratio of 1 : 1 (v/v), and transplanted (Xenograft) into 6-week-old male Balb/c nu-nu mice, followed by observation for 32 days. As a result, in the case of 253J-BV, the size of cancer cells was significantly reduced in the group treated with CA10G at MOI of 2, and cancer cells were terminated in the group treated with CA102 at MOI of 2 (FIG. 35). In addition, in the case of FaDu, the size of cancer cells in the group treated with CA10G at MOI of 5 did not show a significant difference compared to the control group, but cancer cells were terminated in the group treated with CA102 at MOI of 5 (FIG. 36).

10-1-2. Anticancer Effect on Tumors Formed *in Vivo*

After transplanting 5.0 × $10^6$ bladder cancer cell line 253J-BV into Balb/c nu-nu 6-week-old male mice, the size was observed twice a week. When the average tumor size reached 150 to 200 $mm^3$, the average value of the size of each tumor was uniformly divided into each group, and 2.0 × $10^8$ PFU of each of CA10G and CA102 viruses was injected into the tumor, 3 times in total, once daily for 3 days. Thereafter, the tumor size was observed twice a week for 43 days. As a result, when CA10G was administered, it was identified that the tumor size decreased compared to the control group, but the growth rate of cancer cells increased again over time. When CA102 was administered, it was identified that the tumor size was significantly reduced compared to the control group and CA10G, and the growth of cancer cells was significantly inhibited over time (FIG. 37).

10-1-3. Anticancer Effect on Tumors Formed *in vivo* according to Number of Administration

After transplanting 5.0 × $10^6$ bladder cancer cell line 253J-BV into Balb/c nu-nu 6-week-old male mice, the size was observed twice a week. When the average tumor size reached 200 $mm^3$, the average value of the size of each tumor was uniformly divided into each group, and 1.0 × $10^8$ PFU of each of CA10G and CA102 viruses was injected into the tumor, 5 times in total, once daily for 3 days. Thereafter, the tumor size was observed twice a week for 42 days. As a result, it was identified that the size of the tumor was reduced with the number of times CA102 was administered, and that the growth of cancer cells was inhibited according to the number of times of administration (FIG. 38).

10-1-4. Anticancer Effect on Tumors Formed *in vivo* according to Dosage

After subcutaneous transplantation of the glioblastoma cell line (U-87) into Balb/c nu-nu 6-week-old male mice, the size of the tumor was observed. When the average tumor size reached 200 $mm^3$, the average value of each tumor size was uniformly divided into each group, and the dose of CA102 was changed for each group and administered directly into the tumor. Then, the volume and weight of the tumor were observed. As a result, it was identified that the tumor volume and weight were significantly reduced in the CA102-treated group compared to the CA10G-treated group, and the anticancer effect further increased as the dose increased (FIG. 39).

10-2. Identification of Anticancer Effect of CA103

After subcutaneous transplantation of a prostate cancer cell line (22Rv-1) into balb c nu/nu mice to prepare a prostate cancer mouse model, the recombinant adenoviruses CA10G and CA103 prepared in Example 7 were administered directly into the tumor (2 × $10^8$ pfu/spot, 3 times) respectively, and then the growth was observed. As a result, it was identified that the volume and weight of the tumor were significantly reduced in the group treated with CA103 compared to the untreated control group (buffer-treated group) and the vector control group (CA10G-treated group) (FIG. 40).

**Example 11. Identification of Effect by Co-Treatment with Double Target shRNA Coding Adenovirus and Anticancer Agent**

After subcutaneous transplantation of a bladder cancer cell line (253J-BV) into balb c nu/nu mice to prepare a bladder cancer mouse model, the recombinant adenoviruses CA10G and CA102 prepared in Example 7 were administered directly into the tumor respectively, and then the growth of the tumor was observed. As a result, it was identified that the volume and weight of the tumor were significantly reduced in the group treated with CA102 compared to the untreated control group (buffer-treated group) and the vector control group (CA10G-treated group), and that the anticancer effect was synergistically increased when the anticancer drug cisplatin was co-administered (FIG. 41).

**Claims**

**1.** An anti-tumor adenovirus including:

a human telomere promoter (hTERT); and
an expression cassette including a nucleotide sequence having a first nucleic acid as a target sequence and a nucleotide sequence having a second nucleic acid as a target sequence,

wherein, when expressed in cells or tissues, the nucleotide sequence having the first nucleic acid as the target sequence and the nucleotide sequence having the second nucleic acid as the target sequence are partially double-stranded to form an siRNA or shRNA targeting the first nucleic acid and the second nucleic acid.

2. The anti-tumor adenovirus of claim 1, wherein the human telomere promoter is operably linked with an endogenous gene of an adenovirus.

3. The anti-tumor adenovirus of claim 2, wherein the endogenous gene of the adenovirus has a structure of 5'ITR-C1-C2-C3-C4-C5 3'ITR, wherein:

the C1 includes E1A, E1B, or E1A-E1B;
the C2 includes E2B-L1-L2-L3-E2A-L4;
the C3 does not include E3 or includes E3;
the C4 includes L5; and
the C5 does not include E4 or includes E4.

4. The anti-tumor adenovirus of claim 1, wherein the expression cassette is located at a C3 region of an endogenous gene of an adenovirus.

5. The anti-tumor adenovirus of claim 1, wherein the hTERT promoter is operably linked with E1A and E1B of an endogenous gene of an adenovirus.

6. The anti-tumor adenovirus of claim 5, wherein an IRES sequence is further included between the E1A and the E1B.

7. The anti-tumor adenovirus of claim 1, wherein the expression cassette encodes shRNA.

8. The anti-tumor adenovirus of claim 7, wherein the shRNA simultaneously inhibits the first nucleic acid and the second nucleic acid.

9. The anti-tumor adenovirus of claim 1, wherein the nucleic acid is a cancer-related gene.

10. The anti-tumor adenovirus of claim 9, wherein the cancer-related gene is an oncogene whose expression is increased in cancer.

11. The anti-tumor adenovirus of claim 10, wherein the oncogene is an apoptosis-related gene, a transcription factor gene, a metastasis-related gene, an angiogenesis-related gene, or a tyrosine-kinase gene.

12. The anti-tumor adenovirus of claim 11, wherein the apoptosis-related gene is ABL1, AKT1, AKT2, BARD1, BAX, BCL11B, BCL2, BCL2A1, BCL2L1, BCL2L12, BCL3, BCL6, BIRC2, BIRC3, BIRC5, BRAF, CARD11, CAV1, CBL, CDC25A, CDKN1A, CFLAR, CNR2, CTNNB1, CUL4A, DAXX, DDIT3, E2F1, E2F3, E2F5, ESPL1, FOXO1, HDAC1, HSPA5, IGF1R, IGF2, JUN, JUNB, JUND, MALT1, MAP3K7, MCL1, MDM2, MDM4, MYB, MYC, NFKB2, NPM1, NTRK1, PAK1, PAX3, PML, PRKCA, PRKCE, PTK2B, RAF1, RHOA, TGFB1, TNFRSF1B, TP73, TRAF6, YWHAG, YWHAQ, or YWHAZ.

13. The anti-tumor adenovirus of claim 11, wherein the transcription factor gene is AR, ARID3A, ASCL1, ATF1, ATF3, BCL11A, BCL11B, BCL3, BCL6, CDC5L, CDX2, CREB1, CUX1, DDIT3, DLX5, E2F1, E2F3, E2F5, ELF4, ELK1, ELK3, EN2, ERG, ETS1, ETS2, ETV1, ETV3, ETV4, ETV6, FEV, FEZF1, FLI1, FOS, FOSL1, FOXA1, FOXG1, FOXM1, FOXO1, FOXP1, FOXQ1, GATA1, GATA6, GFI1, GFI1B, GLI1, GLI2, GLI3, HES6, HHEX, HLF, HMGA1, HMGA2, HOXA1, HOXA9, HOXD13, HOXD9, ID1, ID2, IKZF1, IRF2, IRF4, JUN, JUNB, JUND, KAT6A, KDM2A, KDM5B, KLF2, KLF4, KLF5, KLF6, KLF8, KMT2A, LEF1, LHX1, LMX1B, MAF, MAFA, MAFB, MBD1, MECOM, MEF2C, MEIS1, MITF, MYB, MYC, MYCL, MYCN, NANOG, NCOA3, NFIB, NFKB2, NKX2-1, OTX2, PATZ1, PAX2, PAX3, PAX4, PAX8, PBX1, PBX2, PITX2, PLAG1, PLAGL2, PPARG, PPP1R13L, PRDM10, PRDM13, PRDM14, PRDM15, PRDM16, PRDM6, PRDM8, PRDM9, RARA, REL, RERE, RUNX1, RUNX3, SALL4, SATB1, SFPQ, SIX1, SNAI1, SOX2, SOX4, SPI1, SREBF1, STAT3, TAF1, TAL1, TAL2, TBX2, TBX3, TCF3, TFCP2, TFE3, THRA, TLX1, TP63, TP73, TWIST1, WT1, YBX1, YY1, ZBTB16, ZBTB7A, ZIC2, ZNF217, or ZNF268.

14. The anti-tumor adenovirus of claim 11, wherein the metastasis-related gene is AKT1, AKT2, AR, CBL, CDH1, CRK, CSF1, CTNNB1, CTTN, CXCR4, EGFR, FGFR1, FLT3, FYN, GLI1, ILK, ITGA3, JAK2, MET, PDGFRB, PLXNB1, PRKCI, PTCH1, PTPN11, RAC1, RHOA, RHOC, ROCK1, SMO, SNAI1, SRC, TCF3, or WT1.

**15.** The anti-tumor adenovirus of claim 11, wherein the angiogenesis-related gene is BRAF, CAV1, CTGF, EGFR, ERBB2, ETS1, FGF4, FGF6, FGFR1, FGFR3, FGFR4, ID1, NRAS, PDGFB, PDGFRA, PDGFRB, or SPARC.

**16.** The anti-tumor adenovirus of claim 11, wherein the tyrosine-kinase gene is ABL1, ABL2, ALK, AXL, BLK, EGFR, EPHA2, ERBB2, ERBB3, ERBB4, FES, FGFR1, FGFR2, FGFR3, FGFR4, FGR, FLT3, FYN, ITK, JAK1, JAK2, KIT, LCK, MERTK, MET, MST1R, NTRK1, NTRK3, PDGFRA, PDGFRB, PTK2B, PTK7, RET, ROS1, SRC, SYK, TEC, or YES1.

**17.** The anti-tumor adenovirus of claim 10, wherein the oncogene is SEPTIN9, ACOD1, ACTN4, ADAM28, ADAM9, ADGRF1, ADRBK2, AFF1, AFF3, AGAP2, AGFG1, AGRN, AHCYL1, AHI1, AIMP2, AKAP13, AKAP9, AKIRIN2, AKTIP, ALDH1A1, ALL1, ANIB1, ANP32C, ANP32D, AQP1, ARAF, ARHGEF1, ARHGEF2, ARHGEF5, ASPSCR1, AURKA, BAALC, BAIAP2L1, BANP, BCAR4, BCKDHB, BCL9, BCL9L, BCR, BMI1, BMP7, BOC, BRD4, BRF2, CABIN1, CAMK1D, CAPG, CBFB, CBLB, CBLL1, CBX7, CBX8, CCDC28A, CCDC6, CCNB1, CCNB2, CCND1, CCNE1, CCNL1, CD24, CDC25C, CDC6, CDH17, CDK1, CDK14, CDK4, CDK5R2, CDK6, CDK8, CDKN1B, CDKN3, CDON, CEACAM6, CENPW, CHD1L, CHIC1, CHL1, CKS1B, CMC4, CNTN2, COPS3, COPS5, CRKL, CRLF2, CROT, CRTC1, CRYAB, CSF1R, CSF3, CSF3R, CSNK2A1, CSNK2A2, CT45A1, CTBP2, CTNND2, CTSZ, CUL7, CXCL1, CXCL2, CXCL3, CYGB, CYP24A1, DCD, DCUN1D1DDB2, DDHD2, DDX6, DEK, DIS3, DNPH1, DPPA2, DPPA4, DSG3, DUSP12, DUSP26, ECHS1, ECT2, EEF1A1, EEF1A2, EEF1D, EIF3E, EIF3I, EIF4E, EIF5A2, ELAVL1, ELL, EML4, EMSY, ENTPD5, EPCAM, EPS8, ERAS, ERGIC1, ERVW-1, EVI2A, EVI5, EWSR1, EZH2, FAM189B, FAM72A, FAM83D, FASN, FDPS, FGF10, FGF3, FGF5, FGF8, FR1OP, FHL2, FIP1L1, FNDC3B, FRAT1, FUBP1, FUS, FZD2, GAB2, GAEC1, GALNT10, GALR2, GLO1, GMNN, GNA12, GNA13, GNAI2, GNAQ, GNAS, GOLPH3, GOPC, GPAT4, GPM6A, GPM6B, GPR132, GREM1, GRM1, GSK3A, GSM1, H19, HAS1, HAX1, HDGFRP2, HMGN5, HNRNPA1, HOTAIR, HOTTIP, HOXA-AS2, HRAS, HSPA1A, HSPA4, HSPB1, HULC, IDH1, IFNG, IGF2BP1, IKBKE, IL7R, INPPL1, INTS1, INTS2, INTS3, INTS4, INTS5, INTS7, INTS8, IRS2, IST1, JUP, KDM4C, KIAA0101, KIAA1524, KIF14, KRAS, KSR2, LAMTOR5, LAPTM4B, LCN2, LDHB, LETMD1, LIN28A, LIN28B, LMO1, LMO2, LMO3, LMO4, LSM1, LUADT1, MACC1, MACROD1, MAGEA11, MALAT1, MAML2, MAP3K8, MAPRE1, MAS1, MCC, MCF2, MCF2L, MCTS1, MEFV, MFHAS1, MFNG, MIEN1, MINA, MKL2, MLANA, MLLT1, MLLT11, MLLT3, MLLT4, MMP12, MMS22L, MN1, MNAT1, MOS, MPL, MPST, MRAS, MRE11A, MSI1, MTCP1, MTDH, MTOR, MUC1, MUC4, MUM1, MYD88, NAAA, NANOGP8, NBPF12, NCOA4, NEAT1, NECTIN4, NEDD4, NEDD9, NET1, NINL, NME1, NOTCH1, NOTCH4, NOV, NSD1, NUAK2, NUP214, NUP98, NUTM1, OLR1, PA2G4, PADI2, PAK7, PARK7, PARM1, PBK, PCAT1, PCAT5, PDGFA, PDZK1IP1, PELP1, PFN1P3, PIGU, PIK3CA, PIK3R1, PIM1, PIM2, PIM3, PIR, PIWIL1, PLAC8, PLK1, PPM1D, PPP1R10, PPP1R14A, PPP2R1A, PRAME, PRDM12, PRMT5, PSIP1, PSMD10, PTCH2, PTMA, PTP4A1, PTP4A2, PTP4A3, PTTG1, PTTG1IP, PTTG2, PVT1, RAB11A, RAB18, RAB22A, RAB23, RAB8A, RALGDS, RAP1A, RASSF1, RBM14, RBM15, RBM3, RBMY1A1, RFC3, RGL4, RGR, RHO, RING1, RINT1, RIT1, RNF43, RPL23, RRAS, RRAS2, RSF1, RUNX1T1, S100A4, S100A7, S100A8, SAG, SART3, SBSN, SEA, SEC62, SERTAD1, SERTAD2, SERTAD3, SET, SETBP1, SETDB1, SGK1, SIRT1, SIRT6, SKI, SKIL, SKP2, SLC12A5, SLC3A2, SMR3B, SMURF1, SNCG, SNORA59A, SNORA80E, SPAG9, SPATA4, SPRY2, SQSTM1, SRSF1, SRSF2, SRSF3, SRSF6, SS18, SSX1, SSX2, SSX2B, STIL, STMN1, STRA6, STYK1, SUZ12, SWAP70, SYT1, TAC1, TACSTD2, TAF15, TALDO1, TAZ, TBC1D1, TBC1D15, TBC1D3, TBC1D3C, TBC1D7, TCL1A, TCL1B, TCL6, TCP1, TFG, TGM3, TINCR, TKTL1, TLE1, TMEM140, TMPOP2, TMPRSS2, TNS4, TPD52, TPR, TRE17, TREH, TRIB1, TRIB2, TRIM28, TRIM32, TRIM8, TRIO, TRIP6, TSPAN1, TSPY1, TXN, TYMS, TYRP1, UBE2C, UBE3C, UCA1, UCHL1, UHRF1, URI1, USP22, USP4, USP6, VAV1, VAV2, VAV3, VIM, WAPL, WHSC1, WHSC1L1, WISP1, WNT1, WNT10A, WNT10B, WNT2, WNT3, WNT5A, WWTR1, XCL1, XIAP, YAP1, YEATS4, YY1AP1, ZEB1-AS1, ZFAND4, ZFAS1, ZMYM2, ZNF703, or ZNHIT6.

**18.** The anti-tumor adenovirus of claim 1, wherein the nucleic acid is ABL1, AKT1, AKT2, BARD1, BAX, BCL11B, BCL2, BCL2A1, BCL2L1, BCL2L12, BCL3, BCL6, BIRC2, BIRC3, BIRC5, BRAF, CARD11, CAV1, CBL, CDC25A, CDKN1A, CFLAR, c-MET, CNR2, CTNNB1, CUL4A, DAXX, DDIT3, E2F1, E2F3, E2F5, ESPL1, FOXO1, HDAC1, HSPA5, IGF1R, IGF2, JUN, JUNB, JUND, MALT1, MAP3K7, MCL1, MDM2, MDM4, MYB, MYC, NFKB2, NPM1, NTRK1, PAK1, PAX3, PML, PRKCA, PRKCE, PTK2B, RAF1, RHOA, TGFB1, TNFRSF1B, TP73, TRAF6, YWHAG, YWHAQ, YWHAZ, AR, ARID3A, ASCL1, ATF1, ATF3, BCL11A, BCL11B, BCL3, BCL6, CDC5L, CDX2, CREB1, CUX1, DDIT3, DLX5, E2F1, E2F3, E2F5, ELF4, ELK1, ELK3, EN2, ERG, ETS1, ETS2, ETV1, ETV3, ETV4, ETV6, FEV, FEZF1, FLI1, FOS, FOSL1, FOXA1, FOXG1, FOXM1, FOXO1, FOXP1, FOXQ1, GATA1, GATA6, GFI1, GFI1B, GLI1, GLI2, GLI3, HES6, HHEX, HLF, HMGA1, HMGA2, HOXA1, HOXA9, HOXD13, HOXD9, ID1, ID2, IKZF1, IRF2, IRF4, JUN, JUNB, JUND, KAT6A, KDM2A, KDM5B, KLF2, KLF4, KLF5, KLF6, KLF8, KMT2A, LEF1, LHX1, LMX1B, MAF, MAFA, MAFB, MBD1, MECOM, MEF2C, MEIS1, MITF, MYB, MYC, MYCL, MYCN, NANOG, NCOA3, NFIB, NFKB2, NKX2-1, OTX2, PATZ1, PAX2, PAX3, PAX4, PAX8, PBX1, PBX2, PD-L1, PITX2, PLAG1, PLAGL2, PPARG, PPP1R13L, PRDM10, PRDM13, PRDM14, PRDM15, PRDM16, PRDM6, PRDM8, PRDM9, RARA, REL, RERE, RUNX1, RUNX3, SALL4, SATB1, SFPQ, SIX1, SNAI1, SOX2, SOX4, SPI1, SREBF1, STAT3, TAF1, TAL1, TAL2, TBX2, TBX3, TCF3, TFCP2, TFE3, THRA, TLX1, TP63, TP73, TWIST1, WT1, YBX1, YY1, ZBTB16, ZBTB7A, ZIC2, ZNF217, ZNF268, AKT1, AKT2, AR, CBL, CDH1, CRK, CSF1, CTNNB1, CTTN, CXCR4, EGFR, FGFR1, FLT3, FYN, GLI1, ILK, ITGA3, JAK2, MET, PDGFRB, PLXNB1, PRKCI, PTCH1, PTPN11, RAC1, RHOA, RHOC, ROCK1, SMO, SNAI1, SRC, TCF3, WT1, BRAF, CAV1, CTGF, EGFR, ERBB2, ETS1, FGF4, FGF6, FGFR1, FGFR3, FGFR4, ID1, NRAS, PDGFB, PDGFRA, PDGFRB, SPARC, ABL1, ABL2, ALK, AXL, BLK, EGFR, EPHA2, ERBB2, ERBB3, ERBB4, FES, FGFR1, FGFR2, FGFR3, FGFR4, FGR, FLT3, FYN, ITK, JAK1, JAK2, KIT, LCK, MERTK, MET, MST1R, NTRK1, NTRK3, PDGFRA, PDGFRB, PTK2B, PTK7, RET, ROS1, SRC, SYK, TEC, YES1, SEPTIN9, ACOD1, ACTN4, ADAM28, ADAM9, ADGRF1, ADRBK2, AFF1, AFF3, AGAP2, AGFG1, AGRN, AHCYL1, AHI1, AIMP2, AKAP13, AKAP9, AKIRIN2, AKTIP, ALDH1A1, ALL1, ANIB1, ANP32C, ANP32D, AQP1, ARAF, ARHGEF1, ARHGEF2, ARHGEF5, ASPSCR1, AURKA, BAALC, BAIAP2L1, BANP, BCAR4, BCKDHB, BCL9, BCL9L, BCR, BMI1, BMP7, BOC, BRD4, BRF2, CABIN1, CAMK1D, CAPG, CBFB, CBLB, CBLL1, CBX7, CBX8, CCDC28A, CCDC6, CCNB1, CCNB2, CCND1, CCNE1, CCNL1, CD24, CDC25C, CDC6, CDH17, CDK1, CDK14, CDK4, CDK5R2, CDK6, CDK8, CDKN1B, CDKN3, CDON, CEACAM6, CENPW, CHD1L, CHIC1, CHL1, CKS1B, CMC4, CNTN2, COPS3, COPS5, CRKL, CRLF2, CROT, CRTC1, CRYAB, CSF1R, CSF3, CSF3R, CSNK2A1, CSNK2A2, CT45A1, CTBP2, CTNND2, CTSZ, CUL7, CXCL1, CXCL2, CXCL3, CYGB, CYP24A1, DCD, DCUN1D1DDB2, DDHD2, DDX6, DEK, DIS3, DNPH1, DPPA2, DPPA4, DSG3, DUSP12, DUSP26, ECHS1, ECT2, EEF1A1, EEF1A2, EEF1D, EIF3E, EIF3I, EIF4E, EIF5A2, ELAVL1, ELL, EML4, EMSY, ENTPD5, EPCAM, EPS8, ERAS, ERGIC1, ERVW-1, EVI2A, EVI5, EWSR1, EZH2, FAM189B, FAM72A, FAM83D, FASN, FDPS, FGF10, FGF3, FGF5, FGF8, FR1OP, FHL2, FIP1L1, FNDC3B, FRAT1, FUBP1, FUS, FZD2, GAB2, GAEC1, GALNT10, GALR2, GLO1, GMNN, GNA12, GNA13, GNAI2, GNAQ, GNAS, GOLPH3, GOPC, GPAT4, GPM6A, GPM6B, GPR132, GREM1, GRM1, GSK3A, GSM1, H19, HAS1, HAX1, HDGFRP2, HMGN5, HNRNPA1, HOTAIR, HOTTIP, HOXA-AS2, HRAS, HSPA1A, HSPA4, HSPB1, HULC, IDH1, IFNG, IGF2BP1, IKBKE, IL7R, INPPL1, INTS1, INTS2, INTS3, INTS4, INTS5, INTS7, INTS8, IRS2, IST1, JUP, KDM4C, KIAA0101, KIAA1524, KIF14, KRAS, KSR2, LAMTOR5, LAPTM4B, LCN2, LDHB, LETMD1, LIN28A, LIN28B, LMO1, LMO2, LMO3, LMO4, LSM1, LUADT1, MACC1, MACROD1, MAGEA11, MALAT1, MAML2, MAP3K8, MAPRE1, MAS1, MCC, MCF2, MCF2L, MCTS1, MEFV, MFHAS1, MFNG, MIEN1, MINA, MKL2, MLANA, MLLT1, MLLT11, MLLT3, MLLT4, MMP12, MMS22L, MN1, MNAT1, MOS, MPL, MPST, MRAS, MRE11A, MSI1, MTCP1, MTDH, MTOR, MUC1, MUC4, MUM1, MYD88, NAAA, NANOGP8, NBPF12, NCOA4, NEAT1, NECTIN4, NEDD4, NEDD9, NET1, NINL, NME1, NOTCH1, NOTCH4, NOV, NSD1, NUAK2, NUP214, NUP98, NUTM1, OLR1, PA2G4, PADI2, PAK7, PARK7, PARM1, PBK, PCAT1, PCAT5, PDGFA, PDZK1IP1, PELP1, PFN1P3, PIGU, PIK3CA, PIK3R1, PIM1, PIM2, PIM3, PIR, PIWIL1, PLAC8, PLK1, PPM1D, PPP1R10, PPP1R14A, PPP2R1A, PRAME, PRDM12, PRMT5, PSIP1, PSMD10, PTCH2, PTMA, PTP4A1, PTP4A2, PTP4A3, PTTG1, PTTG1IP, PTTG2, PVT1, RAB11A, RAB18, RAB22A, RAB23, RAB8A, RALGDS, RAP1A, RASSF1, RBM14, RBM15, RBM3, RBMY1A1, RFC3, RGL4, RGR, RHO, RING1, RINT1, RIT1, RNF43, RPL23, RRAS, RRAS2, RSF1, RUNX1T1, S100A4, S100A7, S100A8, SAG, SART3, SBSN, SEA, SEC62, SERTAD1, SERTAD2, SERTAD3, SET, SETBP1, SETDB1, SGK1, SIRT1, SIRT6, SKI, SKIL, SKP2, SLC12A5, SLC3A2, SMR3B, SMURF1, SNCG, SNORA59A, SNORA80E, SPAG9, SPATA4, SPRY2, SQSTM1, SRSF1, SRSF2, SRSF3, SRSF6, SS18, SSX1, SSX2, SSX2B, STIL, STMN1, STRA6, STYK1, SUZ12, SWAP70, SYT1, TAC1, TACSTD2, TAF15, TALDO1, TAZ, TBC1D1, TBC1D15, TBC1D3, TBC1D3C, TBC1D7, TCL1A, TCL1B, TCL6, TCP1, TFG, TGM3, TINCR, TKTL1, TLE1, TMEM140, TMPOP2, TMPRSS2, TNS4, TPD52, TPR, TRE17, TREH, TRIB1, TRIB2, TRIM28, TRIM32, TRIM8, TRIO, TRIP6, TSPAN1, TSPY1, TXN, TYMS, TYRP1, UBE2C, UBE3C, UCA1, UCHL1, UHRF1, URI1, USP22, USP4, USP6, VAV1, VAV2, VAV3, VIM, WAPL, WHSC1, WHSC1L1, WISP1, WNT1, WNT10A, WNT10B, WNT2, WNT3, WNT5A, WWTR1, XCL1, XIAP, YAP1, YEATS4, YY1AP1, ZEB1-AS1, ZFAND4, ZFAS1, ZMYM2, ZNF703, or ZNHIT6.

**19.** The anti-tumor adenovirus of claim 1, wherein the first nucleic acid and the second nucleic acid are different from each other.

**20.** The anti-tumor adenovirus of claim 1, wherein the first nucleic acid includes a nucleotide sequence having at least 60% complementarity with a reverse complementary sequence of the second nucleic acid.

**21.** The anti-tumor adenovirus of claim 1, wherein the second nucleic acid includes a nucleotide sequence having at least 60% complementarity with a reverse complementary sequence of the first nucleic acid.

**22.** The anti-tumor adenovirus of claim 1, wherein the expression cassette includes a nucleotide sequence sequentially encoding a nucleotide sequence having a first nucleic acid as a target sequence, a loop sequence capable of forming a hairpin structure, and a nucleotide sequence having a second nucleic acid as a target sequence.

**23.** The anti-tumor adenovirus of claim 1, wherein an expression of the expression cassette is regulated by a U6 promoter.

**24.** The anti-tumor adenovirus of claim 1, wherein the adenovirus is a group C adenovirus.

**25.** The anti-tumor adenovirus of claim 1, wherein a serotype is type 5.

**26.** The anti-tumor adenovirus of claim 1, wherein the anti-tumor adenovirus has a high oncolytic ability compared to a wild-type adenovirus.

**27.** The anti-tumor adenovirus of claim 1, wherein the anti-tumor adenovirus has a high oncolytic ability compared to an adenovirus in which the hTERT promoter is introduced into a wild-type adenovirus.

**28.** A composition for treating cancer including the anti-tumor adenovirus of claim 1.

**29.** The composition of claim 28, further comprising an anticancer agent.

**30.** A use of the anti-tumor adenovirus of claim 1 for preventing or treating a tumor.

**31.** A method of treating a tumor using the anti-tumor adenovirus of claim 1.

Fig.1

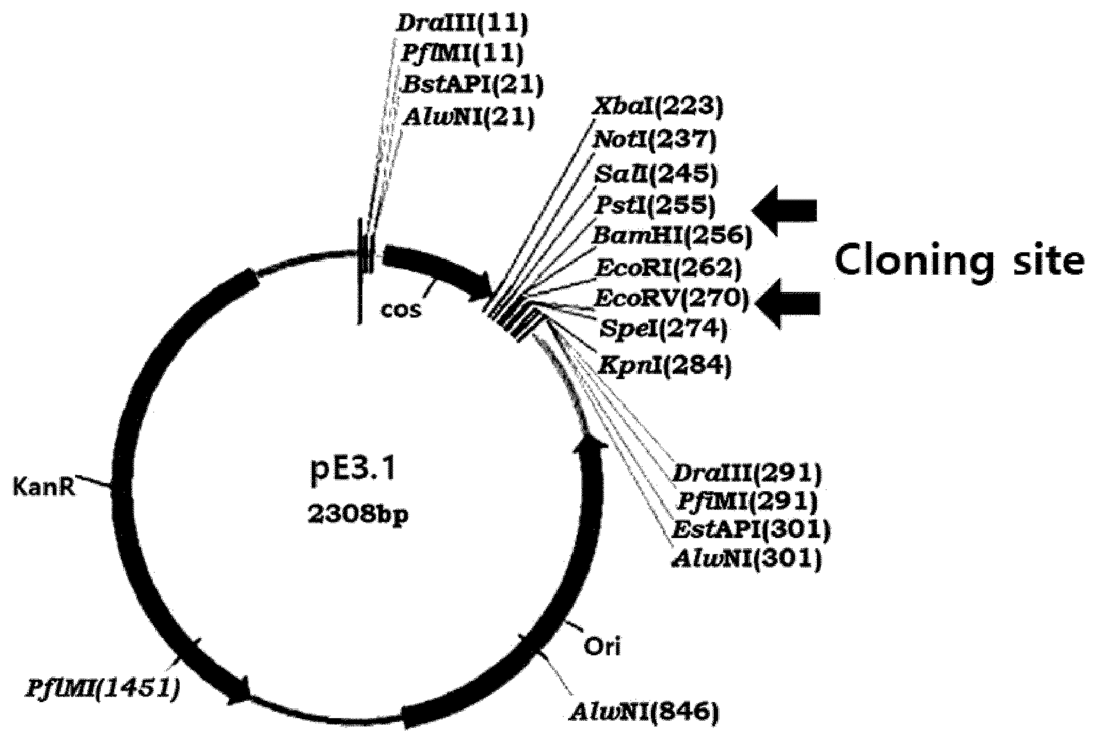
DraIII(11)
PflMI(11)
BstAPI(21)
AlwNI(21)
XbaI(223)
NotI(237)
SalI(245)
PstI(255)
BamHI(256)
EcoRI(262)
EcoRV(270)
SpeI(274)
KpnI(284)
Cloning site
cos
pE3.1
2308bp
KanR
DraIII(291)
PflMI(291)
EstAPI(301)
AlwNI(301)
Ori
PflMI(1451)
AlwNI(846)

Fig.2

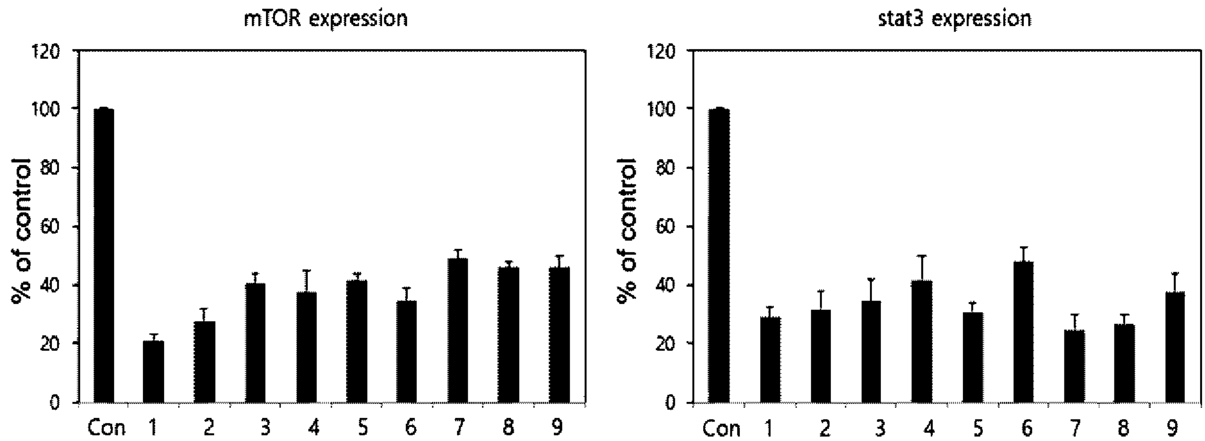
mTOR expression
stat3 expression
% of control
0
20
40
60
80
100
120
Con
1
2
3
4
5
6
7
8
9

Fig.3

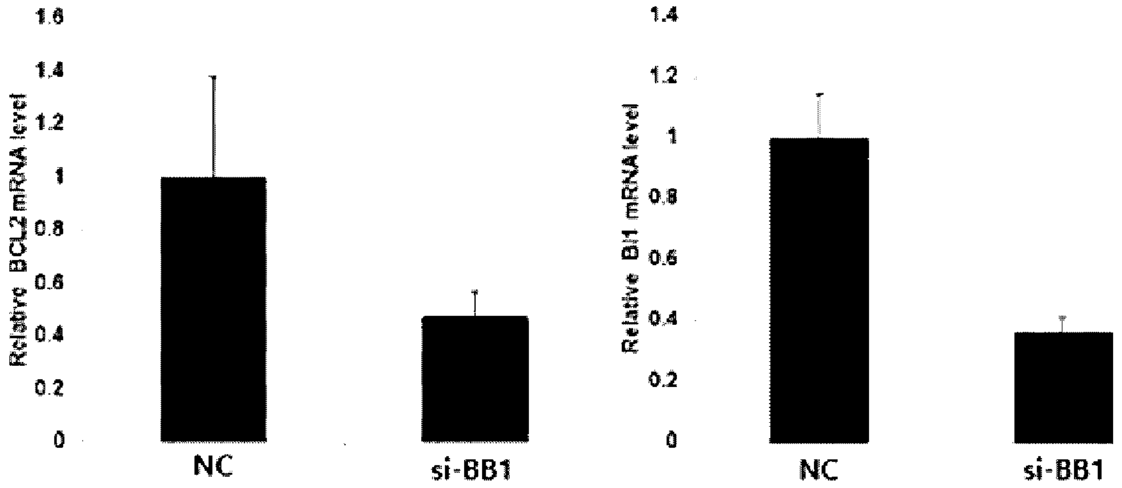


| | BCL2 | stud dev | BI-1 | stud dev |
|---|---|---|---|---|
| NC | 1 | 0.377795 | 1 | 0.143635 |
| si-BB1 | 0.471846 | 0.09471 | 0.361987 | 0.052985 |

Fig.4

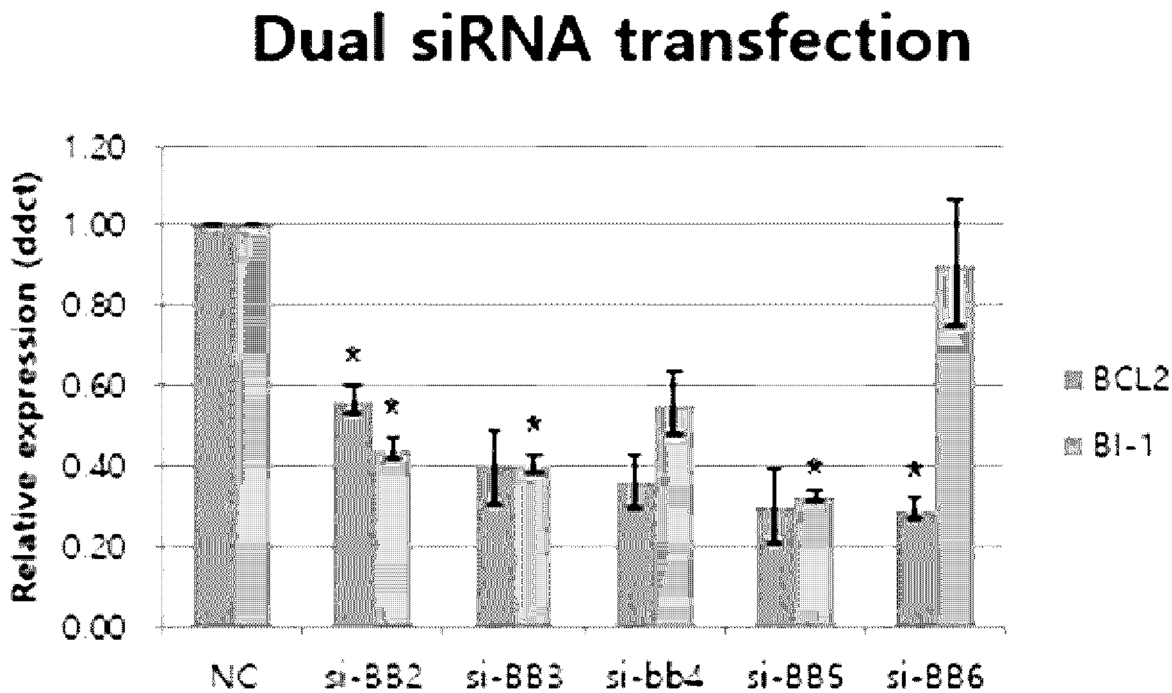

Fig.5

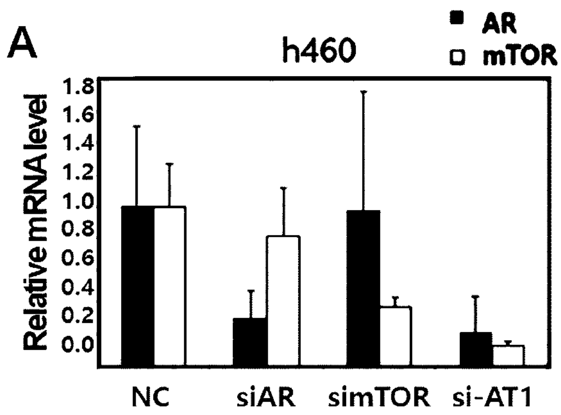


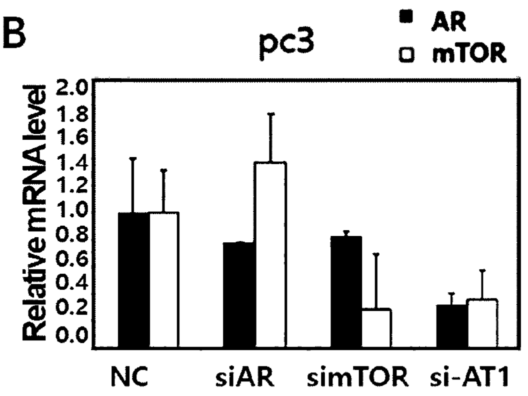


| | AR | std dev | mTOR | std dev |
|---|---|---|---|---|
| NC | 1.0000 | 0.505044 | 1.0000 | 0.268226 |
| siAR | 0.2996 | 0.168051 | 0.8174 | 0.298036 |
| simTOR | 0.9699 | 0.747167 | 0.3716 | 0.05666 |
| si-AT1 | 0.2071 | 0.231052 | 0.1240 | 0.031018 |

| | AR | std dev | mTOR | std dev |
|---|---|---|---|---|
| NC | 1.0000 | 0.399525 | 1.0000 | 0.306402 |
| siAR | 0.7830 | 0.001484 | 1.3722 | 0.357067 |
| simTOR | 0.8289 | 0.029483 | 0.2825 | 0.412371 |
| si-AT1 | 0.3174 | 0.08554 | 0.3601 | 0.215689 |

Fig.6

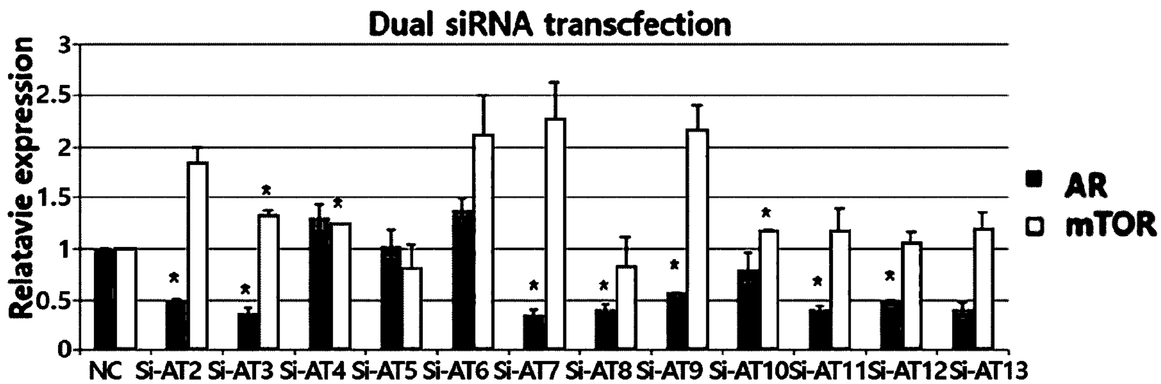

Fig.7

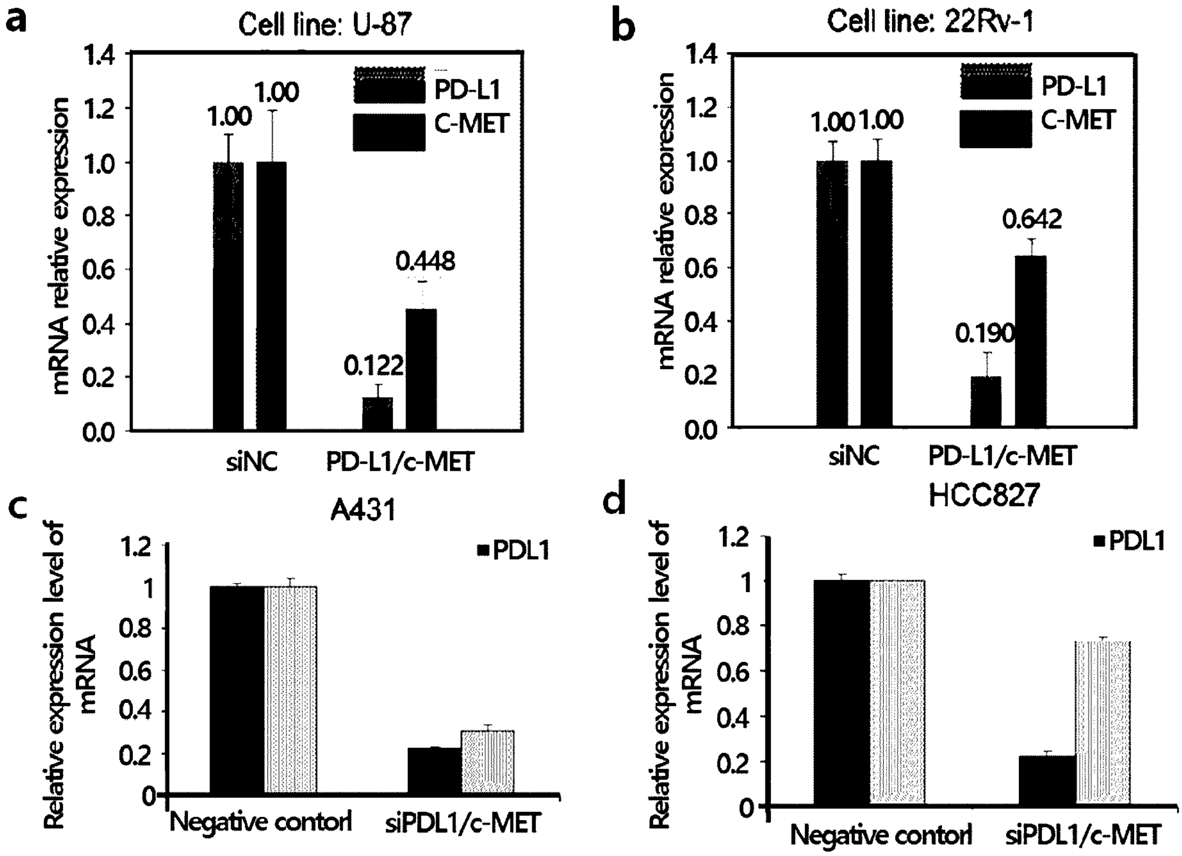
a
Cell line: U-87
PD-L1
C-MET
1.00
1.00
0.448
0.122
mRNA relative expression
1.4
1.2
1.0
0.8
0.6
0.4
0.2
0.0
siNC
PD-L1/c-MET
b
Cell line: 22Rv-1
PD-L1
C-MET
1.00 1.00
0.642
0.190
mRNA relative expression
siNC
PD-L1/c-MET
c
A431
■PDL1
Relative expression level of mRNA
1.2
1
0.8
0.6
0.4
0.2
0
Negative contorl
siPDL1/c-MET
d
HCC827
■PDL1
Relative expression level of mRNA
Negative contorl
siPDL1/c-MET

Fig.8

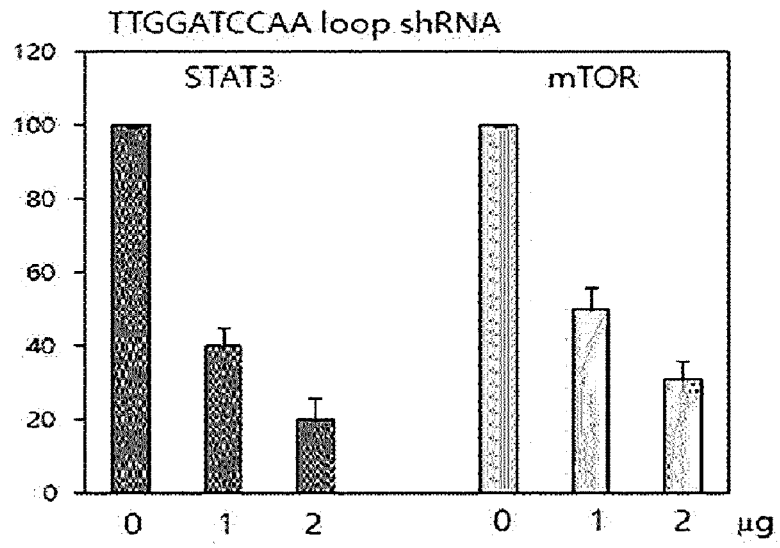
TTGGATCCAA loop shRNA
120
100
80
60
40
20
0
STAT3
mTOR
0
1
2
0
1
2
µg

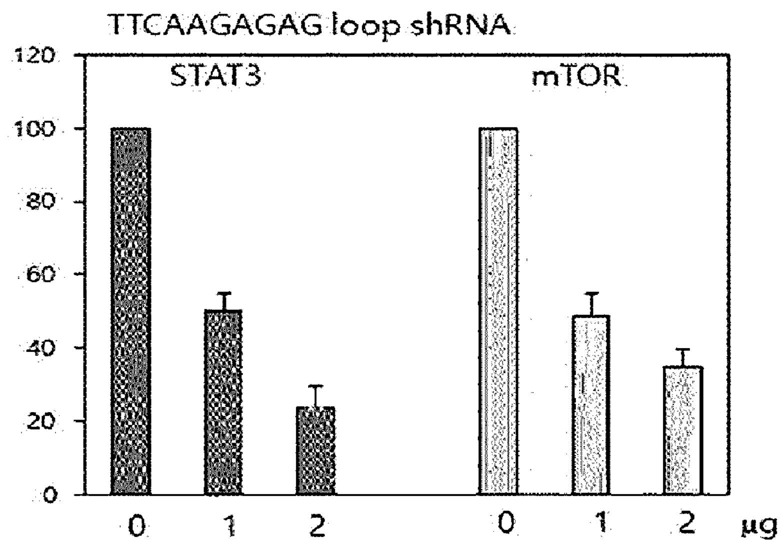
TTCAAGAGAG loop shRNA
120
100
80
60
40
20
0
STAT3
mTOR
0
1
2
0
1
2
µg

Fig.9

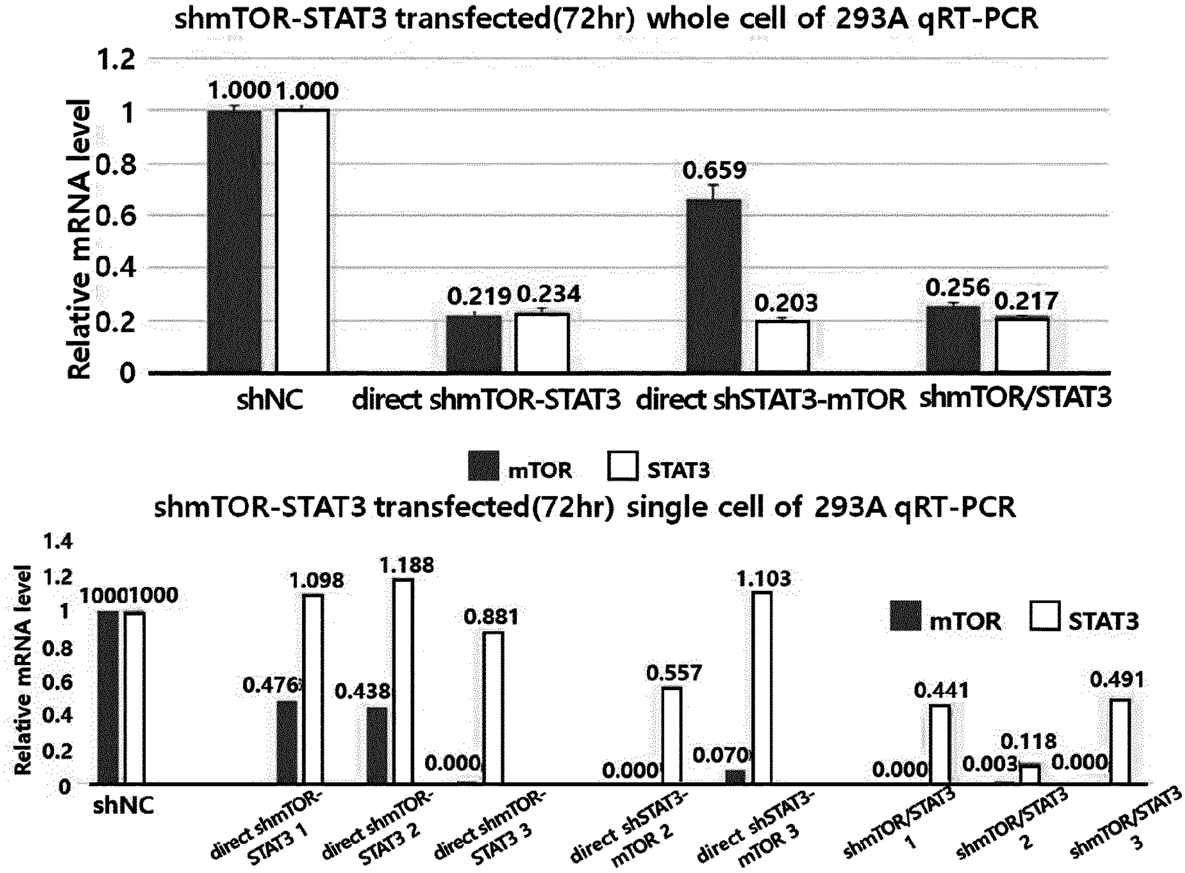
shmTOR-STAT3 transfected(72hr) whole cell of 293A qRT-PCR
Relative mRNA level
1.000 1.000
0.219 0.234
0.659
0.203
0.256 0.217
shNC
direct shmTOR-STAT3
direct shSTAT3-mTOR
shmTOR/STAT3
mTOR STAT3
shmTOR-STAT3 transfected(72hr) single cell of 293A qRT-PCR
Relative mRNA level
10001000
0.476 1.098
0.438 1.188
0.000 0.881
0.000 0.557
0.070 1.103
0.000 0.441
0.003 0.118
0.000 0.491
mTOR STAT3
shNC
direct shmTOR-STAT3 1
direct shmTOR-STAT3 2
direct shmTOR-STAT3 3
direct shSTAT3-mTOR 2
direct shSTAT3-mTOR 3
shmTOR/STAT3 1
shmTOR/STAT3 2
shmTOR/STAT3 3

Fig.10

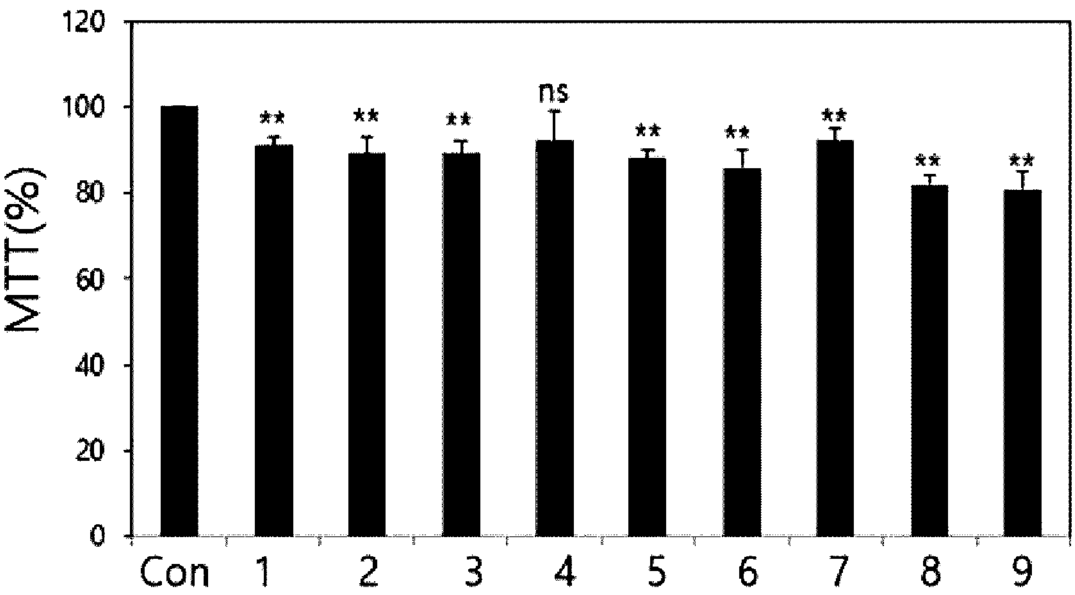
MTT(%)
**
ns
Con
1
2
3
4
5
6
7
8
9

Fig.11

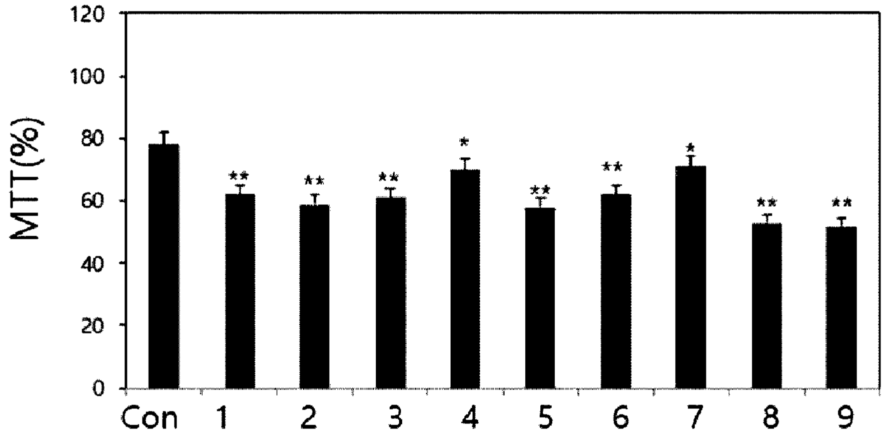


Fig.12

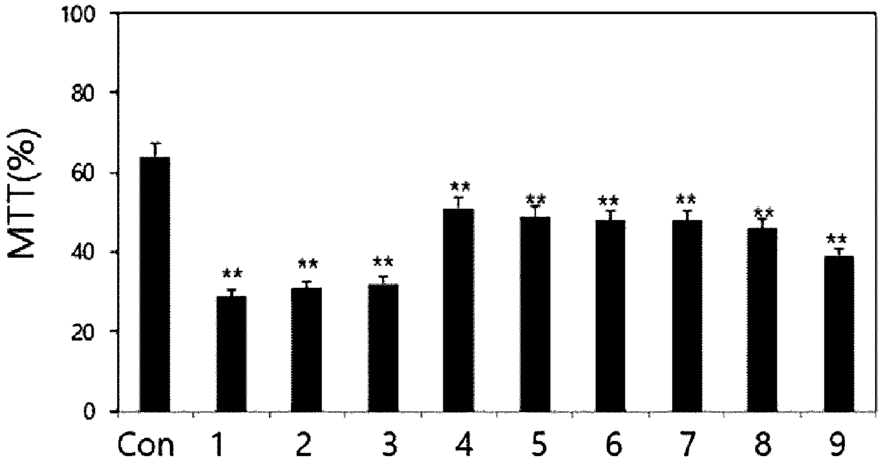


Fig.13

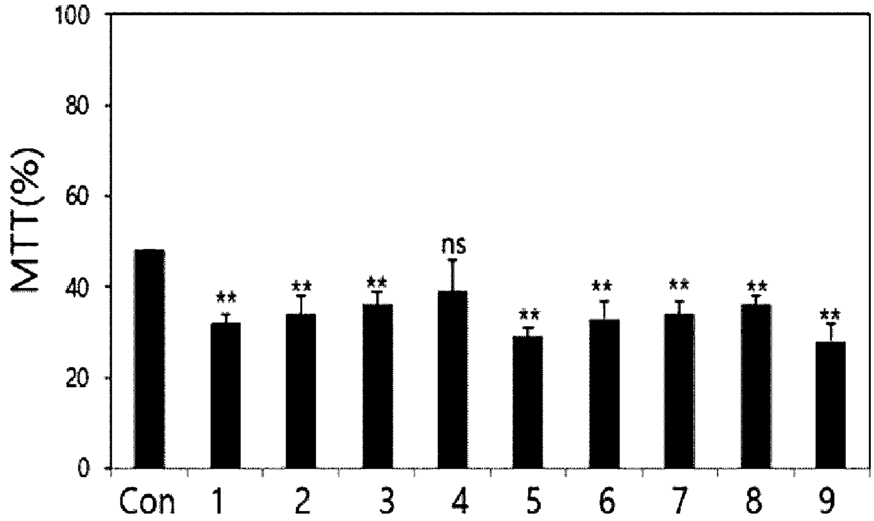

Fig.14

**A**

**031816 siRNA efficacy test(MTT, Hela, 6hr)**

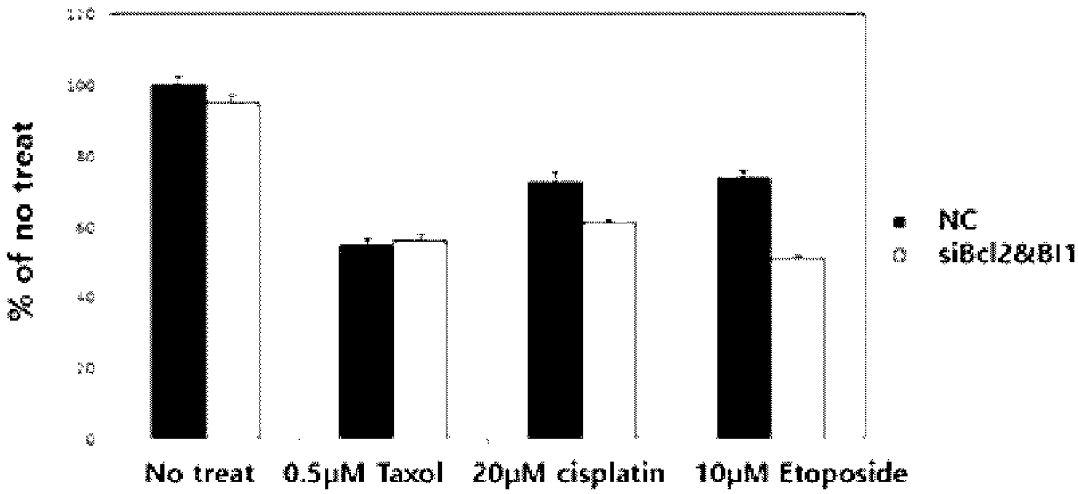


| % of no treat | NC | siBcl2&BI1 |
|---|---|---|
| no treat | 100 | 94.88511 |
| 0.5μM Taxol | 54.75789 | 56.17292 |
| 20μM cisplatin | 72.50422 | 60.88537 |
| 10μM Etoposide | 73.64663 | 51.05803 |

**B**

**032516 siRNA efficacy test(MTT, Hela, 6hr)**

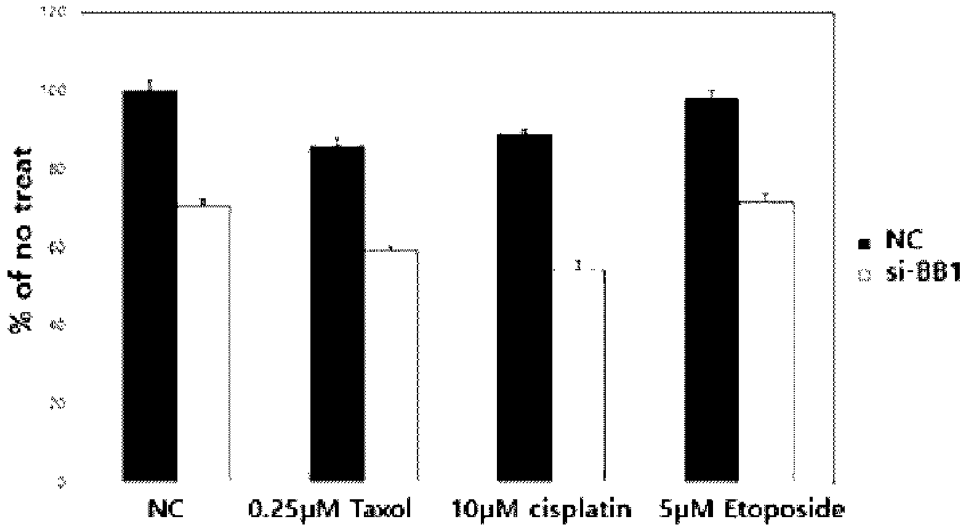


| % of no treat | NC | si-BB1 |
|---|---|---|
| no treat | 100 | 70.5518 |
| 0.25μM Taxol | 85.6258 | 59.16184 |
| 10μM cisplatin | 88.77896 | 53.9897 |
| 5μM Etoposide | 97.9408 | 71.64575 |

Fig.15

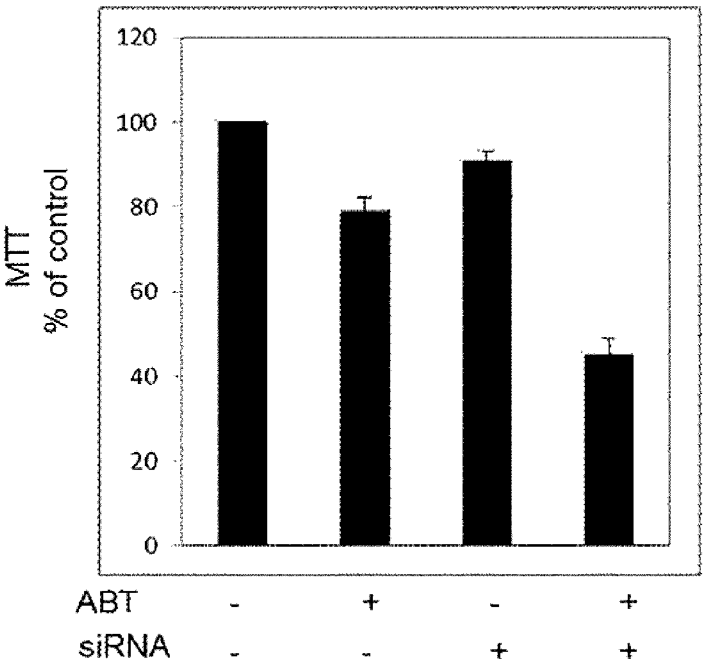
120
100
80
60
40
20
0
MTT
% of control
ABT - + - +
siRNA - - + +

Fig.16

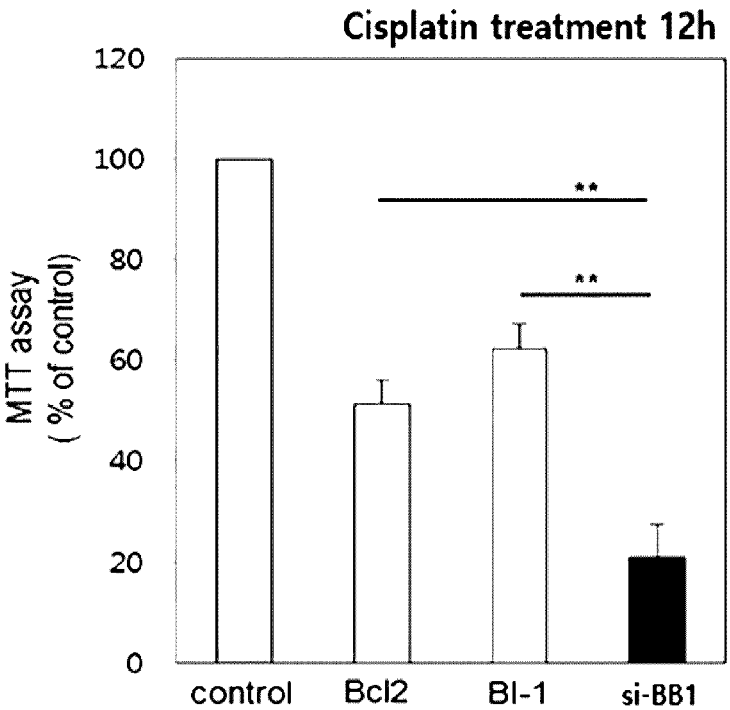
Cisplatin treatment 12h
120
100
80
60
40
20
0
MTT assay
( % of control)
**
**
control
Bcl2
BI-1
si-BB1

Fig.17

A

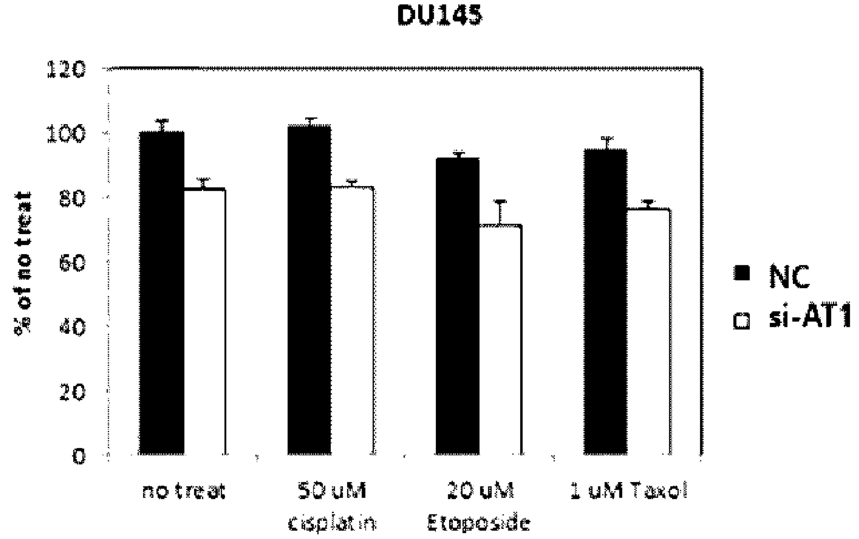


| % of no treat | | |
|---|---|---|
| si-AT1 | NC | si-AT1 |
| no treat | 100 | 82.42259 |
| 50μM cisplatin | 101.9776 | 83.22925 |
| 20μM Etoposide | 91.94639 | 70.92115 |
| 1μM Taxol | 94.00208 | 76.02134 |

B

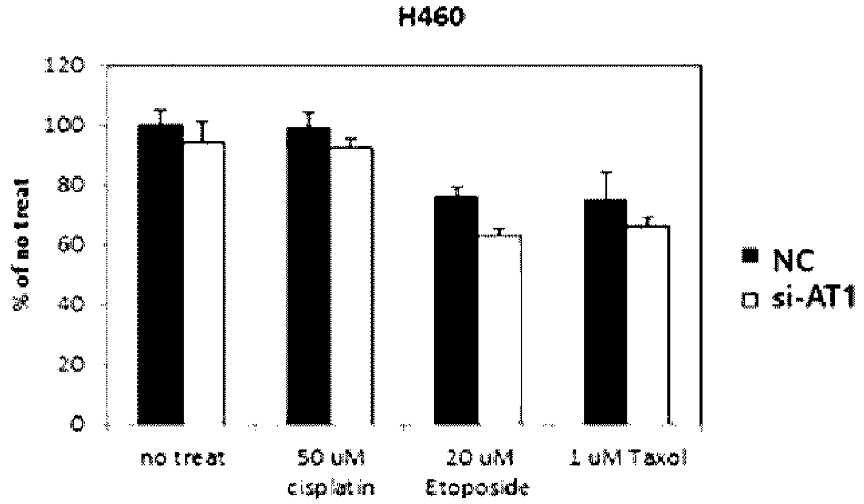


| % of no treat | | |
|---|---|---|
| si-AT1 | NC | si-AT1 |
| no treat | 100 | 94.01997 |
| 50μM cisplatin | 99.45916 | 92.2527 |
| 20μM Etoposide | 76.45173 | 62.75417 |
| 1μM Taxol | 75.1786 | 65.83715 |

Fig.18

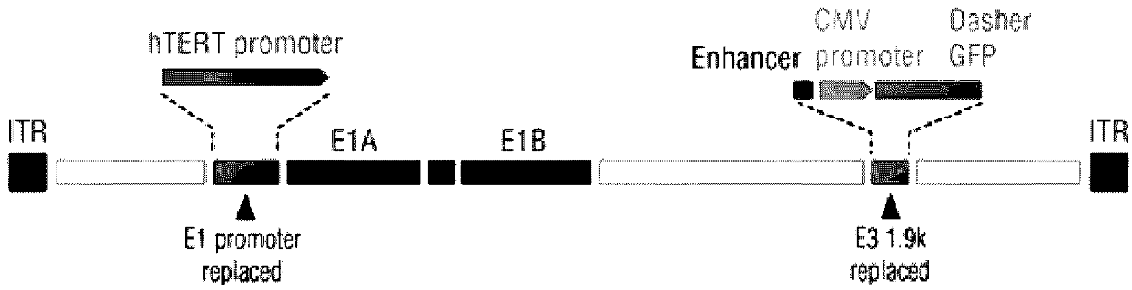


Fig.19

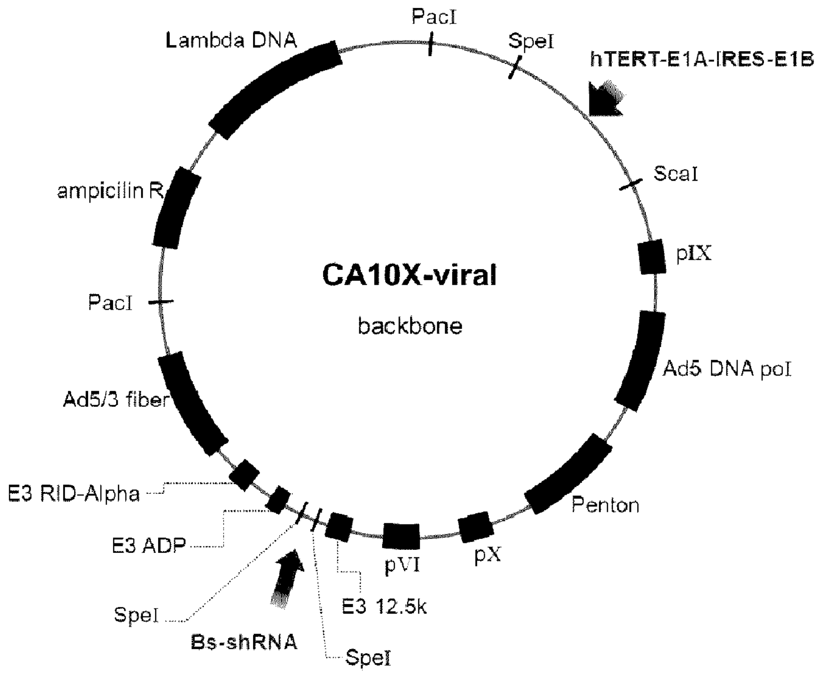


Fig.20

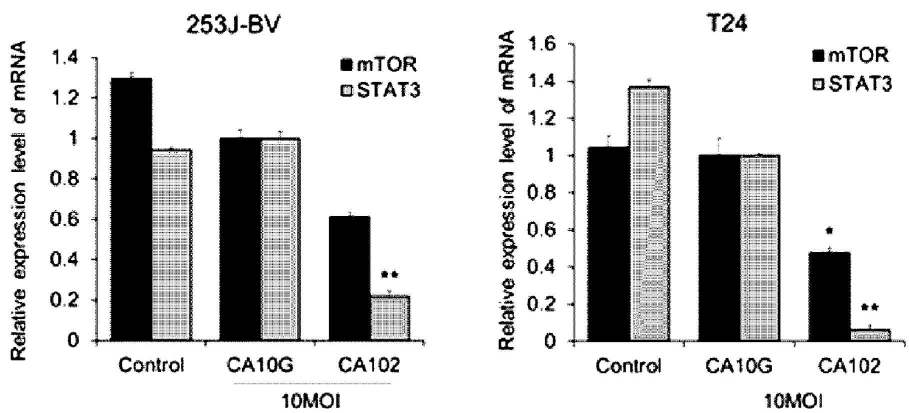

Fig.21

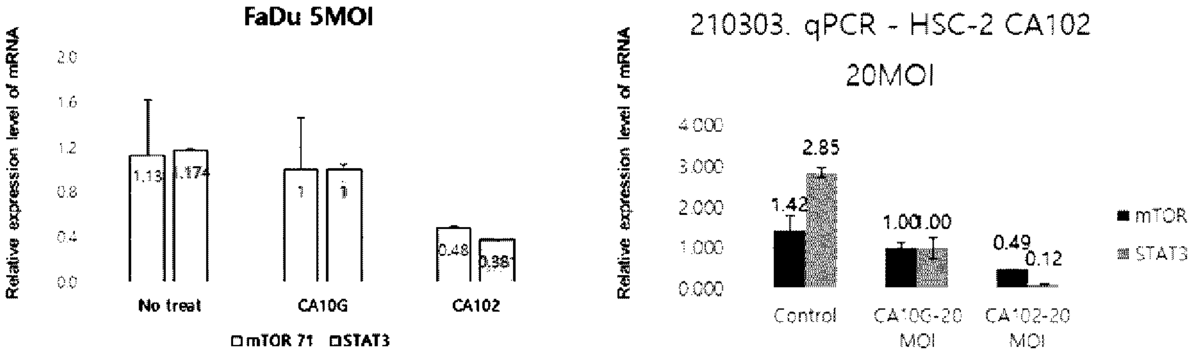


Fig.22

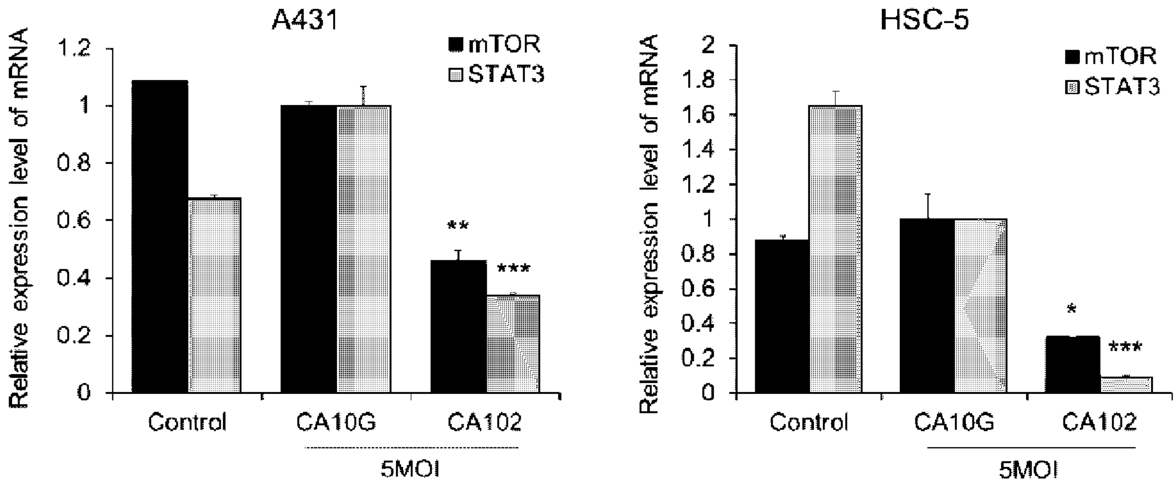


Fig.23

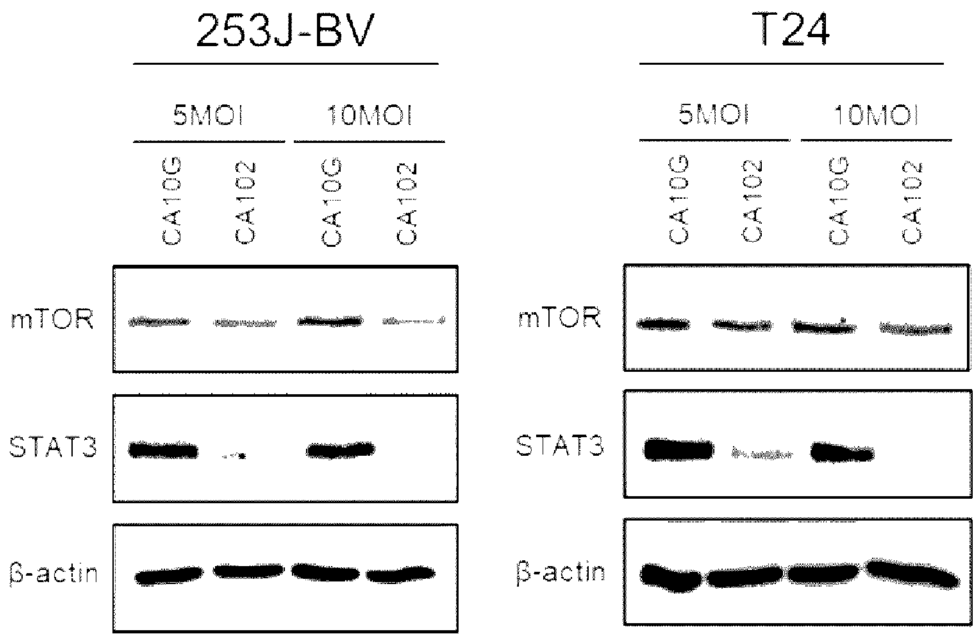

Fig.24

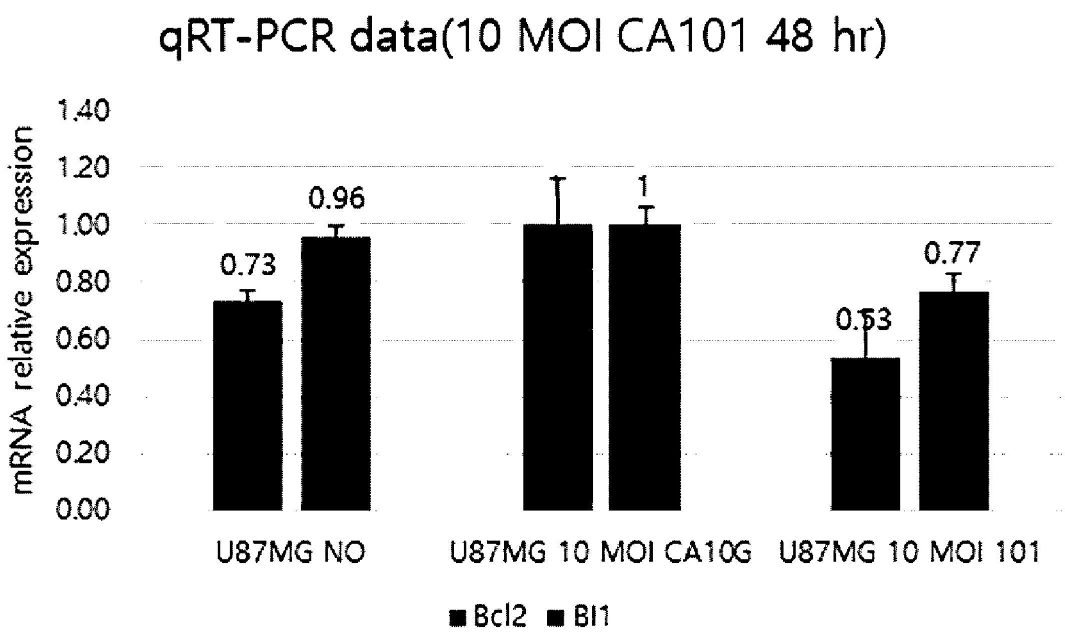
qRT-PCR data(10 MOI CA101 48 hr)
mRNA relative expression
1.40
1.20
1.00
0.80
0.60
0.40
0.20
0.00
0.73
0.96
1
0.77
U87MG NO
U87MG 10 MOI CA10G
U87MG 10 MOI 101
Bcl2
BI1

Fig.25

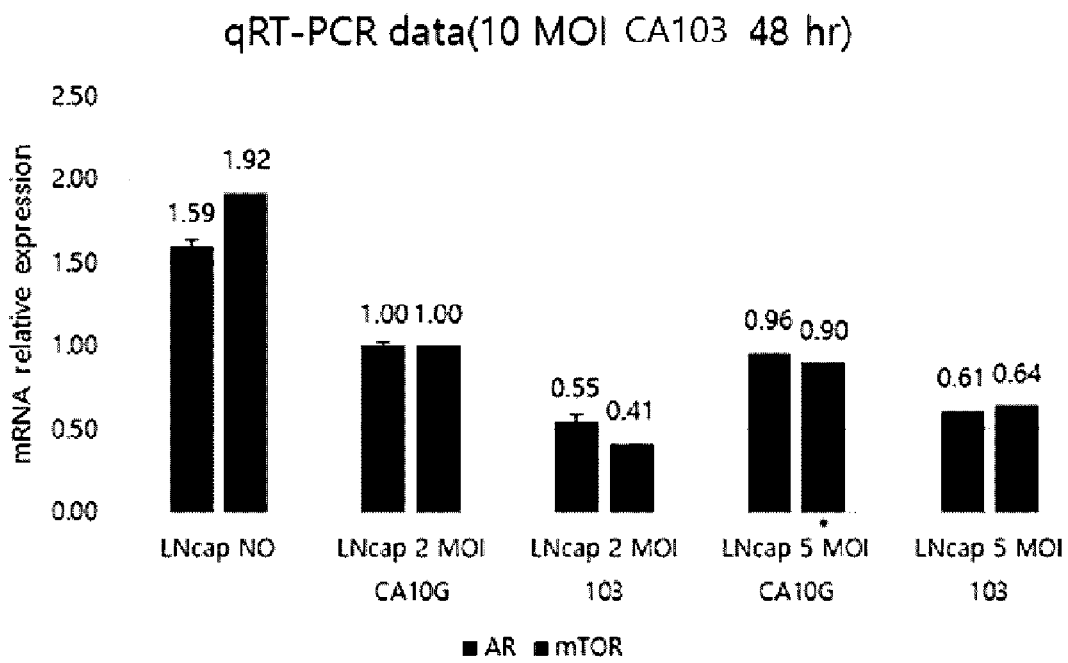
qRT-PCR data(10 MOI CA103 48 hr)
mRNA relative expression
2.50
2.00
1.50
1.00
0.50
0.00
1.59
1.92
1.00 1.00
0.55
0.41
0.96
0.90
0.61
0.64
LNcap NO
LNcap 2 MOI CA10G
LNcap 2 MOI 103
LNcap 5 MOI CA10G
LNcap 5 MOI 103
AR
mTOR

Fig.26

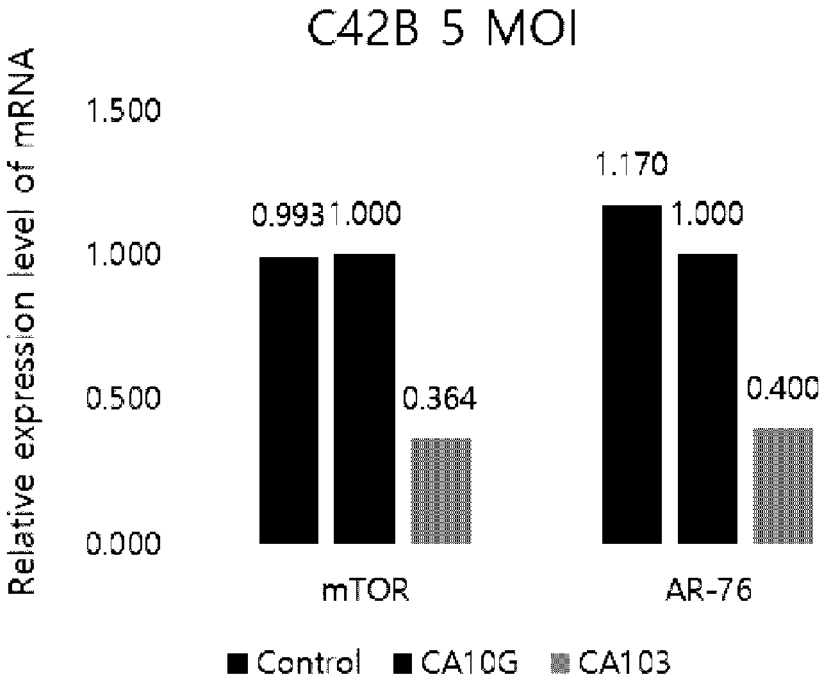
C42B 5 MOI
Relative expression level of mRNA
1.500
1.000
0.500
0.000
0.993
1.000
0.364
1.170
1.000
0.400
mTOR
AR-76
Control
CA10G
CA103

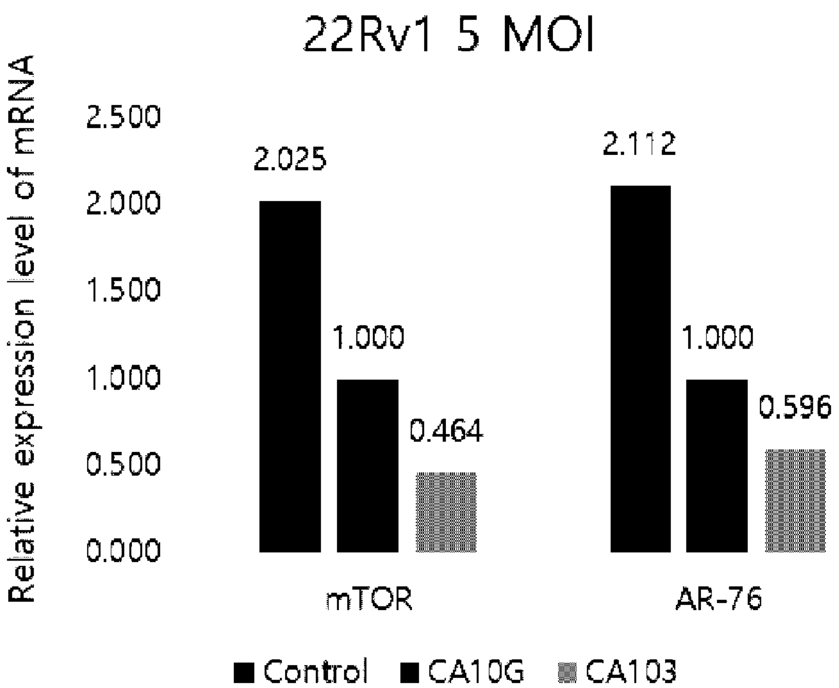
22Rv1 5 MOI
Relative expression level of mRNA
2.500
2.000
1.500
1.000
0.500
0.000
2.025
1.000
0.464
2.112
1.000
0.596
mTOR
AR-76
Control
CA10G
CA103

Fig.27

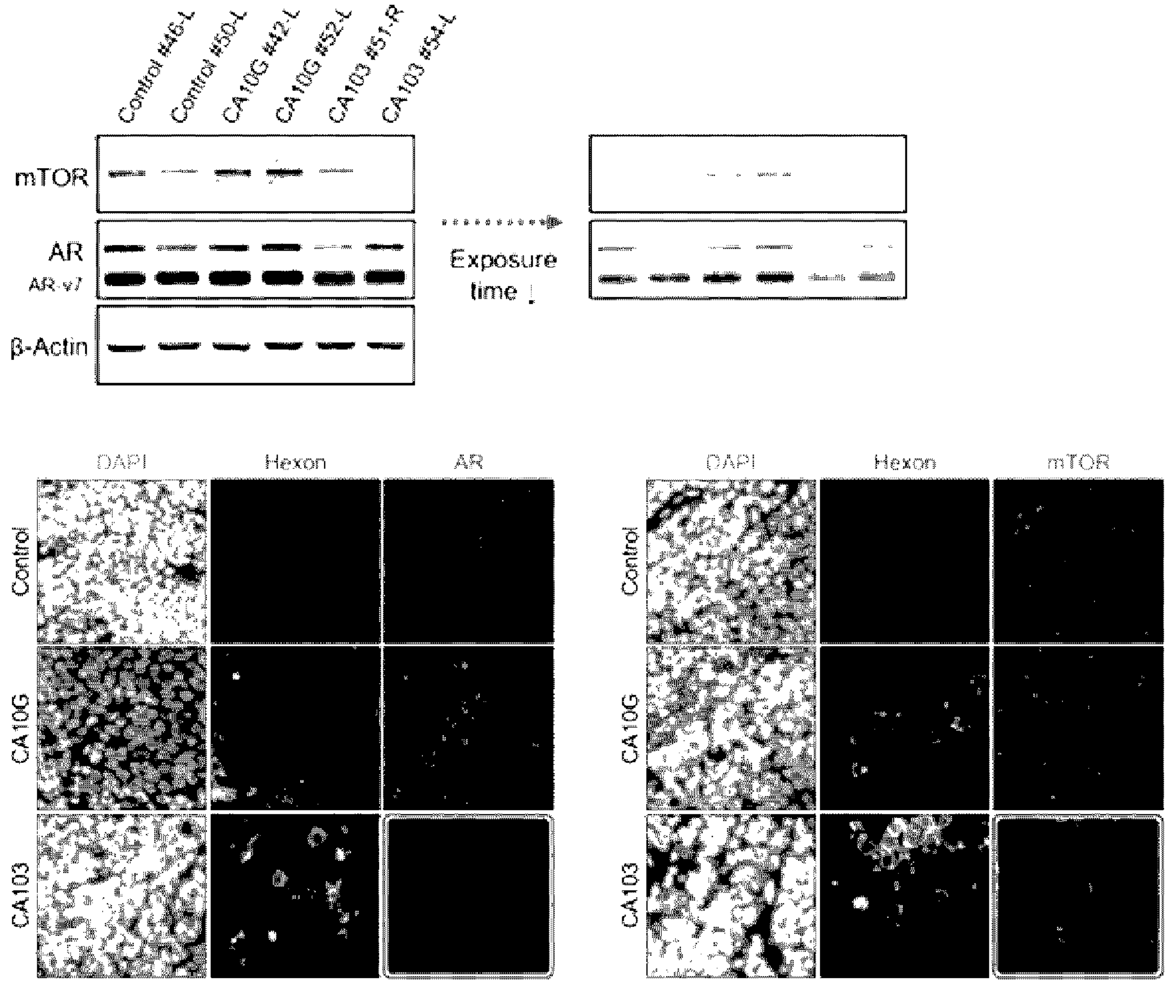
Control #46-L
Control #50-L
CA10G #42-L
CA10G #52-L
CA103 #51-R
CA103 #54-L
mTOR
AR
AR-v7
β-Actin
Exposure
time
DAPI
Hexon
AR
mTOR
Control
CA10G
CA103

Fig.28

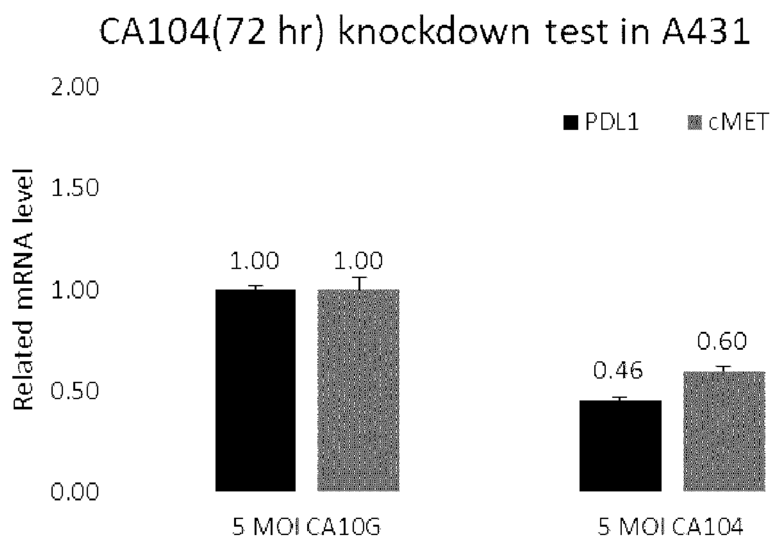
CA104(72 hr) knockdown test in A431
PDL1
cMET
Related mRNA level
2.00
1.50
1.00
0.50
0.00
1.00
1.00
0.46
0.60
5 MOI CA10G
5 MOI CA104

Fig.29

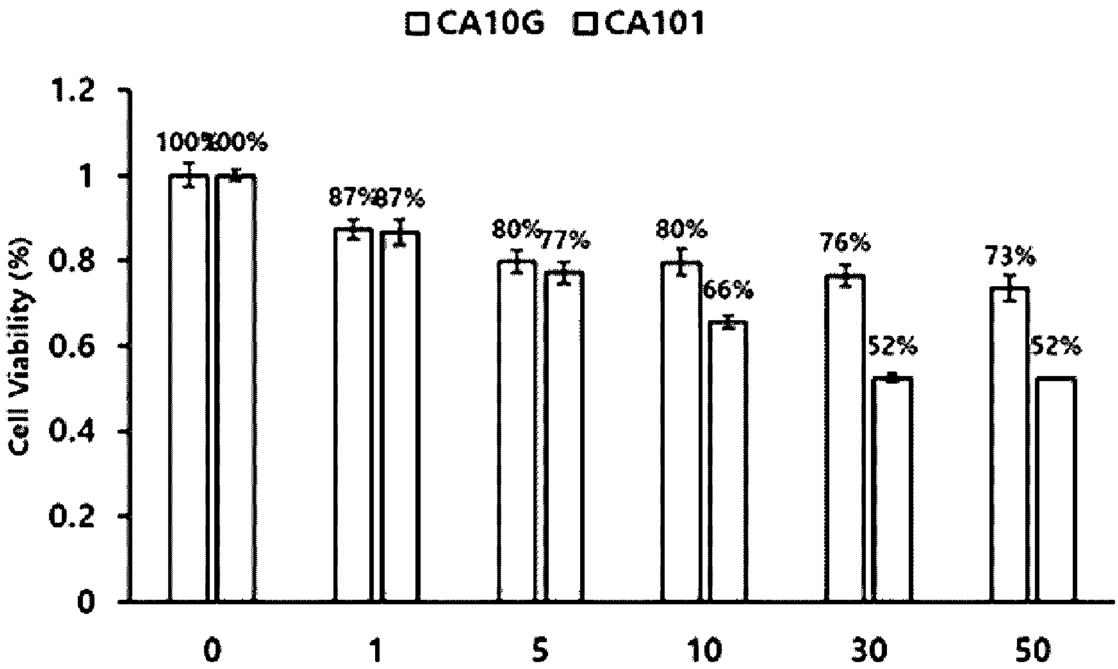
CA10G
CA101
1.2
1
0.8
0.6
0.4
0.2
0
Cell Viability (%)
100%
100%
87%
87%
80%
77%
80%
66%
76%
52%
73%
52%
0
1
5
10
30
50

Fig.30

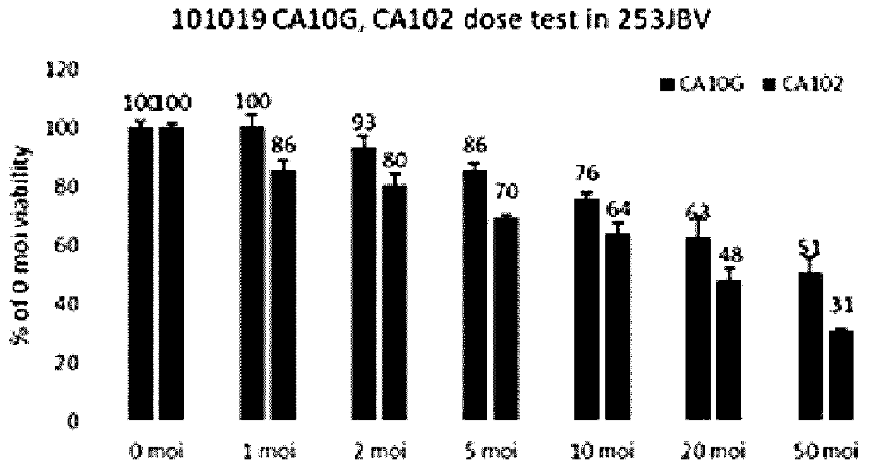
101019 CA10G, CA102 dose test in 253JBV
% of 0 moi viability
0
20
40
60
80
100
120
CA10G
CA102
100
100
100
86
93
80
86
70
76
64
63
48
51
31
0 moi
1 moi
2 moi
5 moi
10 moi
20 moi
50 moi

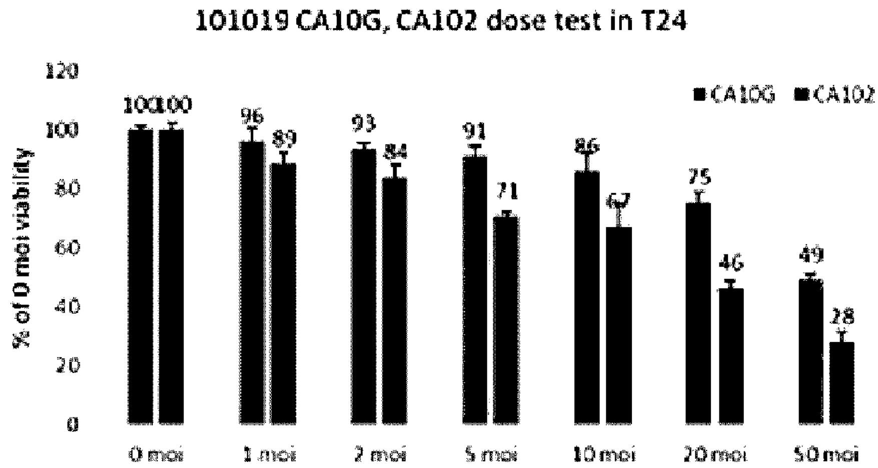
101019 CA10G, CA102 dose test in T24
% of 0 moi viability
0
20
40
60
80
100
120
CA10G
CA102
100
100
96
89
93
84
91
71
86
67
75
46
49
28
0 moi
1 moi
2 moi
5 moi
10 moi
20 moi
50 moi

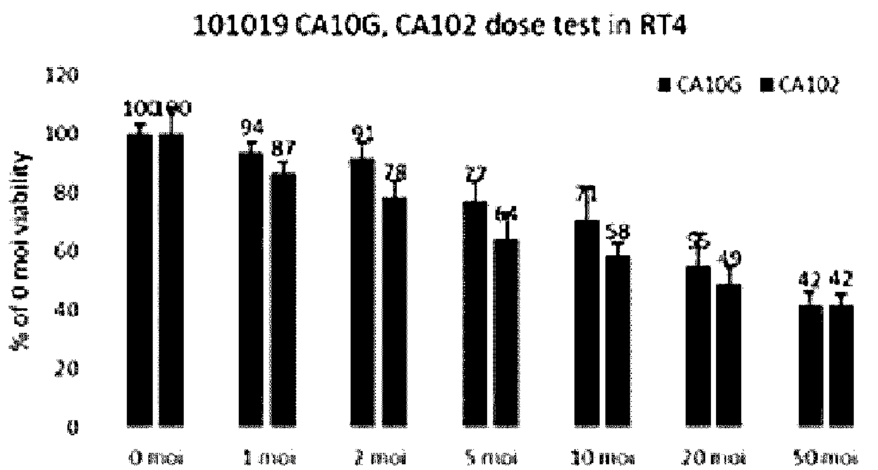
101019 CA10G, CA102 dose test in RT4
% of 0 moi viability
0
20
40
60
80
100
120
CA10G
CA102
100
100
94
87
91
78
77
64
71
58
55
49
42
42
0 moi
1 moi
2 moi
5 moi
10 moi
20 moi
50 moi

Fig.31

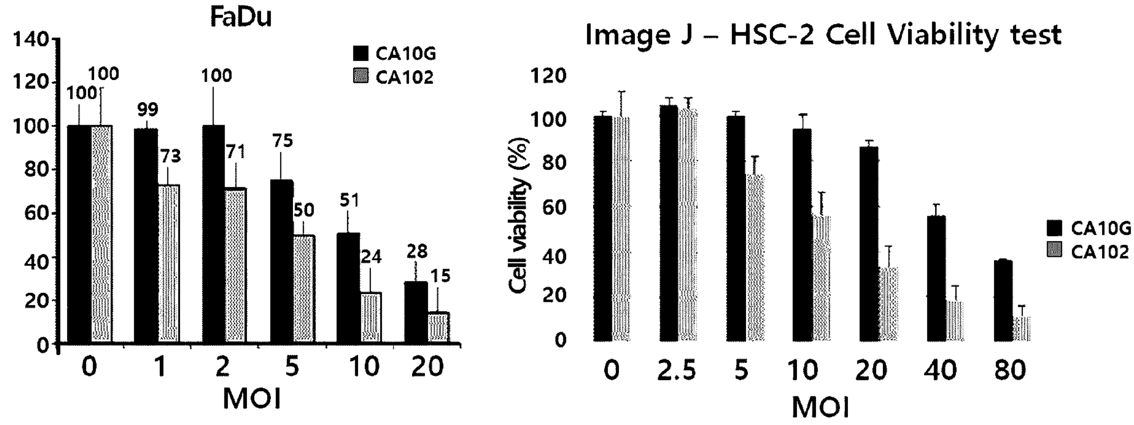


Fig.32

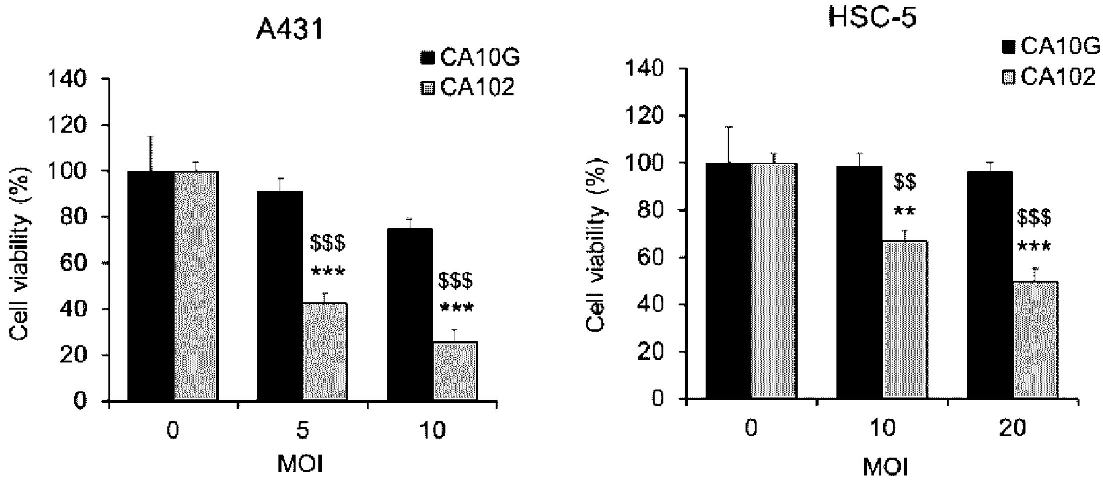


Fig.33

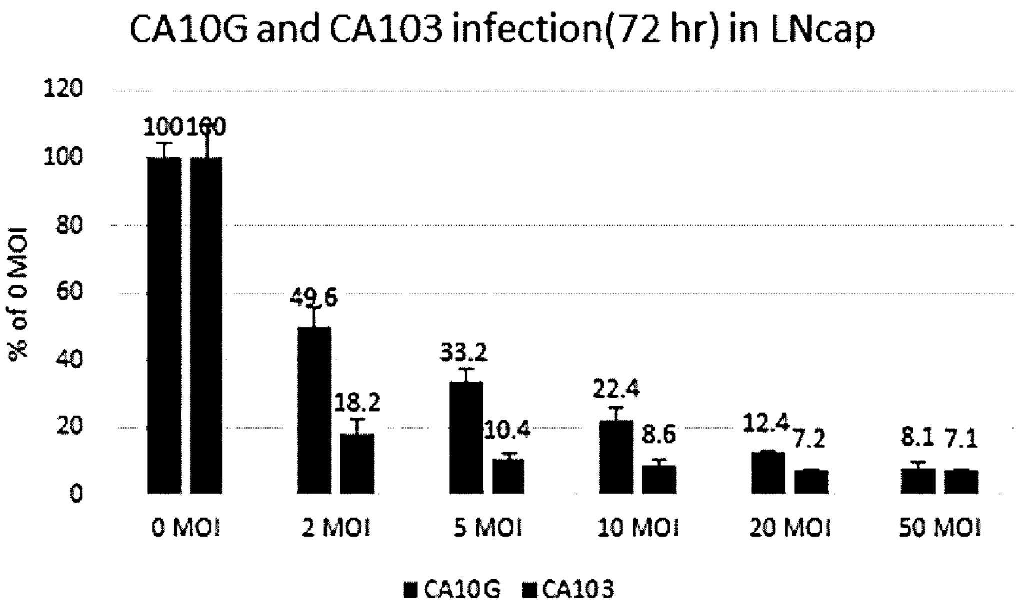

Fig.34

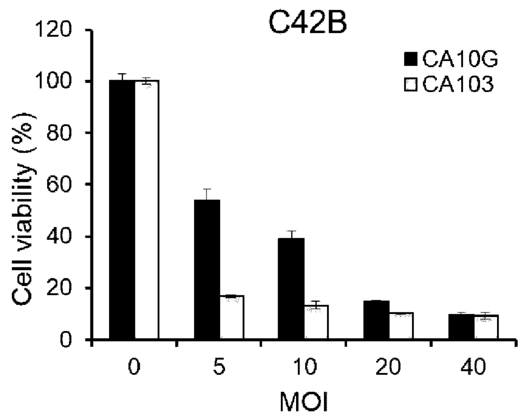
C42B
CA10G
CA103
Cell viability (%)
120
100
80
60
40
20
0
0
5
10
20
40
MOI

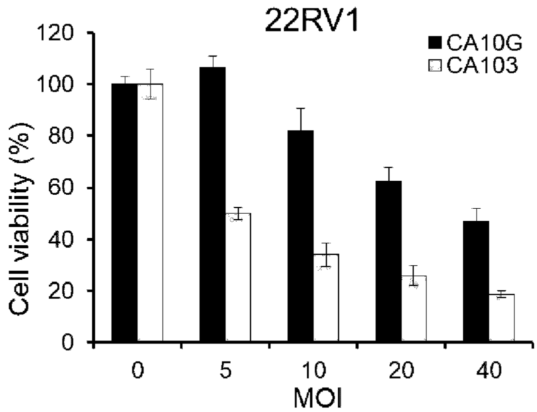
22RV1
CA10G
CA103
Cell viability (%)
120
100
80
60
40
20
0
0
5
10
20
40
MOI

Fig.35

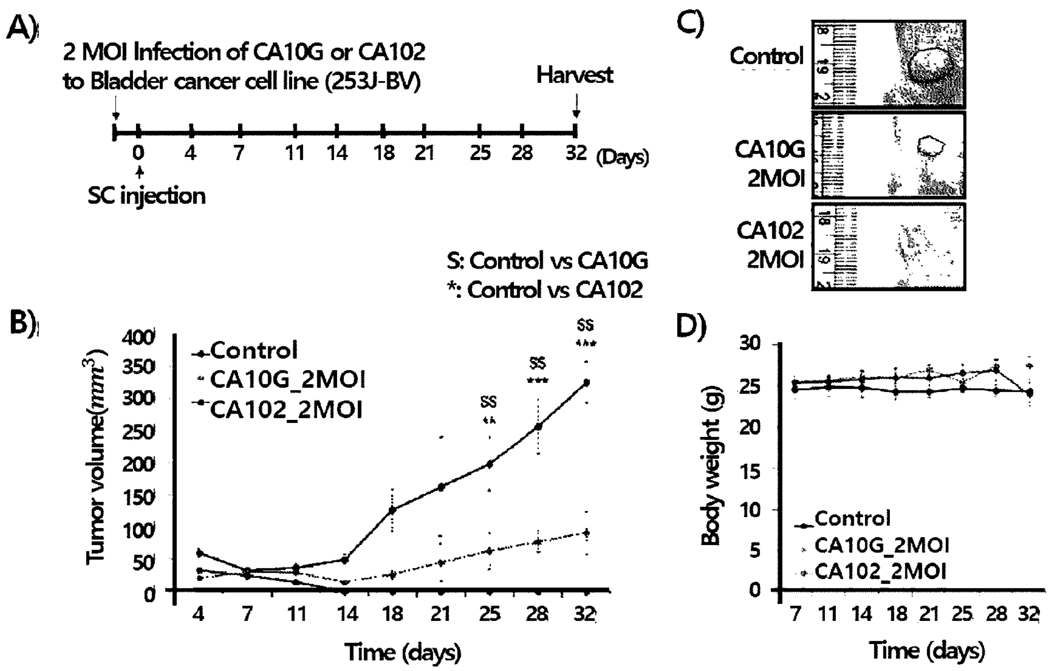
A)
2 MOI Infection of CA10G or CA102
to Bladder cancer cell line (253J-BV)
Harvest
0 4 7 11 14 18 21 25 28 32 (Days)
SC injection
C)
Control
CA10G
2MOI
CA102
2MOI
S: Control vs CA10G
*: Control vs CA102
B)
Tumor volume($mm^3$)
Control
CA10G_2MOI
CA102_2MOI
Time (days)
D)
Body weight (g)
Control
CA10G_2MOI
CA102_2MOI
Time (days)

Fig.36

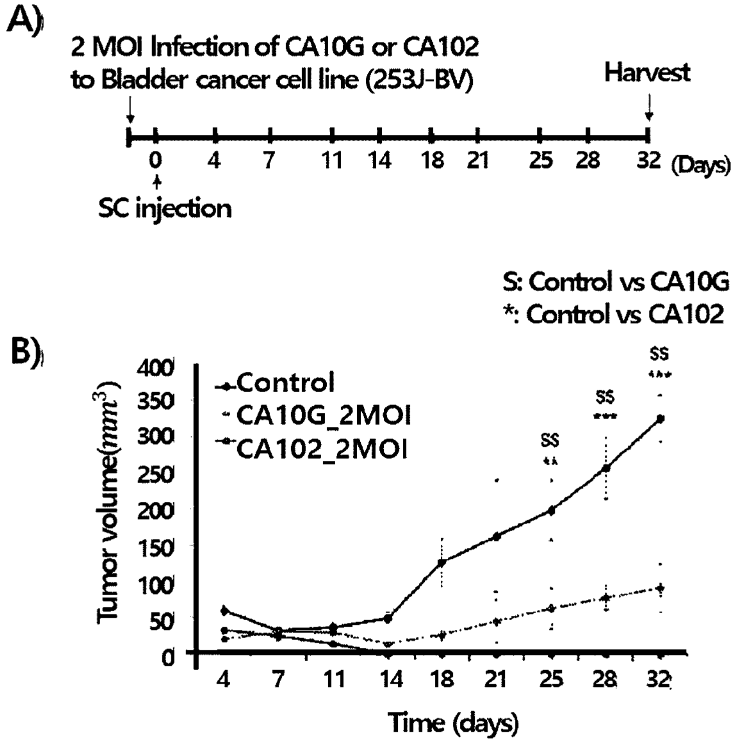
A)
2 MOI Infection of CA10G or CA102
to Bladder cancer cell line (253J-BV)
Harvest
0 4 7 11 14 18 21 25 28 32 (Days)
SC injection
B)
S: Control vs CA10G
*: Control vs CA102
Control
CA10G_2MOI
CA102_2MOI
Tumor volume($mm^3$)
Time (days)
C)
Control
CA10G 2MOI
CA102 2MOI
D)
Body weight (g)
Control
CA10G_2MOI
CA102_2MOI
Time (days)

Fig.37

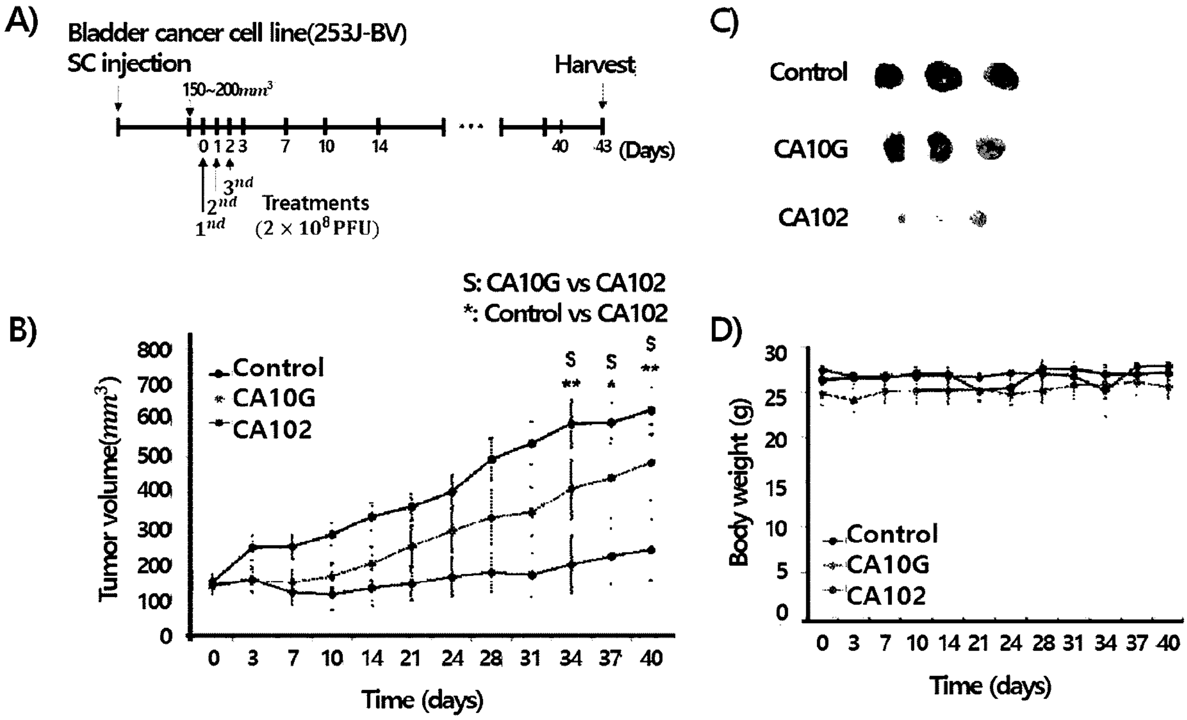
A)
Bladder cancer cell line(253J-BV)
SC injection
150~200$mm^3$
Harvest
0 1 2 3 7 10 14 40 43 (Days)
$1^{nd}$ $2^{nd}$ $3^{nd}$
Treatments
($2 \times 10^8$ PFU)
B)
S: CA10G vs CA102
*: Control vs CA102
Control
CA10G
CA102
Tumor volume($mm^3$)
Time (days)
C)
Control
CA10G
CA102
D)
Body weight (g)
Control
CA10G
CA102
Time (days)

Fig.38

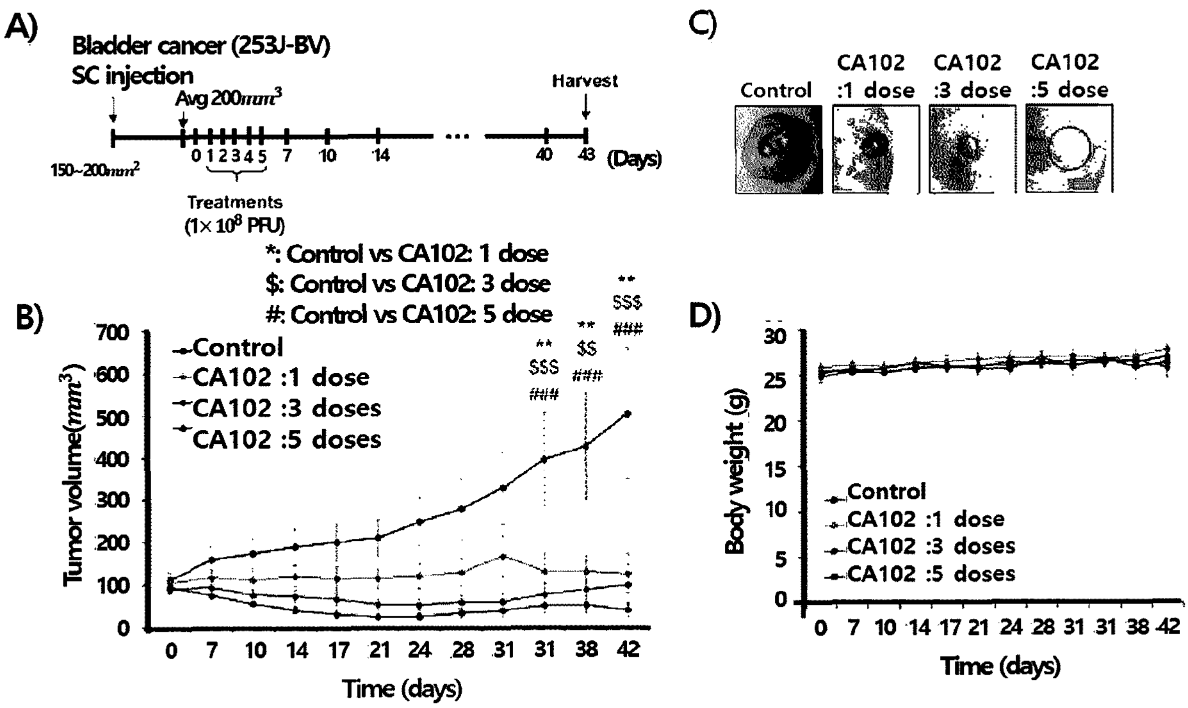
A)
Bladder cancer (253J-BV)
SC injection
Avg 200mm³
Harvest
150~200mm²
0 1 2 3 4 5 7 10 14 40 43 (Days)
Treatments
(1×10⁸ PFU)
C)
Control
CA102 :1 dose
CA102 :3 dose
CA102 :5 dose
*: Control vs CA102: 1 dose
$: Control vs CA102: 3 dose
#: Control vs CA102: 5 dose
B)
Control
CA102 :1 dose
CA102 :3 doses
CA102 :5 doses
Tumor volume(mm³)
Time (days)
D)
Body weight (g)
Control
CA102 :1 dose
CA102 :3 doses
CA102 :5 doses
Time (days)

Fig.39

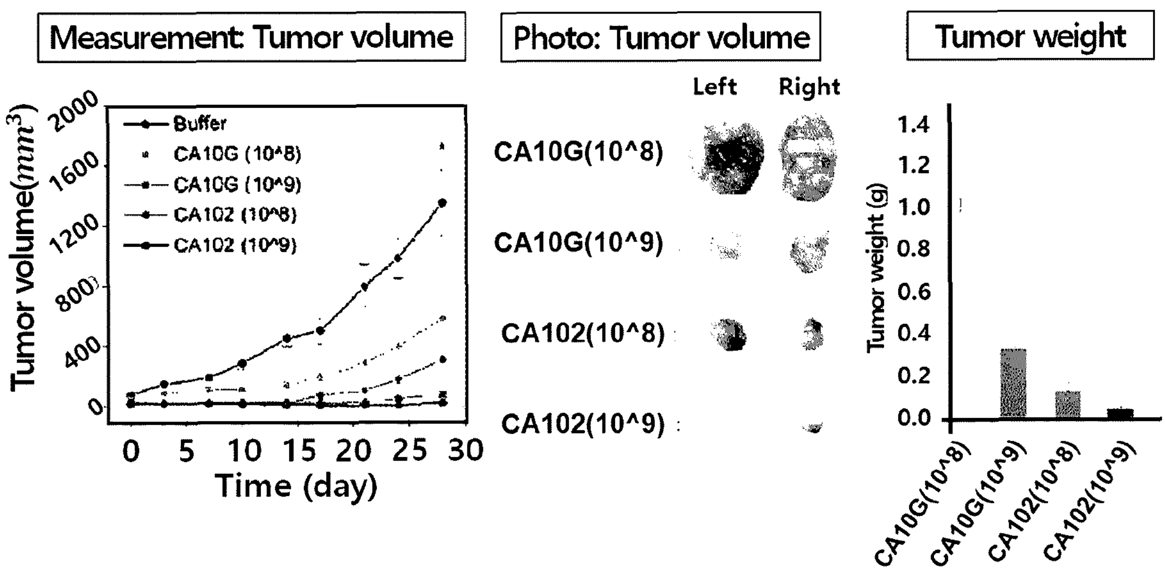
Measurement: Tumor volume
Photo: Tumor volume
Tumor weight
Buffer
CA10G (10^8)
CA10G (10^9)
CA102 (10^8)
CA102 (10^9)
Tumor volume(mm³)
Time (day)
Left
Right
CA10G(10^8)
CA10G(10^9)
CA102(10^8)
CA102(10^9)
Tumor weight (g)
CA10G(10^8)
CA10G(10^9)
CA102(10^8)
CA102(10^9)

Fig.40

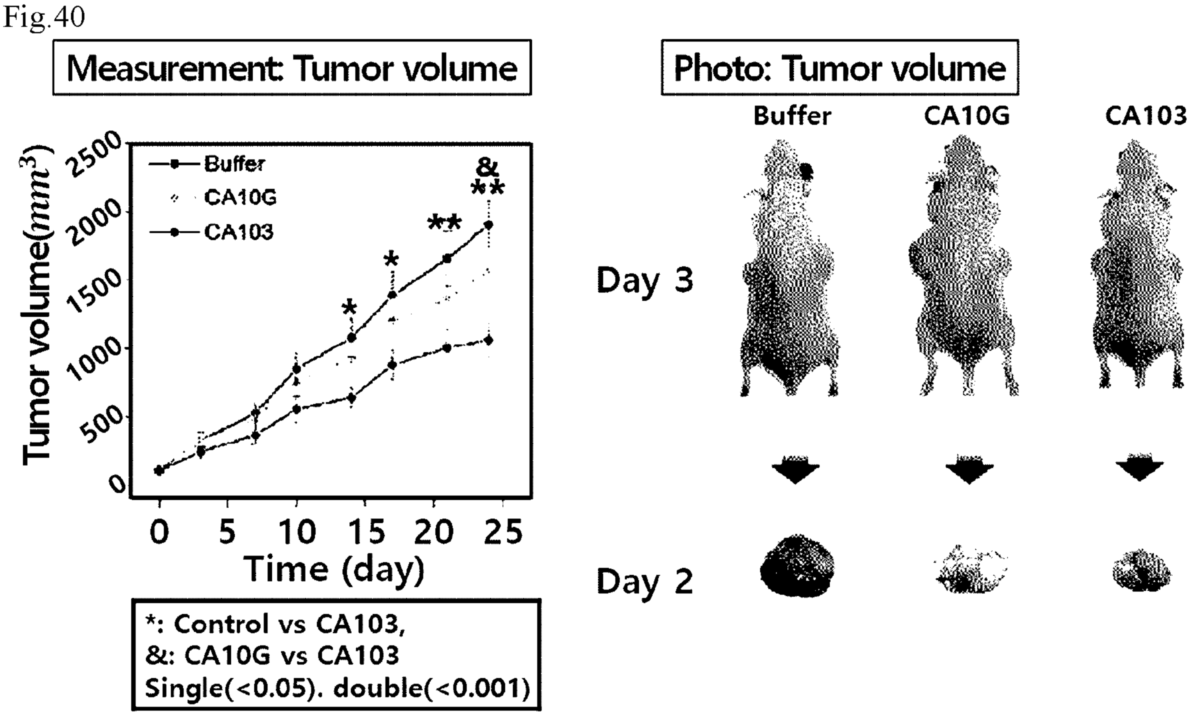
Measurement: Tumor volume
Tumor volume($mm^3$)
2500
2000
1500
1000
500
0
Buffer
CA10G
CA103
&
**
**
*
*
0 5 10 15 20 25
Time (day)
*: Control vs CA103,
&: CA10G vs CA103
Single(<0.05). double(<0.001)
Photo: Tumor volume
Buffer
CA10G
CA103
Day 3
Day 2

Fig.41

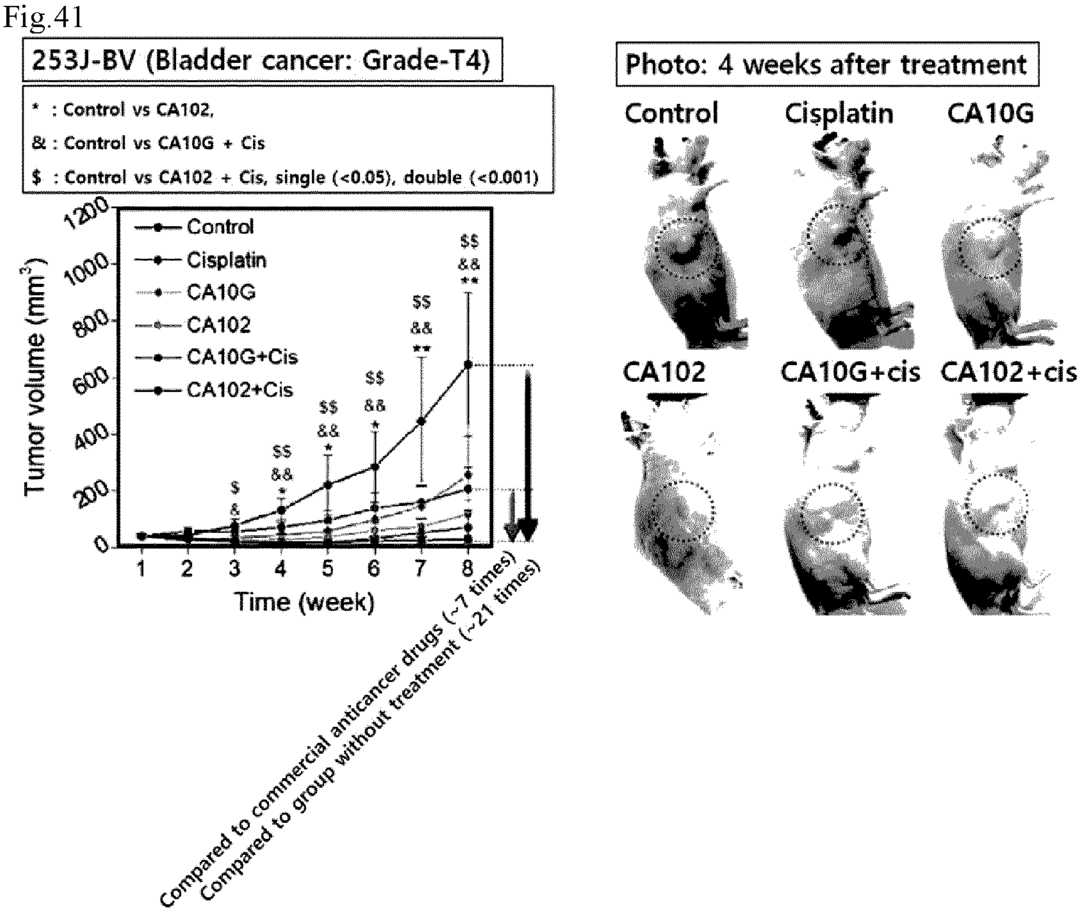
253J-BV (Bladder cancer: Grade-T4)
* : Control vs CA102,
& : Control vs CA10G + Cis
$ : Control vs CA102 + Cis, single (<0.05), double (<0.001)
Tumor volume ($mm^3$)
1200
1000
800
600
400
200
0
Control
Cisplatin
CA10G
CA102
CA10G+Cis
CA102+Cis
1 2 3 4 5 6 7 8
Time (week)
Compared to commercial anticancer drugs (~7 times)
Compared to group without treatment (~21 times)
Photo: 4 weeks after treatment
Control
Cisplatin
CA10G
CA102
CA10G+cis
CA102+cis

## INTERNATIONAL SEARCH REPORT

International application No.
**PCT/KR2021/003488**

**A. CLASSIFICATION OF SUBJECT MATTER**

**C12N 7/00**(2006.01)i; **C12N 15/113**(2010.01)i; **A61P 35/00**(2006.01)i; **A61K 35/761**(2014.01)i

According to International Patent Classification (IPC) or to both national classification and IPC

**B. FIELDS SEARCHED**

Minimum documentation searched (classification system followed by classification symbols)

C12N 7/00(2006.01); C12N 15/113(2010.01); C12N 15/34(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 인간 텔로미어 프로모터(Human telomerase reverse transcriptase, hTERT), 아데노바이러스(adenovirus), siRNA, shRNA, AR, mTOR, 종양(tumor)

**C. DOCUMENTS CONSIDERED TO BE RELEVANT**

| Category* | Citation of document, with indication, where appropriate, of the relevant passages | Relevant to claim No. |
|---|---|---|
| Y | SUGIO, K. et al. Enhanced safety profiles of the telomerase-specific replication-competent adenovirus by incorporation of normal cell-specific microRNA-targeted sequences. Clinical Cancer Research. 2011, vol. 17, no. 9, pp. 2807-2818. See abstract; figure 5; and pages 2808-2809, 2814-2815 and 2817. | 1-30 |
| Y | KR 10-2019-0009947 A (CURIGIN CO., LTD.) 30 January 2019 (2019-01-30) See abstract; paragraphs [0021] and [0029]; and claims 1-13. | 1-30 |
| Y | KR 10-1993377 B1 (CURIGIN CO., LTD.) 26 June 2019 (2019-06-26) See abstract; and claims 1 and 3-13. | 1-30 |
| Y | KR 10-1865025 B1 (CURIGIN CO., LTD.) 08 June 2018 (2018-06-08) See abstract; and claims 1 and 3-13. | 1-30 |
| A | EP 1767642 A1 (CHENGDU KANGHONG BIOTECHNOLOGIES CO., LTD.) 28 March 2007 (2007-03-28) See abstract; and claims 1-25. | 1-30 |

☐ Further documents are listed in the continuation of Box C.  ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **06 July 2021** | **06 July 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
**Information on patent family members**

International application No.
**PCT/KR2021/003488**

| Patent document cited in search report | Publication date (day/month/year) | Patent family member(s) | Publication date (day/month/year) |
|---|---|---|---|
| KR 10-2019-0009947 A | 30 January 2019 | AU 2018-303104 A1 | 06 February 2020 |
| | | CA 3070217 A1 | 24 January 2019 |
| | | CN 110945129 A | 31 March 2020 |
| | | EP 3656859 A2 | 27 May 2020 |
| | | JP 2020-530984 A | 05 November 2020 |
| | | KR 10-2020-0009286 A | 30 January 2020 |
| | | KR 10-2145664 B1 | 18 August 2020 |
| | | US 10947542 B2 | 16 March 2021 |
| | | US 2020-0231972 A1 | 23 July 2020 |
| | | WO 2019-017713 A2 | 24 January 2019 |
| | | WO 2019-017713 A3 | 11 April 2019 |
| | | WO 2019-017713 A9 | 24 January 2019 |
| KR 10-1993377 B1 | 26 June 2019 | KR 10-2020-0009498 A | 30 January 2020 |
| | | KR 10-2145665 B1 | 18 August 2020 |
| | | WO 2019-017714 A2 | 24 January 2019 |
| | | WO 2019-017714 A3 | 07 March 2019 |
| | | WO 2019-017714 A9 | 24 January 2019 |
| KR 10-1865025 B1 | 08 June 2018 | AU 2018-216509 A1 | 15 August 2019 |
| | | CA 3052038 A1 | 09 August 2018 |
| | | CN 110234764 A | 13 September 2019 |
| | | EP 3578655 A1 | 11 December 2019 |
| | | JP 2020-509782 A | 02 April 2020 |
| | | KR 10-2019-0087726 A | 25 July 2019 |
| | | KR 10-2034764 B1 | 22 October 2019 |
| | | US 2019-0345497 A1 | 14 November 2019 |
| | | US 2021-0054378 A1 | 25 February 2021 |
| | | WO 2018-143626 A1 | 09 August 2018 |
| EP 1767642 A1 | 28 March 2007 | BR PI0418805 A | 16 October 2007 |
| | | BR PI0418805 B1 | 26 January 2021 |
| | | CA 2568995 A1 | 22 December 2005 |
| | | CA 2568995 C | 21 February 2012 |
| | | CA 2568995 E | 22 December 2005 |
| | | CN 100361710 C | 16 January 2008 |
| | | CN 1706955 A | 14 December 2005 |
| | | DK 1767642 T3 | 26 May 2014 |
| | | EP 1767642 A4 | 25 June 2008 |
| | | EP 1767642 B1 | 23 April 2014 |
| | | ES 2466415 T3 | 10 June 2014 |
| | | JP 2008-501349 A | 24 January 2008 |
| | | JP 4874247 B2 | 15 February 2012 |
| | | PL 1767642 T3 | 29 August 2014 |
| | | PT 1767642 E | 03 June 2014 |
| | | RU 2006146665 A | 20 July 2008 |
| | | RU 2361611 C2 | 20 July 2009 |
| | | US 2010-0047208 A1 | 25 February 2010 |
| | | US 7951585 B2 | 31 May 2011 |
| | | WO 2005-121343 A1 | 22 December 2005 |

Form PCT/ISA/210 (patent family annex) (July 2019)

**INTERNATIONAL SEARCH REPORT**

International application No. **PCT/KR2021/003488**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **31**
because they relate to subject matter not required to be searched by this Authority, namely:

Claim 31 pertains to a method for treatment of the human body, and thus pertains to a subject matter on which the International Searching Authority is not required to carry out an international search under the provisions of PCT Article 17(2)(a)(i) and PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 991601
- US 409297
- US 5608149 A
- US 5608144 A
- US 5604121 A
- US 5569597 A
- US 5466785 A
- US 5399680 A
- US 5268463 A
- US 5608142 A


### Non-patent literature cited in the description


- *J Mol Med,* 2005, vol. 83, 764773
- NUCLEIC-ACID THERAPEUTICS: BASIC PRINCIPLES AND RECENT APPLICATIONS. *Nature Reviews Drug Discovery.,* 2002, vol. 1, 503-514
- *Cell Culture and in Vivo, Biochem. Biophys. Res. Commun.,* 2002, vol. 296, 1000-1004
- Progress Towards in Vivo Use of siRNAs. *MOLECULAR THERAPY.,* 2006, vol. 13 (4), 664-670
- **HITOSHI NIWA et al.** *Gene,* 1991, vol. 108, 193-199
- **ODELL et al.** *Nature,* 1985, vol. 313, 810-812
- **MCELROY et al.** *Plant Cell,* 1990, vol. 2, 163-171
- **CHRISTENSEN et al.** *Plant Mol. Biol.,* 1989, vol. 12, 619-632